# EUROPÄISCHE PATENTSCHRIFT

(11) **EP 2 822 704 B1**
(45) Veröffentlichungstag und Bekanntmachung des Hinweises auf die Patenterteilung: **11.11.2020**
(21) Anmeldenummer: 13712680.1
(22) Anmeldetag: 22.02.2013
(51) Int. Cl.: B05D 5/00, B05D 5/08, B63B 1/38, B05D 5/02, B05D 5/04

(54) **GASHALTENDE OBERFLÄCHENABDECKUNG, ANORDNUNG UND VERWENDUNG**
GAS-CONTAINING SURFACE COVER, ARRANGEMENT, AND USE
RECOUVREMENT SUPERFICIEL CONTENANT DU GAZ, SYSTÈME ET UTILISATION

(30) Priorität: 03.03.2012 DE 102012004067; 10.03.2012 DE 102012004574; 17.03.2012 DE 102012005163; 11.04.2012 DE 102012007068
(43) Veröffentlichungstag der Anmeldung: 14.01.2015
(73) Patentinhaber: Baden-Württemberg Stiftung gGmbH, 70174 Stuttgart (DE)
(72) Erfinder: SCHIMMEL, Thomas, 76131 Karlsruhe (DE)
(74) Vertreter: Müller-Boré & Partner Patentanwälte PartG mbB
(86) Internationale Anmeldenummer: PCT/EP2013/000523
(87) Internationale Veröffentlichungsnummer: WO 2013/131618

(56) Entgegenhaltungen:
- EP-A1- 0 616 940
- WO-A1-03/037702
- US-A1- 2010 236 466

## Beschreibung

Die Erfindung betrifft die Verwendung einer gashaltenden Oberflächenabdeckung für einen mit einer Flüssigkeit kontaktierbaren Körper.

Aus der Natur sind Oberflächen von Pflanzen und Tieren bekannt, welche beim Eintauchen in Wasser derart wenig durch das Wasser benetzbar sind, daß in der Struktur der Oberfläche Luft gehalten wird, so daß die eingetauchten Teile der Pflanze oder des Tieres nicht durch das Wasser benetzt werden. Diese Oberflächen findet man unter anderem bei Schwimmfarnen (beispielsweise *Salvinia molesta*) oder bei Wasserwanzen (beispielsweise *Notonecta glauca*). Mit Hilfe der an der Oberfläche festgehaltenen Luft mit Schichtdicken von etwa 1 µm bis etwa 1 mm können Schwimmfarne beispielsweise ihren Auftrieb vergrößern und Wasserwanzen können den mit unter Wasser genommenen Luftvorrat zur Atmung verwenden.

Jedoch entweicht Luft durch Ablösung von Gasbläschen und durch Lösen der Luft in der umgebenden Flüssigkeit aus der an der Oberfläche der Pflanze oder des Tieres festgehaltenen Luftschicht in das umgebende Wasser, so daß die Luftschicht mit der Zeit geringer wird. Da die Tauchzeit einer Wasserwanze und eines vitalen Schwimmfarnblattes jedoch kürzer ist als die Zeit, die zum Aufzehren der Luftschicht benötigt wird, ist die Lösung von Gasen aus der Luftschicht in das umgebende Wasser unproblematisch.

EP 0 616 940 A1 offenbart ein Verfahren zur Erzeugung einer Luftschicht über einer eingetauchten Oberfläche einer Struktur, die einen in eine Flüssigkeit eingetauchten Bereich aufweist, umfassend die Schritte des Bedeckens der eingetauchten Oberfläche einer Struktur, die einen in eine Flüssigkeit eingetauchten Bereich aufweist, mit einem Überzug, welcher feine Vertiefungen und Vorsprünge an dessen Oberfläche aufweist und wenigstens die Scheitel der Vorsprünge aus einem flüssigkeitsabweisenden Material gebildet sind; und des Zuführens von Luft in die Beschichtungsoberfläche, um eine Luftschicht zwischen der Beschichtungsoberfläche und Wasser zu bilden. WO 03/037702 A1 offenbart eine superhydrophobe Beschichtung, die als Substrat für ein gasförmiges Schmiermittel von sehr geringer Viskosität verwendet wird, welche die Flüssigkeitsreibung verringert. US 2010/236466 A1 offenbart eine Vorrichtung und ein Verfahren zum Verringern des Strömungswiderstands eines Schiffes unter Verwendung von Luftblasen.

Für technische Anwendungen stellt sich jedoch die Aufgabe, die Oberfläche eines untergetauchten Körpers dauerhaft mittels einer Luftschicht vor der umgebenden Flüssigkeit zu trennen.

Diese Aufgabe wird durch die Gegenstände der unabhängigen Ansprüche gelöst. Bevorzugte Ausführungsformen sind Gegenstand der abhängigen Ansprüche.

### Verwendung gemäß einem Aspekt

Ein Aspekt betrifft die Verwendung einer gashaltenden Schicht, welche an einem in eine Flüssigkeit eintauchbaren oder mit einer Flüssigkeit benetzbaren Körper angeordnet ist und welche mit einer flüssigkeitszugewandten Seite bei Eintauchen/Benetzen des Körpers mit der Flüssigkeit in Kontakt ist, wobei der Körper eine Wandung eines Wasserfahrzeugs oder eines im Wasser angeordneten Bauwerks oder eine Innenwandung eines Flüssigkeitsbehälters oder einer Flüssigkeitsleitung ist, wobei die gashaltende Schicht auf der flüssigkeitszugewandten Seite Ausnehmungen und/oder vorstehende Elemente aufweist, wobei die Oberflächen der Ausnehmungen zumindest bereichsweise hydrophob sind und die Oberfläche der vorstehenden Elemente im Wesentlichen hydrophob ist, wobei eine Gasschicht mit einer Dicke von 10 nm bis 10 mm, die in dem eingetauchten/benetzten Bereich der gashaltenden Schicht gehalten wird, die Flüssigkeit und den eingetauchten/benetzten Bereich des Körpers zumindest bereichsweise voneinander trennt, wobei die Flüssigkeit ein polares Lösungsmittel ist, und wobei die gashaltende Schicht durch fluidundurchlässige Trennwände in eine Vielzahl von Teilbereichen unterteilt ist und die Trennwände zumindest bereichsweise hydrophil ausgebildet sind.

Vorteilhafterweise ist es mittels der gashaltenden Schicht möglich, ein im wesentliches konstantes Gasvolumen für eine vordefinierte, insbesondere beliebige, Zeit derart an dem Körper zu halten, so daß der Körper durch die gashaltende Schicht bzw. das damit gehaltene Gas von der den Körper umgebenden Flüssigkeit getrennt werden kann. Insbesondere kann der Körper dadurch vorteilhafterweise vor korrosiven Flüssigkeiten geschützt werden. Weiter vorteilhafterweise kann der Strömungswiderstand des Körpers bei einer relativen Bewegung zwischen Körper und Flüssigkeit verringert werden.

Der Körper kann im Sinne der Anmeldung jeder feste Körper sein, welcher zumindest bereichsweise in eine Flüssigkeit eintauchbar ist, wie eine Wandung eines Wasserfahrzeugs oder eines im Wasser angeordneten Bauwerks oder eine Innenwandung eines Flüssigkeitsbehälters oder einer Flüssigkeitsleitung. Mit anderen Worten ist der Körper beim Eintauchen in die Flüssigkeit nicht darin lösbar und wird nicht durch den wirkenden Flüssigkeitsdruck zerstört. Dabei kann der Flüssigkeitsdruck zum einen von Außen in Richtung des Körperschwerpunktes wirken, wenn der Körper in die Flüssigkeit eingetaucht wird und zum anderen von Innen wirken, wenn die Flüssigkeit einen Hohlraum des Körpers füllt. Beispielhafte Körper im Sinne dieser Anmeldung sind Schiffe, Bojen, Pontons, Leitungen, Pipelines, Seekabel, Ölbohrplattformen, Gasbohrplattformen, Fundamente und dem Wasser ausgesetzte Teile von Offshore-Anlagen (insbesondere Windparks zur Elektrizitätserzeugung), Unterwasserbauwerke, Unterwasseranlagen, der Flüssigkeit ausgesetzte Messtechnik, Uferbauwerke, Behälter und Leitungen für Flüssigkeiten oder Teile davon. Bevorzugt umfaßt der Körper eine im Wesentlichen starre Wandung, auf welche der Flüssigkeitsdruck wirkt. Besonders bevorzugt ist die Wandung des Körpers rückstellfähig, insbesondere elastisch verformbar, ausgebildet.

Die Flüssigkeit, welche den Körper zumindest bereichsweise umgeben kann oder welche den Körper zumindest bereichsweise füllen kann, ist insbesondere Wasser (sowohl Süßwasser als auch Meerwasser) oder eine wässrige Lösung, jedoch kann die Flüssigkeit auch Alkohole und polare Lösungsmittel umfassen.

Die gashaltende Schicht kann verwendet werden, um eine Fläche bzw. Oberfläche des Körpers ganz oder teilweise zu bedecken bzw. auszubilden. Dabei kann die gashaltende Schicht lösbar oder unlösbar an dem Körper befestigt werden. Bevorzugt kann die Oberflächenabdeckung als eine Beschichtung des Körpers ausgebildet werden. Nach dem Befestigen der gashaltenden Schicht an dem Körper kann die gashaltende Schicht die Oberfläche des Körpers zumindest bereichsweise ausbilden bzw. als ein Teil des Körpers angesehen werden. Insbesondere wird die gashaltende Schicht derart an dem Körper angebracht, daß die Flüssigkeit nicht zwischen die gashaltende Schicht und den Körper gelangen kann.

Die zumindest gashaltende Schicht weist eine flüssigkeitszugewandte Seite und eine körperzugewandte Seite auf. Die gashaltende Schicht ist derart ausgebildet, daß bei betriebsgemäßen Gebrauch des Körpers ein Gas von der gashaltenden Schicht in Kontakt mit der gashaltenden Schicht gehalten wird, wobei die flüssigkeitszugewandte Seite der gashaltenden Schicht zumindest partiell, bevorzugt vollständig, durch das Gas von der Kontaktfläche bzw. Grenzfläche zwischen dem gehaltenen Gas und der Flüssigkeit (Flüssigkeit-Gas-Grenzfläche) beabstandet wird. Das Gas, welches durch die gashaltende Schicht gehalten wird, ist im Sinne der Anmeldung nicht Teil der gashaltenden Schicht bzw. des Körpers, sondern als Teil einer gasenthaltenden Schicht, welche die gashaltende Schicht und das daran gehaltene Gas umfaßt. Dieses gehaltene Gas wird mit anderen Worten durch die gashaltende Schicht fixiert, so daß es vorteilhafterweise nicht zur Flüssigkeitsoberfläche aufsteigt oder durch eine Flüssigkeitsströmung mitgerissen wird.

Die gashaltende Schicht kann eine Basis bzw. Basislage aufweisen, welche bevorzugt Strukturelemente wie Vorsprünge, vorstehende Elemente und/oder Ausnehmungen aufweisen kann, welche insbesondere ausgelegt sind, das Gas zu halten, und welche bevorzugt mit der Basis einstückig ausgebildet sind. Die Basis kann netzförmig oder als geschlossene Lage ausgebildet sein.

Die gashaltende Schicht weist demnach auch eine Kontaktfläche zwischen dem in der gashaltenden Schicht enthaltenen Gas und einem festen Material auf. Die flüssigkeitszugewandte Seite bzw. die gaszugewandte Seite der gashaltenden Schicht ist bevorzugt hydrophob ausgebildet bzw. kann mit einem hydrophoben Material beschichtet sein. Der Begriff "hydrophob" bedeutet im Sinne dieser Anmeldung soviel wie "flüssigkeitsabweisend", also "liquidophob". Der Begriff "hydrophob" impliziert, daß es sich bei der Flüssigkeit um Wasser oder eine wässrige Lösung oder im allgemeinen um ein polares Lösungsmittel handelt.

Ob eine Oberfläche bzw. ein Material flüssigkeitsabweisend ist, läßt sich über den Kontaktwinkel eines Flüssigkeitstropfens auf einer Oberfläche des Materials bestimmen. Die Größe des Kontaktwinkels zwischen Flüssigkeit und Feststoff hängt dabei von der Wechselwirkung zwischen der Flüssigkeit und dem Feststoff an der Kontaktfläche ab. Je geringer diese Wechselwirkung ist, desto größer wird der Kontaktwinkel. Hydrophile Feststoffe schließen mit der Oberfläche der Flüssigkeit, insbesondere mit Wasser, Kontaktwinkeln von etwa 0° bis etwa 90°, insbesondere Winkel kleiner als etwa 80° ein. Kontaktwinkel von etwa 90° und mehr treten bei hydrophoben Feststoffen auf. Feststoffe, die mit der Flüssigkeit, insbesondere mit Wasser, einen Kontaktwinkel von deutlich mehr als 90° aufweisen, insbesondere Kontaktwinkel von etwa 160° und mehr werden als superhydrophob bezeichnet. Der Begriff "hydrophob" schließt somit auch den bevorzugten Fall ein, daß das Material "superhydrophob" ist.

Der Gegenstand der Erfindung betrifft insbesondere die Koexistenz von hydrophilen und hydrophoben Bereichen bzw. Elementen. Im Sinne der Erfindung wird daher insbesondere ein erstes Element auch als hydrophob von einem zweiten hydrophilen Element unterschieden, wenn beide Elemente nach den oben beschriebenen absoluten Kriterien hinsichtlich des Kontaktwinkels mit einer Flüssigkeit als hydrophob oder hydrophil einzustufen sind, das erste Element jedoch hydrophober ist als das zweite Element. Mit anderen Worten wird das relativ hydrophobere Element bzw. der relativ hydrophobere Bereich als hydrophob und das relativ hydrophilere, also weniger hydrophobere, Element bzw. der relativ hydrophilere Bereich als hydrophil bezeichnet. Mit anderen Worten können die Begriffe hydrophob und hydrophil im Sinne der Erfindung eine relative Hydrophobizität oder einen Kontrast in der Hydrophobizität beschreiben.

Der gashaltenden Schicht kann bei betriebsgemäßem Gebrauch beispielsweise Luft, Kohlendioxid oder ein anderes Gas zugeführt werden.

Vorzugsweise weist die gashaltende Schicht auf der flüssigkeitszugewandten Seite zumindest bereichsweise Ausnehmungen und/oder Mulden auf. Die Oberfläche der gashaltenden Schicht kann im Bereich der Ausnehmungen und/oder Mulden vorzugsweise hydrophob ausgebildet sein. Beispielsweise kann das Material der gashaltenden Schicht aus einem hydrophoben Material bestehen. Alternativ kann die gashaltende Schicht ein hydrophiles Material umfassen, welches bereichsweise mit einer hydrophoben Beschichtung versehen ist. Insbesondere kann die hydrophobe Beschichtung lediglich an den Wandungen der Ausnehmungen oder Mulden ausgebildet sein. Besonders bevorzugt besteht die gashaltende Schicht zumindest bereichsweise aus einem porösen Material, wobei die Ausnehmungen bzw. Mulden durch Poren gebildet werden, die mit der Oberfläche in Verbindung stehen.

Vorzugsweise weist die gashaltende Schicht auf der flüssigkeitszugewandten Seite zumindest bereichsweise Vorsprünge oder vorstehende Elemente auf, wobei die Oberfläche der gashaltenden Schicht im Bereich der Vorsprünge oder vorstehenden Elemente im Wesentlichen hydrophob ist. Zweckmäßigerweise ist der Abstand zwischen den vorstehenden Elementen derart bemessen, daß sich keine Flüssigkeitstropfen zwischen den vorstehenden Elementen anordnen können. Vorteilhafterweise werden die einzelnen Tropfen der Flüssigkeit von einer Vielzahl von vorstehenden Elementen getragen, so daß sich die Grenzfläche zwischen Flüssigkeit und dem zwischen den vorstehenden Elementen befindlichen Gas im Wesentlichen als Einhüllende der vorstehenden Elemente ausbildet. Insbesondere kann der Abstand zwischen zwei benachbarten vorstehenden Elementen etwa 50µm bis etwa 500µm, bevorzugt etwa 100 µm bis etwa 200 µm, betragen.

Vorzugsweise weisen die Vorsprünge oder vorstehenden Elemente einen zentralen Oberflächenbereich auf, der hydrophil ist und der von einem hydrophoben Oberflächenbereich der Vorsprünge oder vorstehende Elemente umgeben ist. Vorteilhafterweise wird die Grenzfläche zwischen der Flüssigkeit und dem Gas an den Bereichen, welche hydrophil ausgebildet sind, lokalisiert. Dadurch wird weiter vorteilhafterweise ein Ablösen von Gasblasen durch eine Strömung der Flüssigkeit vermieden.

Die gashaltende Schicht ist durch fluidundurchlässige Trennwände in eine Vielzahl von Teilbereichen (auch "compartments" genannt) unterteilt, wobei die Trennwände (42) zumindest bereichsweise oder vollständig hydrophil ausgebildet oder zumindest bereichsweise oder vollständig mit einer hydrophilen Oberfläche versehen sind. Unter einem Fluid wird sowohl ein Gas, eine Flüssigkeit sowie ein Gemisch davon verstanden. Folglich verhindert die Trennwand, daß sich eine Flüssigkeitsströmung oder eine Gasströmung zwischen benachbarten Teilbereichen ausbildet. Vorteilhafterweise wird bei einem Druckunterschied zwischen zwei benachbarten Teilbereichen mittels der Trennwände verhindert, daß Gas von einem Teilbereich weg zu dem benachbarten Teilbereich strömt und dadurch der Strömungswiderstand gegenüber einer kontaktierenden Flüssigkeit lokal erhöht wird und im Gegensatz dazu aus dem Teilbereich, in den das Gas strömt, überschüssiges Gas in die Flüssigkeit abgegeben wird.

Bevorzugt können die Trennwände zusammen mit den weiteren Elementen der gashaltenden Schicht einstückig bzw. integral ausgebildet sein. Weiter bevorzugt befindet sich eine Vielzahl von hydrophoben vorstehenden Elementen in einer zweidimensionalen Anordnung in jedem der Teilbereiche der gashaltenden Schicht.

Vorzugsweise umfaßt die gashaltende Schicht ein geprägtes Kunstharz oder einen geprägten Lack. Insbesondere kann die gashaltende Schicht aus einem flüssigen Kunstharz gegossen werden, wobei bevorzugt vorstehende Elemente integral bzw. einstückig mit einer Basislage der gashaltenden Schicht und/oder mit der gasdurchlässigen Lage ausgebildet werden. Insbesondere kann die Basislage der gashaltenden Schicht identisch mit der gasdurchlässigen Lage sein. Besonders bevorzugt wird die gashaltende Schicht mittels des Kunstharzes bzw. des Lacks mittelbar oder unmittelbar an der Wandung des eintauchbaren Körpers ausgebildet. Insbesondere kann die gashaltende Schicht verwendet werden, um eine Oberflächenbeschichtung bzw. Oberflächenversiegelung des Körpers durchzuführen.

Vorzugsweise ist die gashaltende Schicht zumindest bereichsweise mit Polytetrafluorethylen (PTFE), auch unter dem Handelsnamen Teflon bekannt, oder dessen Derivaten beschichtet. Insbesondere kann die Beschichtung auch Mikropartikel bzw. Nanopartikel aus Polytetrafluorethylen oder anderen Materialien umfassen. Vorteilhafterweise wirkt die Beschichtung aus PTFE als hydrophobe Schicht und als Antihaftmittel, so daß ein Anhaften von Flüssigkeiten oder Feststoffen an der gashaltenden Schicht verhindert wird. Bevorzugt ist die Beschichtung der gashaltenden Schicht etwa 0,15 nm bis etwa 500 nm dick.

Vorzugsweise ist die Flüssigkeit Wasser und der Körper ein Wasserfahrzeug, dessen Wandung in einer Betriebsposition des Wasserzeugs zumindest bereichsweise in das Wasser eingetaucht ist.

Vorteilhafterweise kann das Wasser, insbesondere Meerwasser, die Wandung des Wasserfahrzeugs zumindest bereichsweise nicht benetzen, so daß das Wasserfahrzeug vor dem Einfluß des Wassers geschützt ist. Das Wasserfahrzeug kann beispielsweise ein Schiff, eine Bohrinsel, oder eine Boje sein. Der Einfluß des Wassers betrifft insbesondere die Korrosion der Wandung des Wasserfahrzeugs. Insbesondere Meerwasser oder Brackwasser fördern aufgrund des Salzgehaltes die Korrosion der Wandung des Wasserfahrzeugs. Da der Kontakt zwischen dem Wasser und der Wandung des Wasserfahrzeugs mittels des dazwischen angeordneten Gases, welches durch die gashaltende Schicht gehalten wird, unterbunden ist, wird auch die Korrosion gemindert.

Ein weiterer Vorteil besteht darin, daß der Bewuchs der Wandung des Wasserfahrzeugs durch im Wasser lebende Organismen, beispielsweise Algen, Muscheln, Seepocken und andere, vermindert wird. Durch die Gasschicht wird diesen Organismen erschwert sich an der Wandung des Wasserfahrzeugs festzusetzen. Mit anderen Worten besitzt die Oberflächenabdeckung eine Anti-Fouling-Wirkung, wobei vorteilhafterweise auf Biozide verzichtet werden kann, deren giftige Substanzen sich über die Zeit im Wasser lösen. Durch die reduzierte Anhaftung von Organismen an der Wandung des Wasserfahrzeugs ist auch der Strömungswiderstand des Wasserfahrzeugs verringert.

Vorzugsweise wird die gashaltende Schicht zumindest bereichsweise derart mit einem Gas beaufschlagt, um den Strömungswiderstand zwischen dem Wasser und dem Wasserfahrzeug zu vermindern. Die Gasschicht weist eine Dicke von etwa 10 nm bis etwa 10 mm, bevorzugt von etwa 500 nm bis etwa 3 mm, insbesondere etwa 0,1 mm bis etwa 3 mm, auf. Soll die Gasschicht lediglich zum Korrosionschutz dienen, so kann auch ein dünnere Gasschicht mit einer Dicke von etwa 10 nm bis etwa 3 mm, bevorzugt von etwa 50 nm bis etwa 1 mm, insbesondere etwa 100 nm bis etwa 100 µm, ausreichen, um den Korrosionsschutz zu erzielen. Da die Gasschicht die Wandung des Wasserfahrzeugs von dem vorbeiströmenden Wasser entkoppelt und insbesondere die Kontaktfläche zwischen Gas und Wasser durch die Strömung verformbar ist, so daß eine möglichst stromlinienförmige Kontaktfläche ausgebildet wird, ist der Strömungswiderstand des Wasserfahrzeugs bei der Fahrt durch das Wasser vorteilhafterweise reduziert. Insbesondere kann aufgrund des verringerten Strömungswiderstandes zwischen dem Wasserfahrzeug und dem Wasser vorteilhafterweise der Treibstoffverbrauch des Wasserfahrzeugs gesenkt werden.

Vorzugsweise ist zwischen der gashaltenden Schicht und der Wandung des Wasserfahrzeugs eine Korrosionsschutzschicht und/oder eine Bewuchsschutzschicht angeordnet, wobei die gashaltende Schicht zumindest bereichsweise die Korrosionsschutzschicht und/oder die Bewuchsschutzschicht von dem Wasser trennt.

Die Korrosionsschutzschicht ist in der Regel als ein Anstrich der Wandung des Wasserfahrzeugs ausgebildet und kann giftige Substanzen wie beispielsweise Schwermetalle enthalten. Das Lösen dieser giftigen Substanzen aus der Korrosionsschutzschicht durch das Wasser wird durch die Anordnung der gashaltenden Schicht zwischen der Korrosionsschutzschicht und dem Wasser vermindert bzw. verhindert. Dadurch wird vorteilhafterweise eine Kontamination des Wassers, insbesondere des Meerwassers, durch giftige Substanzen vermieden. Insbesondere wird vermieden, daß Schwermetalle aus einem Korrosionsschutzanstrich ins Wasser gelangen und sich dort in der Nahrungskette anreichern.

Alternativ oder zusätzlich kann eine Bewuchsschutzschicht bzw. eine Antifoulingschicht an der Wandung des Wasserfahrzeugs, beispielsweise in Form eines Anstrichs der Wandung, ausgebildet sein. Die Bewuchsschutzschicht enthält in der Regel Biozide, also giftige Substanzen, die für die Organismen, die sich an die Wandung des Wasserfahrzeugs anhaften, tödlich sind. Das Lösen dieser giftigen Substanzen aus der Bewuchsschutzschicht durch das Wasser wird durch die Anordnung der gashaltenden Schicht zwischen der Bewuchsschutzschicht und dem Wasser vermindert bzw. verhindert. Dadurch wird vorteilhafterweise eine Kontamination des Wassers, insbesondere des Meerwassers, durch die Biozide vermieden. Insbesondere wird vermieden, daß die Biozide im Wasser lebende Organismen, wie beispielsweise Plankton, schädigen oder töten, wodurch die Nahrungskette im Wasser gestört würde.

Vorzugsweise wird die gashaltende Schicht mit einem bewuchshemmenden Gas beschickt. Das bewuchshemmende Gas kann beispielsweise Kohlendioxid enthalten, um den Organismen, welche sich an der Wandung des Wasserfahrzeugs angesiedelt haben, den Sauerstoff zu entziehen bzw. mit Kohlendioxid zu sättigen. Dadurch sterben diese Organismen ab, ohne daß eine toxische Substanz in einer Bewuchsschutzschicht verwendet werden müßte. Dies führt vorteilhafterweise zu einer geringeren Belastung des Wassers durch Giftstoffe.

Vorzugsweise ist der Körper eine Behälterwandung, welche mit einer Flüssigkeit benetzbar ist und an deren Wandung zumindest bereichsweise die gashaltende Schicht angeordnet ist.

Vorteilhafterweise kann die Flüssigkeit die Behälterwandung zumindest bereichsweise nicht benetzen, so daß die Behälterwandung vor dem Einfluß der Flüssigkeit geschützt ist. Der Behälter, welcher die Behälterwandung aufweist, kann beispielsweise ein Tank, eine Leitung, ein Reaktor oder dergleichen sein. Der Einfluß der Flüssigkeit betrifft insbesondere die Korrosion der Behälterwandung, die chemische Reaktion der Flüssigkeit mit der Behälterwandung bzw. die mechanische Beanspruchung der Behälterwandung durch in der Flüssigkeit enthaltene Partikel. Insbesondere salzige Lösungen, Laugen oder Säuren fördern die Korrosion der Behälterwandung. Da der Kontakt zwischen der Flüssigkeit und der Behälterwandung mittels des dazwischen angeordneten Gases, welches durch die gashaltende Schicht gehalten wird, unterbunden ist, wird auch die Korrosion gemindert.

Besonders bevorzugt umfaßt die Behälterwandung ein Sensorfenster, an welchem eine gashaltende Schicht angeordnet ist. Vorteilhafterweise kann die Erfassung von Sensordaten verbessert werden, da sich keine Ablagerungen, beispielsweise Partikel oder Organismen, an dem Sensorfenster festsetzen können. Insbesondere ist das Sensorfenster und/oder die gashaltende Schicht optisch durchsichtig bzw. transparent.

Vorzugsweise ist zwischen der gashaltenden Schicht und der Wandung des Behälters eine Korrosionsschutzschicht angeordnet, wobei die gashaltende Schicht zumindest bereichsweise die Korrosionsschutzschicht von der Flüssigkeit trennt.

Die Korrosionsschutzschicht ist in der Regel als ein Anstrich der Behälterwandung oder durch Galvanisieren bzw. Eloxieren ausgebildet und kann giftige Substanzen wie beispielsweise Schwermetalle enthalten. Das Lösen dieser giftigen Substanzen aus der Korrosionsschutzschicht durch die Flüssigkeit wird durch die Anordnung der gashaltenden Schicht zwischen der Korrosionsschutzschicht und der Flüssigkeit vermindert bzw. verhindert. Dadurch wird vorteilhafterweise eine Kontamination der Flüssigkeit, insbesondere durch giftige Substanzen, vermieden. Insbesondere wird vermieden, daß diese Substanzen aus der Korrosionsschutzschicht eine chemische Reaktion innerhalb des Behälters beeinflussen.

Vorzugsweise wird die gashaltende Schicht von einer der flüssigkeitszugewandten Seite gegenüberliegenden körperzugewandten Seite der gashaltenden Schicht her mit Gas beaufschlagt.

Vorzugsweise ist eine gasdurchlässige Lage an der körperzugewandten Seite an der gashaltenden Schicht angeordnet. Mit anderen Worten kann die gasdurchlässige Lage auf der körperzugewandten Seite der gashaltenden Schicht angeordnet bzw. befestigt sein. Alternativ kann die gasdurchlässige Lage auch mit der gashaltenden Schicht einstückig ausgebildet sein bzw. integraler Bestandteil der gashaltenden Schicht sein. Durch die flüssigkeitszugewandte Seite der gasdurchlässigen Lage, welche mit der körperzugewandten Seite der gashaltenden Schicht kontaktieren kann, ist es möglich der gashaltenden Schicht ein Gas zuzuführen. Mit anderen Worten kann die gasdurchlässige Lage für ein Gas durchlässig sein, insbesondere in einer Richtung welche senkrecht zur körperzugewandten Seite der gashaltenden Schicht orientiert ist.

Vorzugsweise ist die gasdurchlässige Lage als flüssigkeitsundurchlässige und/oder hydrophobe Lage ausgebildet. Vorteilhafterweise kann die Flüssigkeit nicht durch die gasdurchlässige Lage in Richtung des Körpers strömen, beispielsweise wenn der Flüssigkeitsdruck temporär größer ist als der Gasdruck in der gasdurchlässigen Lage. Mit anderen Worten stößt die gasdurchlässige Lage Wasser und andere polare Lösungsmittel ab, wodurch vorteilhafterweise ein Eindringen dieser polaren Lösungsmittel in die gasdurchlässige Lage verhindert wird.

Vorzugsweise ist eine Gaszufuhreinrichtung mit der gasdurchlässigen Lage verbunden, so daß Gas von einer Gaszufuhreinrichtung durch die gasdurchlässige Lage zur gashaltenden Schicht strömen kann.

Vorteilhafterweise kann der gashaltenden Schicht Gas mittels der Gaszufuhreinrichtung und über die gasdurchlässige Lage zugeführt werden. Insbesondere ist zumindest die Menge an Gas zuführbar, welche aus der gashaltenden Schicht in die umgebende Flüssigkeit entweicht. Dadurch ist es vorteilhafterweise möglich innerhalb der gashaltenden Schicht ein im wesentliches konstantes Gasvolumen für eine beliebige Zeit zu halten, wodurch der Körper und die umgebende Flüssigkeit dauerhaft mittels des Gases in der gashaltenden Schicht getrennt werden können.

Das Gas kann mittels der Gaszufuhreinrichtung bereitgestellt werden, welche mit der gasdurchlässigen Lage verbunden ist. Bevorzugt kann die Gaszufuhreinrichtung eine Lage eines porösen Materials mit durchgängigem Porenraum sein, so daß Gas von der Gaszufuhreinrichtung durch die gasdurchlässige Lage zur gashaltenden Schicht strömen kann.

Vorzugsweise umfaßt die gasdurchlässige Lage ein gewebtes oder nicht gewebtes Textil, ein Flockmaterial, eine poröse Keramik, ein poröses Metall, ein Filz aus Polymer oder Metallfasern und/oder ein Metalldrahtgeflecht. Beispielsweise kann die gasdurchlässige Lage aus Polymerfasern gewebt sein oder aus einem Filz oder Vlies aus Polymerfasern bestehen. Eine gasdurchlässige Lage aus einem Polymer kann insbesondere durch Laminieren mit der gashaltenden Schicht verbunden werden. Die gasdurchlässige Lage kann alternativ oder zusätzlich ein Gewebe aus einem Metalldraht umfassen, insbesondere aus einem korrosionsbeständigem Metalldraht, beispielsweise einem nicht-rostenden Edelstahldraht, wodurch vorteilhafterweise eine hohe mechanische Reißfestigkeit und Beständigkeit gegenüber UV Strahlung und chemischen Einflüssen erreicht wird. Alternativ kann die gasdurchlässige Lage auch ein Flockmaterial, beispielsweise Polymerflocken bzw. Elastomerflocken, insbesondere aus einem geschäumten Material, umfassen. Dadurch kann das Gewicht der gasdurchlässigen Lage vorteilhafterweise reduziert werden. Weiter kann die gasdurchlässige Lage bevorzugt ein Sintermaterial, wie beispielsweise ein poröses Sintermaterial aus Metallpartikeln umfassen. Die gasdurchlässige Lage kann bevorzugt mit einem Klebemittel mit der gashaltenden Schicht verbunden werden. Alternativ können die gashaltende Schicht und die gasdurchlässige Lage durch Schweißen, insbesondere Ultraschallschweißen (ultrasonic welding), miteinander verbunden werden. Besonders bevorzugt sind die gashaltige Schicht und die gasdurchlässige Lage einstückig miteinander ausgebildet, beispielsweise durch ein (kontinuierliches) Formen bzw. Gießen aus einem Polymer.

Vorzugsweise ist die gasdurchlässige Lage als poröse, semipermeable Membran ausgebildet. Die gasdurchlässige Lage kann insbesondere als gasdurchlässige Folie aus einem Polymer ausgebildet sein. Gasdurchlässige Folien können beispielsweise eine Dicke von etwa 0,5 µm bis 5 µm aufweisen. Dadurch kann eine Oberflächenabdeckung mit geringer Dicke und geringem Gewicht bereitgestellt werden. Insbesondere können Folien aus einem Polymer durch Laminieren mit der gashaltenden Schicht verbunden werden.

Vorzugsweise ist die Gaszufuhreinrichtung als gasdurchlässige Schicht ausgebildet, welche an der körperzugewandten Seite der gasdurchlässigen Lage angeordnet ist. Insbesondere kann die Gaszufuhreinrichtung auch ein poröses Material umfassen, welches durchgängige Poren aufweist. Zweckmäßigerweise ist die Gasdurchgängigkeit durch die Gaszufuhreinrichtung höher als die Gasdurchgängigkeit durch die gasdurchlässige Lage.

Vorzugsweise ist die Gaszufuhreinrichtung in Form eines Aerenchyms ausgebildet. Ein Aerenchym ist ein Durchlüftungsgewebe von Wasserpflanzen, welches einen Gastransport und eine Gasspeicherung ermöglicht. Insbesondere versteht man unter dem Begriff "Aerenchym" eine Form des pflanzlichen Grundgewebes, bei der die Interzellularräume so weit sind, daß ein regelrechtes "Durchlüftungsgewebe" entsteht. Es tritt besonders bei Sumpf- und Wasserpflanzen auf und dient dem Gaswechsel der untergetauchten Pflanzenorgane.

Mit anderen Worten ist die Gaszufuhreinrichtung als Gasspeicher ausgebildet, so daß bei steigendem Flüssigkeitsdruck Gas über die gasdurchlässige Lage zurück in die Gaszufuhreinrichtung verlagerbar ist und dort speicherbar ist, um bei einem Sinken des Flüssigkeitsdrucks wieder über die gasdurchlässige Lage in die gashaltende Schicht verlagerbar zu sein. Die Gaszufuhreinrichtung kann beispielsweise ein poröses Material aufweisen, in welchem Gas speicherbar ist. Ferner kann das Material rückstellfähig dehnbar sein, so daß sich das Volumen bei steigendem Gasdruck erhöht und durch die Rückstellkraft des Material Gas durch die gasdurchlässige Lage in die gashaltende Schicht gepreßt werden kann. Weiter bevorzugt sind die Innenwandungen der Gaszufuhreinrichtung, welche mit dem Gas kontaktieren, zumindest bereichsweise hydrophob ausgebildet. Vorteilhafterweise kann das Gas aus der gashaltenden Schicht bei Schwankungen des Flüssigkeitsdrucks in der Gaszufuhreinrichtung zwischengespeichert werden, anstatt von der gashaltenden Schicht losgelöst zu werden.

Vorzugsweise wird die gashaltende Schicht von der flüssigkeitszugewandten Seite der gashaltenden Schicht her mit Gas beaufschlagt.

Vorzugsweise ist zumindest eine Gasaustrittseinrichtung vorgesehen, welche eine Gasaustrittsöffnung an der flüssigkeitszugewandten Seite der gashaltenden Schicht aufweist; wobei eine Gaszufuhreinrichtung vorgesehen ist, welche mit der Gasaustrittseinrichtung verbunden ist, wobei von der Gaszufuhreinrichtung bereitgestelltes Gas aus der Gasaustrittseinrichtung strömt und von der gashaltenden Schicht zumindest partiell aufgenommen wird.

Vorzugsweise durchstößt die Gasaustrittseinrichtung die gashaltende Schicht. Mit anderen Worten ist eine Gasaustrittsöffnung der Gasaustrittseinrichtung an der flüssigkeitszugewandten Seite der gashaltenden Schicht angeordnet.

Vorzugsweise ist die Gaszufuhreinrichtung als gasdurchlässige Schicht ausgebildet, welche an der körperzugewandten Seite der gasdurchlässigen Lage angeordnet ist.

Besonders bevorzugt ist die Gaszufuhreinrichtung in Form eines Aerenchyms ausgebildet. Vorteilhafterweise kann die Gaszufuhreinrichtung in einfacher Weise mit einer Vielzahl von Gasaustrittseinrichtungen fluidisch verbunden sein, wobei die Gasaustrittseinrichtungen insbesondere regelmäßig oder unregelmäßig flächig angeordnet sein können.

Ein Aspekt betrifft ein Wasserfahrzeug mit:
- einer Wandung, welche in einer Betriebsposition des Wasserzeugs zumindest bereichsweise in Wasser eingetaucht ist, wobei an einer dem Wasser zugewandten Seite eine zumindest partiell gashaltende Schicht angeordnet ist; und mit:
-- einer gasdurchlässigen Lage, welche an einer der wasserzugewandten Seite gegenüberliegenden wandungszugewandten Seite zwischen der gashaltenden Schicht und der Wandung angeordnet ist;
-- einer Gaszufuhreinrichtung, welche mit der gasdurchlässigen Lage verbunden ist, so daß Gas von der Gaszufuhreinrichtung durch die gasdurchlässige Lage zur gashaltenden Schicht strömen kann, oder mit:
-- zumindest einer Gasaustrittseinrichtung, welche eine Gasaustrittsöffnung an der wasserzugewandten Seite der gashaltenden Schicht aufweist;
-- einer Gaszufuhreinrichtung, welche mit der Gasaustrittseinrichtung verbunden ist, wobei von der Gaszufuhreinrichtung bereitgestelltes Gas aus der Gasaustrittseinrichtung strömbar und von der gashaltenden Schicht zumindest partiell aufnehmbar ist.

Vorzugsweise ist zwischen der gashaltenden Schicht und der Wandung des Wasserfahrzeugs eine Korrosionsschutzschicht und/oder eine Bewuchsschutzschicht angeordnet ist und wobei die gashaltende Schicht zumindest bereichsweise die Korrosionsschutzschicht und/oder die Bewuchsschutzschicht von dem Wasser trennt.

Vorzugsweise ist die gashaltende Schicht mit einem bewuchshemmenden Gas beschickbar.

Ein Aspekt betrifft einen Flüssigkeitsbehälter umfassend:
- eine Behälterwandung, welche zumindest bereichsweise mit einer Flüssigkeit benetzbar ist, wobei an einer der Flüssigkeit zugewandten Seite der Behälterwandung eine zumindest partiell gashaltende Schicht angeordnet ist; und umfassend:
-- eine gasdurchlässige Lage, welche an einer der wasserzugewandten Seite gegenüberliegenden wandungszugewandten Seite zwischen der gashaltenden Schicht und der Behälterwandung angeordnet ist; und
-- eine Gaszufuhreinrichtung, welche mit der gasdurchlässigen Lage verbunden ist, so daß Gas von der Gaszufuhreinrichtung durch die gasdurchlässige Lage zur gashaltenden Schicht strömen kann, oder umfassend:
   - zumindest eine Gasaustrittseinrichtung, welche eine Gasaustrittsöffnung an der flüssigkeitszugewandten Seite der gashaltenden Schicht aufweist; und
   - eine Gaszufuhreinrichtung, welche mit der Gasaustrittseinrichtung verbunden ist, wobei von der Gaszufuhreinrichtung bereitgestelltes Gas aus der Gasaustrittseinrichtung strömbar und von der gashaltenden Schicht zumindest partiell aufnehmbar ist.

Vorzugsweise ist zwischen der gashaltenden Schicht und der Wandung des Flüssigkeitsbehälters eine Korrosionsschutzschicht angeordnet, wobei die gashaltende Schicht zumindest bereichsweise die Korrosionsschutzschicht von der Flüssigkeit trennt.

Ein Aspekt betrifft eine Oberflächenabdeckung für einen mit einer Flüssigkeit kontaktierbaren Körper umfassend:
- eine zumindest partiell gashaltende Schicht, welche derart auslegt und angeordnet ist, um mit einer flüssigkeitszugewandten Seite zumindest bereichsweise mit der Flüssigkeit zu kontaktieren;
- eine gasdurchlässige Lage, welche an einer der flüssigkeitszugewandten Seite gegenüberliegenden körperzugewandten Seite an der gashaltenden Schicht angeordnet ist oder welche mit der gashaltenden Schicht integral ausgebildet ist;
- eine Gaszufuhreinrichtung, welche mit der gasdurchlässigen Lage verbunden ist, so daß Gas von der Gaszufuhreinrichtung durch die gasdurchlässige Lage zur gashaltenden Schicht strömen kann.

Vorteilhafterweise kann der gashaltenden Schicht Gas mittels der Gaszufuhreinrichtung und über die gasdurchlässige Lage zugeführt werden. Insbesondere ist zumindest die Menge an Gas zuführbar, welche aus der gashaltenden Schicht in die umgebende Flüssigkeit entweicht. Dadurch ist es vorteilhafterweise möglich innerhalb der gashaltenden Schicht ein im wesentliches konstantes Gasvolumen für eine beliebige Zeit zu halten, wodurch der Körper und die umgebende Flüssigkeit dauerhaft mittels des Gases in der gashaltenden Schicht getrennt werden können. Insbesondere kann der Körper dadurch vorteilhafterweise vor korrosiven Flüssigkeiten geschützt werden. Weiter vorteilhafterweise kann der Strömungswiderstand des Körpers bei einer relativen Bewegung zwischen Körper und Flüssigkeit verringert werden.

Der mit der Flüssigkeit kontaktierbare Körper kann im Sinne der Anmeldung jeder feste Körper sein, welcher zumindest bereichsweise in eine Flüssigkeit eintauchbar ist, wie eine Wandung eines Wasserfahrzeugs oder eines im Wasser angeordneten Bauwerks oder eine Innenwandung eines Flüssigkeitsbehälters oder einer Flüssigkeitsleitung. Mit anderen Worten löst sich der Körper beim Eintauchen in die Flüssigkeit nicht darin auf und wird ebenfalls nicht durch den wirkenden Flüssigkeitsdruck zerstört. Dabei kann der Flüssigkeitsdruck zum einen von Außen in Richtung des Körperschwerpunktes wirken, wenn der Körper in die Flüssigkeit eingetaucht wird und zum anderen von Innen wirken, wenn die Flüssigkeit in einen Hohlraum des Körpers gefüllt wird. Beispielhafte Körper im Sinne dieser Anmeldung sind Schiffe, Bojen, Pontons, Uferbauwerke, Behälter und Leitungen für Flüssigkeiten. Bevorzugt umfaßt der Körper eine im Wesentlichen starre Wandung, auf welche der Flüssigkeitsdruck wirkt. Besonders bevorzugt ist die Wandung des Körpers rückstellfähig, insbesondere elastisch verformbar, ausgebildet.

Der Begriff "rückstellfähig" im Sinne der Anmeldung umfaßt insbesondere, daß die Wandung des Körpers durch Einwirkung einer äußeren Kraft, wie beispielsweise der Flüssigkeitsdruck, verformbar ist, wobei sich die Verformung im Wesentlichen vollständig zurückbildet, wenn die äußere Kraft nicht mehr wirkt, das heißt, daß der Körper nach dem Wirken der äußeren Kraft im Wesentlichen zu seiner ursprünglichen Gestalt oder Lage zurückkehrt.

Die Flüssigkeit, welche den Körper zumindest bereichsweise umgeben kann oder welche in den Körper einfüllbar ist, kann insbesondere Wasser (sowohl Süßwasser als auch Meerwasser) und wässrige Lösungen umfassen, jedoch auch Alkohole und polare Lösungsmittel.

Die Oberflächenabdeckung kann eine Fläche bzw. Oberfläche des Körpers ganz oder teilweise bedecken. Weiter kann die Oberflächenabdeckung lösbar oder unlösbar an dem Körper befestigt sein. Insbesondere kann die Oberflächenabdeckung als eine Beschichtung des Körpers ausgebildet sein. Nach dem Befestigen der Oberflächenabdeckung an dem Körper kann die Oberflächenabdeckung als ein Teil des Körpers angesehen werden.

Die zumindest partiell gashaltende Schicht weist eine flüssigkeitszugewandte Seite und eine körperzugewandte Seite auf. Die gashaltende Schicht ist derart ausgebildet, daß bei betriebsgemäßen Gebrauch des Körpers bzw. der Oberflächenabdeckung ein Gas von der gashaltenden Schicht in Kontakt mit der gashaltenden Schicht gehalten wird, wobei die flüssigkeitszugewandte Seite der gashaltenden Schicht zumindest partiell, bevorzugt vollständig, durch das Gas von der Kontaktfläche bzw. Grenzfläche zwischen dem gehaltenen Gas und der Flüssigkeit (Flüssigkeit-Gas-Grenzfläche) beabstandet wird. Das Gas, welches durch die gashaltende Schicht gehalten wird, ist im Sinne der Anmeldung nicht Teil der gashaltenden Schicht bzw. der Oberflächenabdeckung, sondern als Teil einer gasenthaltenden Schicht, welche die gashaltende Schicht und das daran gehaltene Gas umfaßt. Dieses gehaltene Gas wird mit anderen Worten durch die gashaltende Schicht fixiert, so daß es vorteilhafterweise nicht zur Flüssigkeitsoberfläche aufsteigt oder durch eine Flüssigkeitsströmung mitgerissen wird.

Die gashaltende Schicht kann eine Basis bzw. Basislage aufweisen, welche bevorzugt Strukturelemente wie Vorsprünge, vorstehende Elemente und/oder Ausnehmungen aufweisen kann, welche insbesondere ausgelegt sind, das Gas zu halten, und welche bevorzugt mit der Basis einstückig ausgebildet sind. Die Basis kann netzförmig oder als geschlossene Lage ausgebildet sein.

Die gashaltende Schicht weist demnach auch eine Kontaktfläche zwischen dem in der Schicht enthaltenen Gas und einem festen Material auf. Die flüssigkeitszugewandte Seite bzw. die gaszugewandte Seite der gashaltenden Schicht ist hydrophob ausgebildet bzw. mit einem hydrophoben Material beschichtet. Der Begriff "hydrophob" bedeutet im Sinne dieser Anmeldung soviel wie "flüssigkeitsabweisend", also "liquidophob". Der Begriff "hydrophob" impliziert, daß es sich bei der Flüssigkeit um Wasser oder eine wässrige Lösung oder im allgemeinen um ein polares Lösungsmittel handelt.
Ob eine Oberfläche bzw. ein Material flüssigkeitsabweisend ist, läßt sich über den Kontaktwinkel eines Flüssigkeitstropfens auf einer Oberfläche des Materials bestimmen. Die Größe des Kontaktwinkels zwischen Flüssigkeit und Feststoff hängt dabei von der Wechselwirkung zwischen der Flüssigkeit und dem Feststoff an der Kontaktfläche ab. Je geringer diese Wechselwirkung ist, desto größer wird der Kontaktwinkel. Hydrophile Feststoffe schließen mit der Oberfläche der Flüssigkeit, insbesondere mit Wasser, Kontaktwinkeln von etwa 0° bis etwa 90°, insbesondere Winkel kleiner als etwa 80° ein. Kontaktwinkel von etwa 90° und mehr treten bei hydrophoben Feststoffen auf. Feststoffe, die mit der Flüssigkeit, insbesondere mit Wasser, einen Kontaktwinkel von deutlich mehr als 90° aufweisen, insbesondere Kontaktwinkel von etwa 160° und mehr werden als superhydrophob bezeichnet. Der Begriff "hydrophob" schließt somit auch den bevorzugten Fall ein, daß das Material "superhydrophob" ist.

Auf der körperzugewandten Seite der gashaltenden Schicht ist die gasdurchlässige Lage angeordnet bzw. befestigt. Alternativ kann die gasdurchlässige Lage auch mit der gashaltenden Schicht einstückig ausgebildet sein bzw. integraler Bestandteil der gashaltenden Schicht sein. Durch die flüssigkeitszugewandte Seite der gasdurchlässigen Lage, welche mit der körperzugewandten Seite der gashaltenden Schicht kontaktieren kann, ist es möglich der gashaltenden Schicht ein Gas zuzuführen. Mit anderen Worten ist die gasdurchlässige Lage für ein Gas durchlässig, insbesondere in einer Richtung welche senkrecht zur körperzugewandten Seite der gashaltenden Schicht orientiert ist.

Das der gashaltenden Schicht zugeführte Gas kann beispielsweise Luft, Stickstoff, Kohlendioxid oder ein anderes Gas sein. Das Gas kann mittels der Gaszufuhreinrichtung bereitgestellt werden, welche mit der gasdurchlässigen Lage verbunden ist. Bevorzugt kann die Gaszufuhreinrichtung eine Lage eines porösen Materials mit durchgängigem Porenraum sein, so daß Gas von der Gaszufuhreinrichtung durch die gasdurchlässige Lage zur gashaltenden Schicht strömen kann.

Vorzugsweise ist die gasdurchlässige Lage flüssigkeitsundurchlässig, insbesondere wasserundurchlässig, bzw. flüssigkeitsdicht. Vorteilhafterweise kann ein Gas von der Gaszufuhreinrichtung zur gashaltenden Schicht gegen den Flüssigkeitsdruck strömen, jedoch kann die Flüssigkeit nicht durch die gasdurchlässige Lage in Richtung Gaszufuhreinrichtung strömen, beispielsweise wenn der Flüssigkeitsdruck temporär größer ist als der Gasdruck in der Gaszufuhreinrichtung.

Vorzugsweise umfaßt die gasdurchlässige Lage ein gewebtes oder nicht gewebtes Textil, ein Flockmaterial, eine poröse Keramik, ein poröses Metall, ein Filz aus Polymer oder Metallfasern und/oder ein Metalldrahtgeflecht. Beispielsweise kann die gasdurchlässige Lage aus Polymerfasern gewebt sein oder aus einem Filz oder Vlies aus Polymerfasern bestehen. Eine gasdurchlässige Lage aus einem Polymer kann insbesondere durch Laminieren mit der gashaltenden Schicht verbunden werden. Die gasdurchlässige Lage kann alternativ oder zusätzlich ein Gewebe aus einem Metalldraht umfassen, insbesondere aus einem korrosionsbeständigen Metalldraht, beispielsweise einem nicht-rostenden Edelstahldraht, wodurch vorteilhafterweise eine hohe mechanische Reißfestigkeit und Beständigkeit gegenüber UV Strahlung und chemischen Einflüssen erreicht wird. Alternativ kann die gasdurchlässige Lage auch ein Flockmaterial, beispielsweise Polymerflocken bzw. Elastomerflocken, insbesondere aus einem geschäumten Material, umfassen. Dadurch kann das Gewicht der gasdurchlässigen Lage vorteilhafterweise reduziert werden. Weiter kann die gasdurchlässige Lage bevorzugt ein Sintermaterial, wie beispielsweise ein poröses Sintermaterial aus Metallpartikeln umfassen. Die gasdurchlässige Lage kann bevorzugt mit einem Klebemittel mit der gashaltenden Schicht verbunden werden. Alternativ können die gashaltende Schicht und die gasdurchlässige Lage durch Schweißen, insbesondere Ultraschallschweißen (ultrasonic welding), miteinander verbunden werden. Besonders bevorzugt sind die gashaltige Schicht und die gasdurchlässige Lage einstückig miteinander ausgebildet, beispielsweise durch ein (kontinuierliches) Formen bzw. Gießen aus einem Polymer.

Vorzugsweise ist die gasdurchlässige Lage als poröse, insbesondere mikroporöse oder nanoporöse, semipermeable Membran ausgebildet. Die gasdurchlässige Lage kann insbesondere als gasdurchlässige Folie aus einem Polymer ausgebildet sein. Gasdurchlässige Folien können beispielsweise eine Dicke von etwa 0,5 µm bis 5 µm aufweisen. Dadurch kann eine Oberflächenabdeckung mit geringer Dicke und geringem Gewicht bereitgestellt werden. Insbesondere können Folien aus einem Polymer durch Laminieren mit der gashaltenden Schicht verbunden werden.

Vorzugsweise ist die gasdurchlässige Lage als hydrophobe oder superhydrophobe Lage ausgebildet. Mit anderen Worten stößt die gasdurchlässige Lage Wasser und andere polare Lösungsmittel ab, wodurch vorteilhafterweise ein Eindringen dieser polaren Lösungsmittel in die gasdurchlässige Lage verhindert wird.

Vorzugsweise ist die Gaszufuhreinrichtung als gasdurchlässige Schicht ausgebildet, welche an der körperzugewandten Seite der gasdurchlässigen Lage angeordnet ist. Insbesondere kann die Gaszufuhreinrichtung auch ein poröses Material umfassen, welches durchgängige Poren aufweist. Zweckmäßigerweise ist die Gasdurchgängigkeit durch die Gaszufuhreinrichtung höher als die Gasdurchgängigkeit durch die gasdurchlässige Lage.

Vorzugsweise ist die Gaszufuhreinrichtung in Form eines Aerenchyms ausgebildet. Ein Aerenchym ist ein Durchlüftungsgewebe von Wasserpflanzen, welches einen Gastransport und eine Gasspeicherung ermöglicht. Insbesondere versteht man unter dem Begriff "Aerenchym" eine Form des pflanzlichen Grundgewebes, bei der die Interzellularräume so weit sind, daß ein regelrechtes "Durchlüftungsgewebe" entsteht. Es tritt besonders bei Sumpf- und Wasserpflanzen auf und dient dem Gaswechsel der untergetauchten Pflanzenorgane.

Mit anderen Worten ist die Gaszufuhreinrichtung als Gasspeicher ausgebildet, so daß bei steigendem Flüssigkeitsdruck Gas über die gasdurchlässige Lage zurück in die Gaszufuhreinrichtung verlagerbar ist und dort speicherbar ist, um bei einem Sinken des Flüssigkeitsdrucks wieder über die gasdurchlässige Lage in die gashaltende Schicht verlagerbar zu sein. Die Gaszufuhreinrichtung kann beispielsweise ein poröses Material aufweisen, in welchem Gas speicherbar ist. Ferner kann das Material rückstellfähig dehnbar sein, so daß sich das Volumen bei steigendem Gasdruck erhöht und durch die Rückstellkraft des Material Gas durch die gasdurchlässige Lage in die gashaltende Schicht gepreßt werden kann. Weiter bevorzugt sind die Innenwandungen der Gaszufuhreinrichtung, welche mit dem Gas kontaktieren, zumindest bereichsweise hydrophob ausgebildet. Vorteilhafterweise kann das Gas aus der gashaltenden Schicht bei Schwankungen des Flüssigkeitsdrucks in der Gaszufuhreinrichtung zwischengespeichert werden, anstatt von der gashaltenden Schicht losgelöst zu werden.

Vorzugsweise weist die gashaltende Schicht auf der flüssigkeitszugewandten Seite zumindest bereichsweise Ausnehmungen oder Mulden auf, wobei die Oberfläche der gashaltenden Schicht im Bereich der Ausnehmungen oder Mulden vorzugsweise hydrophob ist. Beispielsweise kann das Material der gashaltenden Schicht aus einem hydrophoben Material bestehen. Alternativ kann die gashaltende Schicht ein hydrophiles Material umfassen, welches bereichsweise mit einer hydrophoben Beschichtung versehen ist. Insbesondere kann die hydrophobe Beschichtung lediglich an den Wandungen der Ausnehmungen oder Mulden ausgebildet sein. Besonders bevorzugt besteht die gashaltende Schicht zumindest bereichsweise aus einem porösen Material, wobei die Ausnehmungen bzw. Mulden durch Poren gebildet werden, die mit der Oberfläche in Verbindung stehen.

Vorzugsweise weist die gashaltende Schicht auf der flüssigkeitszugewandten Seite zumindest bereichsweise Vorsprünge oder vorstehende Elemente auf, wobei die Oberfläche der gashaltenden Schicht im Bereich der Vorsprünge oder vorstehenden Elemente im Wesentlichen hydrophob ist. Zweckmäßigerweise ist der Abstand zwischen den vorstehenden Elementen derart bemessen, daß sich keine Flüssigkeitstropfen zwischen den vorstehenden Elementen anordnen können. Vorteilhafterweise werden die einzelnen Tropfen der Flüssigkeit von einer Vielzahl von vorstehenden Elementen getragen, so daß sich die Grenzfläche zwischen Flüssigkeit und dem zwischen den vorstehenden Elementen befindlichen Gas im Wesentlichen als Einhüllende der vorstehenden Elemente ausbildet. Insbesondere kann der Abstand zwischen zwei benachbarten vorstehenden Elementen etwa 50µm bis etwa 500µm, bevorzugt etwa 100 µm bis etwa 200 µm, betragen.

Vorzugsweise weisen die Vorsprünge oder vorstehenden Elemente einen zentralen Oberflächenbereich auf, der hydrophil ist und der von einem hydrophoben Oberflächenbereich der Vorsprünge oder vorstehende Elemente umgeben ist. Vorteilhafterweise wird die Grenzfläche zwischen der Flüssigkeit und dem Gas an den Bereichen, welche hydrophil ausgebildet sind, lokalisiert. Dadurch wird weiter vorteilhafterweise ein Ablösen von Gasblasen durch eine Strömung der Flüssigkeit vermieden.

Die gashaltende Schicht ist durch fluidundurchlässige Trennwände in eine Vielzahl von Teilbereichen (auch "compartments" genannt) unterteilt. Unter einem Fluid wird sowohl ein Gas, eine Flüssigkeit sowie ein Gemisch davon verstanden. Folglich verhindert die Trennwand, daß sich eine Flüssigkeitsströmung oder eine Gasströmung zwischen benachbarten Teilbereichen ausbildet. Vorteilhafterweise wird bei einem Druckunterschied zwischen zwei benachbarten Teilbereichen mittels der Trennwände verhindert, daß Gas von einem Teilbereich weg zu dem benachbarten Teilbereich strömt und dadurch der Strömungswiderstand gegenüber einer kontaktierenden Flüssigkeit lokal erhöht wird und im Gegensatz dazu aus dem Teilbereich, in den das Gas strömt, überschüssiges Gas in die Flüssigkeit abgegeben wird.

Bevorzugt können die Trennwände zusammen mit den weiteren Elementen der gashaltenden Schicht einstückig bzw. integral ausgebildet sein. Weiter bevorzugt befindet sich eine Vielzahl von hydrophoben vorstehenden Elementen in einer zweidimensionalen Anordnung in jedem der Teilbereiche der gashaltenden Schicht.

Vorzugsweise umfaßt die gashaltende Schicht ein geprägtes Kunstharz oder einen geprägten Lack. Insbesondere kann die gashaltende Schicht aus einem flüssigen Kunstharz gegossen werden, wobei bevorzugt vorstehende Elemente integral bzw. einstückig mit einer Basislage der gashaltenden Schicht und/oder mit der gasdurchlässigen Lage ausgebildet werden. Insbesondere kann die Basislage der gashaltenden Schicht identisch mit der gasdurchlässigen Lage sein.

Vorzugsweise ist die gashaltende Schicht zumindest bereichsweise mit Polytetrafluorethylen (PTFE), auch unter dem Handelsnamen Teflon bekannt, oder dessen Derivaten beschichtet. Insbesondere kann die Beschichtung auch Mikropartikel bzw. Nanopartikel aus Polytetrafluorethylen oder anderen Materialien umfassen. Vorteilhafterweise wirkt die Beschichtung aus PTFE als hydrophobe Schicht und als Antihaftmittel, so daß ein Anhaften von Flüssigkeiten oder Feststoffen an der gashaltenden Schicht verhindert wird. Bevorzugt ist die Beschichtung der gashaltenden Schicht etwa 0,15 nm bis etwa 500 nm dick.

Ein Aspekt betrifft eine Oberflächenabdeckung für einen mit einer Flüssigkeit kontaktierbaren Körper umfassend:
- eine zumindest partiell gashaltende Schicht, welche derart auslegt und angeordnet ist, um mit einer flüssigkeitszugewandten Seite zumindest bereichsweise mit der Flüssigkeit zu kontaktieren;
- zumindest eine Gasaustrittseinrichtung, welche eine Gasaustrittsöffnung an der flüssigkeitszugewandten Seite der gashaltenden Schicht aufweist;
- eine Gaszufuhreinrichtung, welche mit der Gasaustrittseinrichtung verbunden ist, wobei von der Gaszufuhreinrichtung bereitgestelltes Gas aus der Gasaustrittseinrichtung strömbar und von der gashaltenden Schicht zumindest partiell aufnehmbar ist.

Vorzugsweise durchstößt die Gasaustrittseinrichtung die gashaltende Schicht. Mit anderen Worten ist eine Gasaustrittsöffnung der Gasaustrittseinrichtung an der flüssigkeitszugewandten Seite der gashaltenden Schicht angeordnet.

Vorzugsweise ist die Gaszufuhreinrichtung als gasdurchlässige Schicht ausgebildet, welche an der körperzugewandten Seite der gasdurchlässigen Schicht angeordnet ist. Besonders bevorzugt ist die Gaszufuhreinrichtung in Form eines Aerenchyms ausgebildet. Vorteilhafterweise kann die Gaszufuhreinrichtung in einfacher Weise mit einer Vielzahl von Gasaustrittseinrichtungen fluidisch verbunden sein, wobei die Gasaustrittseinrichtungen insbesondere regelmäßig oder unregelmäßig flächig angeordnet sein können.

Weiter können bevorzugte Merkmale der gashaltenden Schicht und deren Ausnehmungen, Mulden oder vorstehende Elemente, die mit Bezug auf den vorigen Aspekt der Erfindung beschrieben sind, analog bei dieser Ausführungsform vorgesehen sein.

Ein Aspekt betrifft eine Anordnung umfassend:
- eine Oberflächenabdeckung und
- eine Gasquelle, welche mit der Gaszufuhreinrichtung der Oberflächenabdeckung fluidisch verbunden ist.

Die Gasquelle kann bevorzugt einen Kompressor, einen Druckbehälter zum Lagern von Gas, einen gaserzeugenden Reaktor oder andere Vorrichtungen umfassen, die Gas mit einem hinreichenden Gasdruck bereitstellen können, um das Gas in die gashaltende Schicht strömen zu lassen. Bevorzugt kann der gaserzeugende Reaktor eine Brennkraftmaschine sein, deren Abgase zur Aufrechterhaltung der Gasschicht genutzt werden.

Vorzugsweise umfaßt die Anordnung weiter:
- zumindest eine Sensoreinrichtung zur Bestimmung des Gasgehaltes in der gashaltenden Schicht der Oberflächenabdeckung und
- eine Regeleinrichtung, mit welcher Messdaten von der zumindest einen Sensoreinrichtung empfangbar sind und welche den Gasfluß von der Gasquelle zur Gaszufuhreinrichtung anhand der empfangenen Messdaten regelt.

Vorteilhafterweise kann mittels der Regeleinrichtung ein konstanter Gasdruck oder eine konstante Gasschichtdicke innerhalb der gashaltenden Schicht gehalten werden. Bevorzugte Sensoreinrichtung können daher Drucksensoren, Ultraschallsensoren und/oder Sensoren zur Ermittlung der Gasschichtdicke umfassen. Alternativ kann auch eine Regeleinrichtung vorgesehen sein, welche derart ausgelegt ist, um Gas in vorbestimmten Zeitabständen der gashaltenden Schicht zuzuführen.

Ein Aspekt betrifft eine Verwendung einer Oberflächenabdeckung, wobei die Oberflächenabdeckung eine Fläche eines Körpers zumindest bereichsweise abdeckt, wobei für den Fall, daß der Körper mit der durch die Oberflächenabdeckung abgedeckten Fläche zumindest bereichsweise in eine Flüssigkeit eingetaucht wird, eine Gasschicht dauerhaft die Flüssigkeit und den eingetauchten Bereich zumindest bereichsweise voneinander beabstandet.

Vorteilhafterweise kann die Flüssigkeit zumindest bereichsweise nicht benetzen, so daß der Körper vor korrosiven Flüssigkeiten geschützt wird. Weiter vorteilhafterweise kann der Körper mit geringerem Kraftaufwand relativ zur Flüssigkeit verlagert werden, da der Strömungswiderstand durch die Flüssigkeit-Gas-Grenzfläche verringert wird. Insbesondere kann es sich bei dem Körper um ein Schiff handeln, so daß aufgrund des verringerten Strömungswiderstandes zwischen Schiffswandung und Wasser vorteilhafterweise der Treibstoffverbrauch gesenkt werden kann. Weiter vorteilhafterweise schützt die Gasschicht zwischen Schiffswandung und Wasser vor Korrosion des Schiffes, insbesondere in Meerwasser, und vor Bewuchs mit Organismen, beispielsweise Algen, Muscheln, Seepocken und andere. Mit anderen Worten besitzt die Oberflächenabdeckung eine Anti-Fouling-Wirkung, wobei vorteilhafterweise auf Biozide verzichtet werden kann, deren giftige Substanzen sich über die Zeit im Wasser lösen.

Die Fläche des Körpers ist eine Wandung eines Wasserfahrzeugs oder eines im Wasser angeordneten Bauwerks oder eine Innenwandung eines Flüssigkeitsbehälters oder einer Flüssigkeitsleitung.

Ein Aspekt betrifft eine Verwendung einer Oberflächenabdeckung, wobei die Oberflächenabdeckung eine Lagerfläche eines Körpers zumindest bereichsweise abdeckt, wobei für den Fall, daß der Körper mit der durch die Oberflächenabdeckung abgedeckten Lagerfläche in eine Flüssigkeit eingetaucht wird, eine Gasschicht dauerhaft die Flüssigkeit und die Lagerfläche voneinander beabstandet, so daß ein zu lagernder zweiter Körper auf der Lagerfläche derart gelagert werden kann, daß sich zwischen dem Körper und dem zweiten Körper zumindest bereichsweise eine Gasschicht befindet.

Vorteilhafterweise kann der zweiten Körper mittels der Gasschicht im wesentlichen berührungsfrei und reibungsfrei an dem Körper gelagert werden.

Vorzugsweise ist die Fläche des Körpers eine Bohrung, deren Innenwandung die Lagerfläche ausbildet. Der zu lagernde zweite Körper ist dann bevorzugt eine Achse, so daß die Anordnung von Körper mit Oberflächenabsdeckung mit der Achse die Wirkung eines Kugellagers aufweist.

### Figurenbeschreibung

Im folgenden werden bevorzugte Ausführungsformen der Oberflächenabdeckung anhand der beigefügten Zeichnungen beispielhaft erläutert. Es zeigt:
- Figur 1:: Eine Schnittansicht durch eine bevorzugte Ausführungsform einer Anordnung einer Oberflächenabdeckung an einen Körper,
- Figur 2:: Eine weitere Ausführungsform einer Anordnung einer bevorzugten Oberflächenabdeckung an einen Körper,
- Figur 3:: Bevorzugte Ausführungsformen einer gashaltenden Schicht der Oberflächenabdeckung,
- Figur 4:: Weitere bevorzugte Ausführungsformen der gashaltenden Schicht,
- Figur 5:: Eine weitere Ausführungsform einer Oberflächenabdeckung,
- Figur 6:: Eine weitere Ausführungsform einer Oberflächenabdeckung,
- Figur 7:: Eine weitere bevorzugte Ausführungsform der gashaltenden Schicht einer Oberflächenabdeckung in einem ersten Zustand,
- Figur 8:: Die in Figur 7 gezeigte Ausführungsform in einem zweiten Zustand,
- Figur 9:: Eine weitere Ausführungsform einer gashaltenden Schicht einer bevorzugten Oberflächenabdeckung in einem ersten Zustand,
- Figur 10:: Die in Figur 9 gezeigte Ausführungsform in einem zweiten Zustand,
- Figur 11:: Eine weitere Ausführungsform einer gashaltenden Schicht,
- Figur 12:: Eine weitere Ausführungsform einer gashaltenden Schicht in verschiedenen Zuständen,
- Figur 13:: zwei Ausführungsformen einer hydrophoben, gashaltenden Faser,
- Figur 14:: zwei weitere Ausführungsformen einer hydrophoben, gashaltenden Faser,
- Figur 15:: eine weitere Ausführungsform einer hydrophoben, gashaltenden Faser,
- Figur 16a:: eine perspektivische Ansicht einer bevorzugten gashaltenden Schicht,
- Figur 16b:: eine perspektivische Ansicht einer bevorzugten gashaltenden Schicht,
- Figur 16c:: eine perspektivische Ansicht einer bevorzugten gashaltenden Schicht,
- Figur 16d:: eine perspektivische Ansicht einer bevorzugten gashaltenden Schicht,
- Figur 17a:: eine Draufsicht auf die in der Figur 16 gezeigten gashaltenden Schicht,
- Figur 17b:: eine Draufsicht auf weitere bevorzugte gashaltende Schicht,
- Figur 17c:: eine Draufsicht auf weitere bevorzugte gashaltende Schicht,
- Figur 17d:: eine Draufsicht auf weitere bevorzugte gashaltende Schicht,
- Figur 18:: eine bevorzugte gashaltende Schicht,
- Figur 19:: verschiedene Oberflächenstrukturen,
- Figur 20:: Anordnung einer gashaltenden Schicht an einer Schiffswand,
- Figur 21:: eine aus Mulden bestehende Oberflächenstruktur,
- Figur 22:: eine aus Mulden bestehende Oberflächenstruktur mit hydrophober Beschichtung,
- Figur 23:: ein Schiff, dessen Wandung mit einer Vielzahl von Fliesen mit einer gashaltenden Schicht versehen ist,
- Figur 24:: eine Oberflächenabdeckung bzw. Fliese, welche Trennwände 42 aufweist,
- Figur 25:: eine Fliese bzw. Kachel,
- Figur 26:: einen Schnitt durch eine Schiffswandung, die mit Fliesen versehen ist,
- Figur 27: System,: eine gashaltende Schicht als einstufiges System und zweistufiges
- Figur 28:: eine gashaltende Schicht, welche durch eine raue Oberfläche ausgebildet ist,
- Figur 29:: eine gashaltende Schicht, welche durch eine raue Oberfläche ausgebildet ist,
- Figur 30:: eine gashaltende Schicht, welche an einem fadenförmigen Element ausgebildet ist,
- Figur 31:: eine gashaltende Schicht, welche an einem fadenförmigen Element ausgebildet ist,
- Figur 32:: eine gashaltende Schicht, welche an einem fadenförmigen Element ausgebildet ist,
- Figur 33:: eine gashaltende Schicht, welche an einem fadenförmigen Element ausgebildet ist.

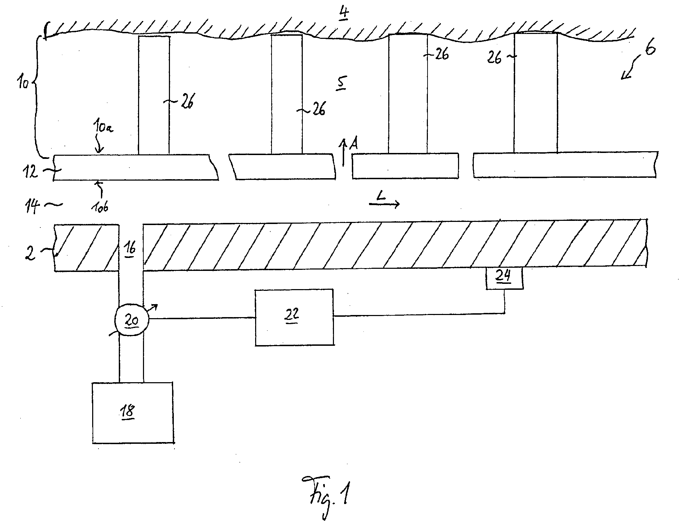

Die **Figur 1** zeigt eine Wandung 2 eines Körpers, welcher ausgelegt ist zumindest bereichsweise in eine Flüssigkeit 4 eingetaucht zu werden. An der flüssigkeitszugewandten Seite der Wandung 2 ist zumindest bereichsweise eine Oberflächenabdeckung 6 an der Wandung 2 angeordnet bzw. befestigt.

Die Oberflächenabdeckung 6 umfaßt eine zumindest partiell gashaltende Schicht 10, welche mit einer flüssigkeitszugewandten Seite 10a zumindest bereichsweise die Flüssigkeit 4 kontaktiert. Weiter umfaßt die Oberflächenabdeckung 6 eine gasdurchlässige Lage 12, welche an einer der flüssigkeitszugewandten Seite 10a gegenüberliegenden Körper zugewandten Seite 10b der gashaltenden Schicht 10 angeordnet bzw. befestigt ist. In der in Figur 1 gezeigten bevorzugten Ausführungsform sind die gashaltende Schicht 10 und die gasdurchlässige Lage 12 einstückig bzw. integral ausgebildet. Es versteht sich jedoch, daß die gashaltende Schicht 10 und gasdurchlässige Lage 12 voneinander getrennt ausgebildet miteinander verbunden bzw. miteinander befestigt sein können.

Weiter umfaßt die Oberflächenabdeckung eine Gaszufuhreinrichtung 14, welche mit der gasdurchlässigen Lage 12 verbunden ist. Mit anderen Worten sind die Gaszufuhreinrichtung 14 und die gasdurchlässige Lage 12 zumindest fluidisch miteinander derart verbunden, daß Gas von der Gaszufuhreinrichtung 14 durch die gasdurchlässige Lage 12 zur gashaltenden Schicht 10 strömen kann. In der in Figur 1 gezeigten Ausführungsform ist die Gaszufuhreinrichtung 14 als ein Gas leitender Kanal ausgebildet, welcher zwischen der gasdurchlässigen Lage 12 und der Wandung 2 angeordnet ist. Bevorzugt kann die Gaszufuhreinrichtung 14 auch als eine gasdurchlässige, insbesondere poröse, Schicht ausgebildet sein, wobei der Porenraum der Gaszufuhreinrichtung besonders bevorzugt durchgängige Poren aufweist, so daß Gas entlang einer Längsrichtung L durch die Gaszufuhreinrichtung strömen kann. Dadurch kann die Gaszufuhreinrichtung vorteilhafterweise flächig an der gasdurchlässigen Lage 12 Gas bereitstellen, welches dann vorzugsweise entlang einer Ausströmrichtung A, welche im Wesentlichen senkrecht zur Längsrichtung L orientiert sein kann, durch die gasdurchlässige Lage 12 in die gashaltende Schicht 10 strömt. Weiter bevorzugt kann die Gaszufuhreinrichtung 14 dazu dienen, die gasdurchlässige Lage 12 sowie die damit verbundene gashaltende Schicht 10 mit der Wandung 2 zu verbinden. Beispielsweise können die Gaszufuhreinrichtung 14 und die gasdurchlässige Lage 12 miteinander mechanisch verbunden sein, beispielsweise durch Verkleben oder Laminieren, so daß die Oberflächenabdeckung 6 durch ein Befestigen einer körperzugewandten Seite der Gaszufuhreinrichtung 14 mit der Wandung 2 an dem Körper befestigt sein kann. Weiter bevorzugt können die gasdurchlässigen Lage 12 und die Gaszufuhreinrichtung 14 miteinander einstückig bzw. integral ausgebildet sein. Besonders bevorzugt sind die gashaltende Schicht 10, die gasdurchlässige Lage 12 und die Gaszufuhreinrichtung 14 zusammen einstückig ausgebildet. Die Gaszufuhreinrichtung 14 ist mittels eines Gaszufuhreinrichtungkanals 16 fluidisch mit einer Gasquelle 18 verbunden, wobei die Menge des in die Gaszufuhreinrichtung 14 fließenden Gases mittels eines Ventils 20 und einer damit verbundenen Regeleinrichtung 22 geregelt bzw. gesteuert werden kann. Die in der Figur 1 gezeigte bevorzugte Ausführungsform umfaßt weiter eine Sensoreinrichtung 24, welche ausgelegt ist, den Gasgehalt in der gashaltenden Schicht 10 der Oberflächenabdeckung 6 zu bestimmen. Dies kann beispielsweise mittels der Reflexion eines Ultraschallsignals oder einer elektromagnetischen Welle erfolgen. Bevorzugt erfolgt die Messung des Gasgehaltes in der gashaltenden Schicht 10 durch die Sensoreinrichtung 24 berührungsfrei bzw. kontaktlos, so daß die Sensoreinrichtung 24 nicht derart angeordnet werden muß, daß die Sensoreinrichtung 24 von der Flüssigkeit 4 benetzt wird. Insbesondere kann die Sensoreinrichtung 24 auf einer flüssigkeitsabgewandten Seite der Wandung 2 angeordnet sein, wobei die Messung des Gasgehaltes bevorzugt durch die Wandung 2 hindurch erfolgen kann. Anhand der von der Sensoreinrichtung 24 gemessenen Gasmenge innerhalb der gashaltenden Schicht 10 der Oberflächenabdeckung 6 bestimmt die Regeleinrichtung 22 die Gasmenge, welche über die Gaszufuhreinrichtung 14 der gashaltenden Schicht 10 zuzuführen ist, um die Gasmenge in der gashaltenden Schicht 10 bzw. die Dicke der Gasschicht in der gashaltenden Schicht 10 und damit den Abstand zwischen der Kontaktfläche Flüssigkeit-Gas zu der Wandung 2 konstant zu halten.

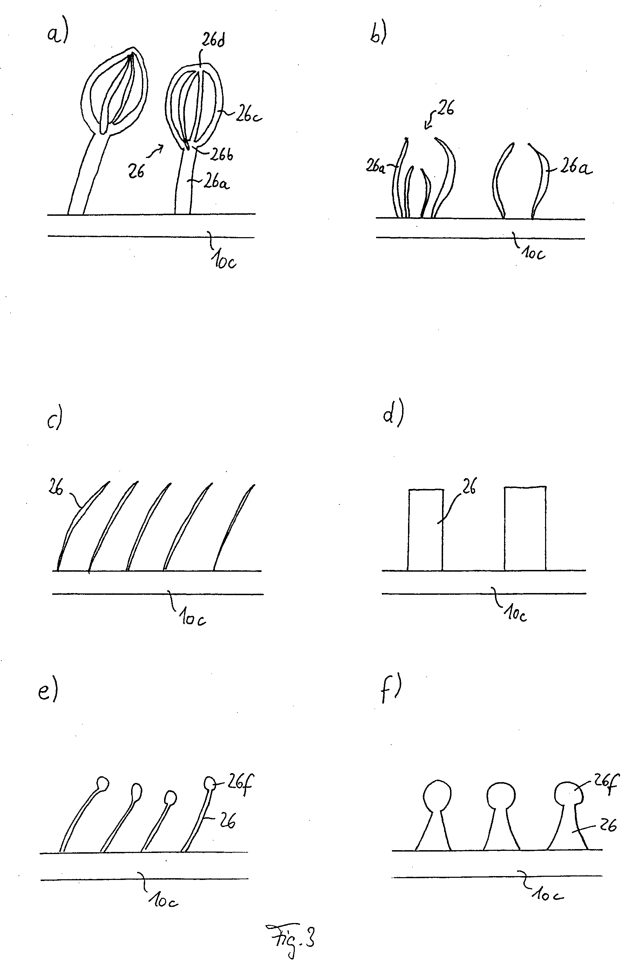

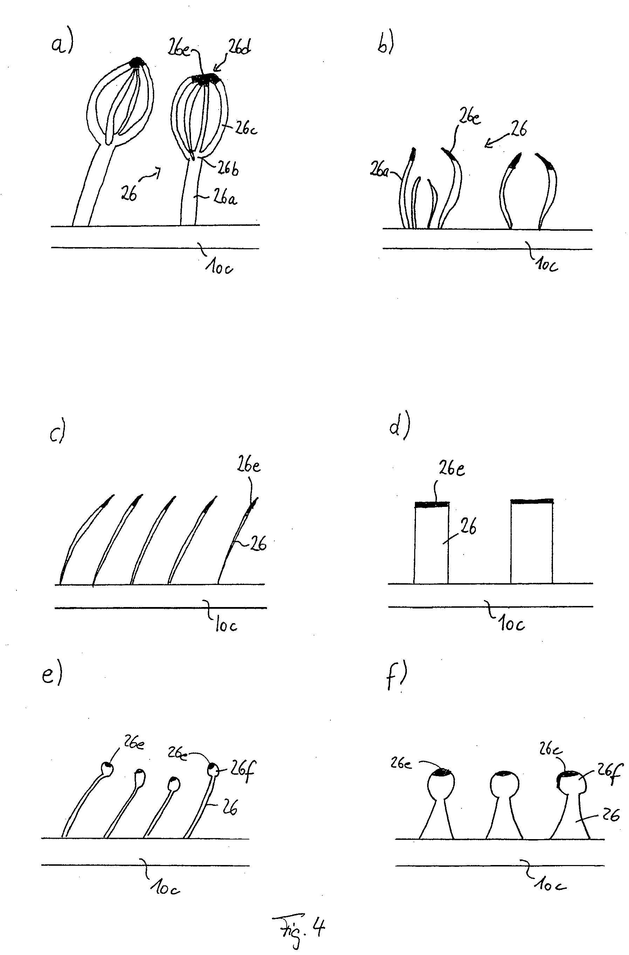

Die gashaltende Schicht 10 umfaßt eine Vielzahl von vorstehenden Elementen 26, welche aus einem hydrophoben Material bestehen oder mit einem hydrophoben Material beschichtet sind. Die vorstehenden Elemente 26 können in regelmäßigem oder unregelmäßigem Abstand entlang der Längsrichtung L an der gashaltenden Schicht 10 angeordnet sein. Die vorstehenden Elemente 26 halten in den dazwischen liegenden Volumen das über die gasdurchlässige Lage 12 austretende Gas, so daß die Flüssigkeit 4 im Wesentlichen nicht in die Volumen, welche zwischen den vorstehenden Elementen 26 ausgebildet sind, eindringen kann. Insbesondere wird die Flüssigkeit 4 daran gehindert, die gasdurchlässige Lage 12 zu benetzen. Die in der Figur 1 gezeigten vorstehenden Elemente 26 weisen eine Türmchenstruktur auf, welche vorteilhafterweise besonders einfach herzustellen ist. Es versteht sich jedoch, daß auch andere Ausgestaltungen der vorstehenden Elemente 26 zu dem gewünschten Effekt führen kann. Weitere vorteilhafte Ausbildungen der vorstehenden Elemente 26 sind in den Figuren 3 und 4 gezeigt.

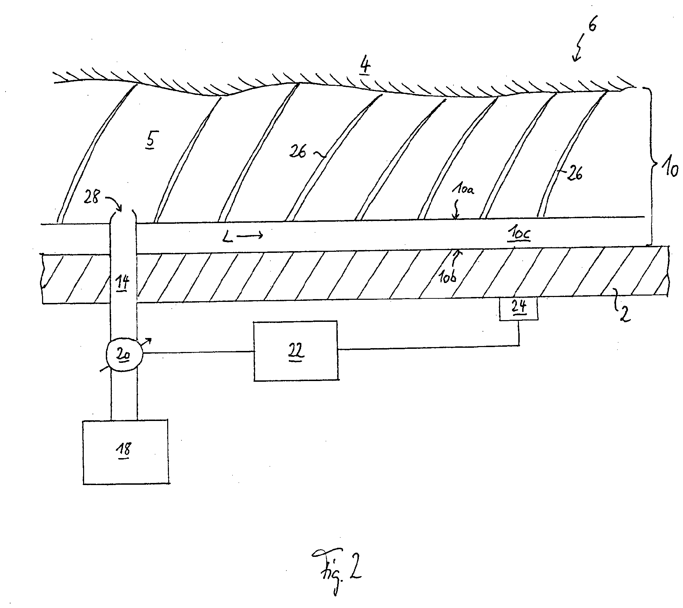

Die **Figur 2** zeigt einen Schnitt durch eine weitere Ausführungsform einer Anordnung von einer Oberflächenabdeckung 6 an der Wandung 2 eines Körpers. Die gashaltende Schicht 10 umfaßt eine Vielzahl von vorstehenden Elementen 26 in Form von dünnen Härchen mit einem Durchmesser von etwa 1 µm bis etwa 100 µm, welche aus einem hydrophoben Material ausgebildet sind. Die vorstehenden Elemente 26 der gashaltenden Schicht 10 sind einstückig mit einer Basis 10c der gashaltenden Schicht 10 verbunden. Die körperzugewandte Seite 10b der gashaltenden Schicht 10 ist an der Wandung 2 des Körpers angeordnet bzw. damit befestigt. Das Befestigen der gashaltenden Schicht 10 an die Wandung 2 kann beispielsweise durch ein Verkleben mittels eines Klebemittels erfolgen. Weiter bevorzugt kann die gashaltende Schicht 10 auch dadurch ausgebildet werden, daß ein im Wesentlichen flüssiges Material auf die Wandung 2 aufgetragen wird und dort in Form der gashaltenden Schicht 10 erstarrt.

Die in der Figur 2 gezeigte Ausführungsform umfaßt eine Gasaustrittseinrichtung 28, welche mittels eines als Kanal ausgebildeten Gaszufuhreinrichtung 14 mit einer regelbaren Gasquelle 18 fluidisch verbunden ist. Das Regeln der Gasmenge, welche durch die Gasaustrittseinrichtung 28 der gashaltenden Schicht 10 zugeführt wird, wird durch das Ventil 20, die Regeleinrichtung 22 und bevorzugt durch die Sensoreinrichtung 24 geregelt bzw. besteuert. Die Steuerung bzw. Regelung erfolgt dabei in Analogie zu der mit Bezug auf Figur 1 beschriebenen Ausführungsform.

Mittels der Gasaustrittseinrichtung 28 wird das Gas in die zwischen den vorstehenden Elementen 26 ausgebildeten Zwischenräume geleitet. Es versteht sich, daß das aus der Gasaustrittseinrichtung 28 austretende Gas entlang der Längsrichtung L durch die zwischen den vorstehenden Elementen 26 ausgebildeten Zwischenräume strömen kann. Da die in der Figur 2 gezeigte Ausführungsform einer Gasquelle 18 aufweist, welche auf einer flüssigkeitsabgewandten Seite der Wandung 2 angeordnet ist, durchstößt die Gaszufuhreinrichtung 14 die Wandung 2 an zumindest einer Stelle. Es versteht sich, daß die Gaszufuhreinrichtung 14 sowohl auf der flüssigkeitszugewandten Seite der Wandung 2 als auch auf der flüssigkeitsabgewandten Seite der Wandung 2 sich entlang der Längsrichtung L erstrecken kann, um eine Vielzahl von Gasaustrittseinrichtungen 28 zu speisen. Dementsprechend kann die Wandung 2 eine Vielzahl von Durchtrittsöffnungen aufweisen, wobei jeder Durchtrittsöffnung der Wandung 2 eine Gasaustrittseinrichtung 28 zugeordnet ist. Somit kann vorteilhafterweise die gashaltende Schicht 10 flächig mit Gas beschickt werden.

Die **Figuren 3a bis 3f** zeigen verschiedene Formen der vorstehenden Elemente 26. Beispielsweise können die vorstehenden Elemente eine Gestalt aufweisen, welche den Haaren an den Blättern des Schwimmfarns Salvinia molesta entspricht. Diese vorstehenden Elemente 26 umfassen einen Stamm 26a, welcher von der Basis 10c im Wesentlichen senkrecht absteht, und von dessen Stammkopf 26b sich eine Vielzahl von Ästen 26c erstreckt, die voneinander divergieren und mit ihren Enden an einem gemeinsamen Kopfpunkt 26d zusammengeführt sind. Aufgrund des Aussehens dieses vorstehenden Elements 26 wird dieses auch als Schneebesenform benannt.

Die Figur 3b zeigt eine alternative Ausgestaltung der vorstehenden Elemente in Krönchenform, welche 1, 2 oder mehr Paare konkav konvex geformter Stämme 26a aufweisen, welche mit einem ersten Endbereich mit der Basis 10c verbunden sind und mit einem gegenüber liegenden Endbereich sich entlang einer Richtung im wesentlichen senkrecht zu Basis 10c erstrecken, wobei der Abstand zwischen diesen Endpunkten geringer ist als der Abstand zwischen den Stämmen 26a in der Stammitte.

Die Figur 3c zeigt eine Vielzahl von vorstehenden Elementen 26 in Form dünner Härchen, welche einen Durchmesser von etwa 1 µm bis etwa 100 µm, bevorzugt von etwa 10 µm bis etwa 50 µm aufweisen können.

Die Figur 3d zeigt vorstehende Elemente 26 in Form von Türmchen, welche in der Schnittansicht eine im Wesentlichen rechteckige Form aufweisen. Die Türmchen können aber auch im Wesentlichen zylindrisch, oval, prismatisch oder ähnlich geformt sein.

Die Figur 3e zeigt vorstehende Elemente 26 in Form eines Härchens, welches ein kugelförmiges Kopfende 26f an dem der Basis 10c entgegengesetzten Ende des Härchens 26 aufweist.

Die Figur 3f zeigt vorstehende Elemente 26, welche im Wesentlichen die Form eines Kegelstumpfes aufweisen, welcher ein kugelförmiges Kopfende 26f an der Spitze des Kegelstumpfes aufweist. Es versteht sich, daß bei den in den Figuren 3e und 3f gezeigten Ausführungsformen auch ein Kopfende in Form eines Rotationsellipsoids vorgesehen sein kann.

Die **Figuren 4a bis 4f** zeigen modifizierte vorstehende Elemente 26, welche im Wesentlichen den in den Figs. 3a bis f gezeigten vorstehenden Elementen entsprechen. Jedoch weisen die vorstehenden Elemente 26 in den in den Figs. 4a bis d gezeigten Ausführungsformen einen hydrophilen Oberflächenbereich 26e auf. Bevorzugt ist der hydrophile Oberflächenbereich 26e an einen zentralen Bereich der Oberfläche der vorstehenden Elemente 26 angeordnet bzw. ausgebildet. Insbesondere ist der hydrophile Oberflächenbereich 26e von einem hydrophoben Oberflächenbereich der vorstehenden Elemente 26 umgeben. Vorteilhafterweise ist der hydrophile Oberflächenbereich 26e geeignet, die Kontaktfläche zwischen dem in der gashaltenden Schicht 10 gehaltenen Gas und der kontaktierenden Flüssigkeit 4 an dem hydrophilen Oberflächenbereich 26 zu lokalisieren. Dadurch kann weiter vorteilhafterweise ein Abreißen der Kontaktfläche zwischen Gas und Flüssigkeit an diesen Stellen verhindert werden, so daß der Gasverlust aus der gashaltenden Schicht 10 verringert werden kann.

Die Figur 4e zeigt vorstehende Elemente 26 in Form eines Härchens, welches ein kugelförmiges Kopfende 26f an dem der Basis 10c entgegengesetzten Ende des Härchens 26 aufweist, wobei ein hydrophiler Bereich 26e an dem kugelförmigen Kopfende ausgebildet ist. Es versteht sich, daß das kugelförmige Kopfende auch vollständig hydrophil ausgebildet sein kann.

Die Figur 4f zeigt vorstehende Elemente 26, welche im Wesentlichen die Form eines Kegelstumpfes aufweisen, welcher ein kugelförmiges Kopfende 26f an der Spitze des Kegelstumpfes aufweist, wobei ein hydrophiler Bereich 26e an dem kugelförmigen Kopfende ausgebildet ist. Es versteht sich, daß das kugelförmige Kopfende auch vollständig hydrophil ausgebildet sein kann. Es versteht sich weiter, daß bei den in den Figuren 4e und 4f gezeigten Ausführungsformen auch ein Kopfende in Form eines Rotationsellipsoids vorgesehen sein kann.

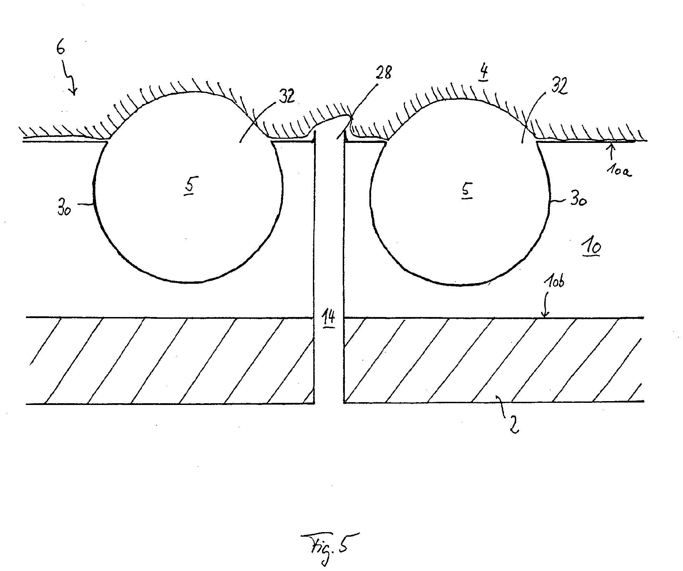

Die **Figur 5** zeigt einen Schnitt durch eine weitere Ausführungsform der Oberflächenabdeckung 6, welche an einer Wandung 2 angeordnet ist. Ähnlich zu der in der Figur 2 gezeigten Ausführungsform umfaßt die in der Figur 5 gezeigte Ausführungsform eine Gasaustrittseinrichtung 28, welche mit einer durch die Wandung 2 durchtretenden Gaszufuhreinrichtung 14 verbunden ist. Die gashaltenden Schicht 10, welche mit einer körperzugewandten Seite 10b an der Wandung 2 angeordnet wird bzw. befestigt ist, umfaßt anstelle der vorstehenden Elemente Mulden 30. Die Mulden 30 umfassen eine Durchtrittsöffnung in der flüssigkeitszugewandten Seite der gashaltenden Schicht 10, wobei der Durchmesser der Durchtrittsöffnung 32 bevorzugt kleiner ist als der Durchmesser der Mulden 30. Insbesondere können die Mulden 30 im Wesentlichen kugelförmig ausgebildet sein. Es versteht sich jedoch, daß die Mulden 30 auch eckig bzw. oval ausgebildet sein können.

Das Material der gashaltenden Schicht 10, in welcher die Mulden 30 ausgebildet sind, ist ein hydrophobes Material. Es versteht sich jedoch, daß die Innenwandung der Mulden 30 in der gasenthaltenden Schicht 10 auch mit einem hydrophoben Material beschichtet sein können.

Das durch die Gasaustrittseinrichtung 28 austretende Gas kann in den Mulden 30 der gasenthaltenden Schicht 10 gespeichert bzw. gehalten werden. Aufgrund der Oberflächenspannung der Kontaktfläche zwischen Gas und der kontaktierenden Flüssigkeit 4 steht die Kontaktfläche zwischen Gas und Flüssigkeit gegenüber der Oberfläche des festen Materials der gashaltenden Schicht 10, also über die Fläche der Durchtrittsöffnung 32, hervor. Dadurch wird ein Gaspolster zwischen der Flüssigkeit 4 und der Wandung 2 bzw. dem festen Material der gashaltenden Schicht 10 zumindest im Bereich der Mulden 30 ausgebildet.

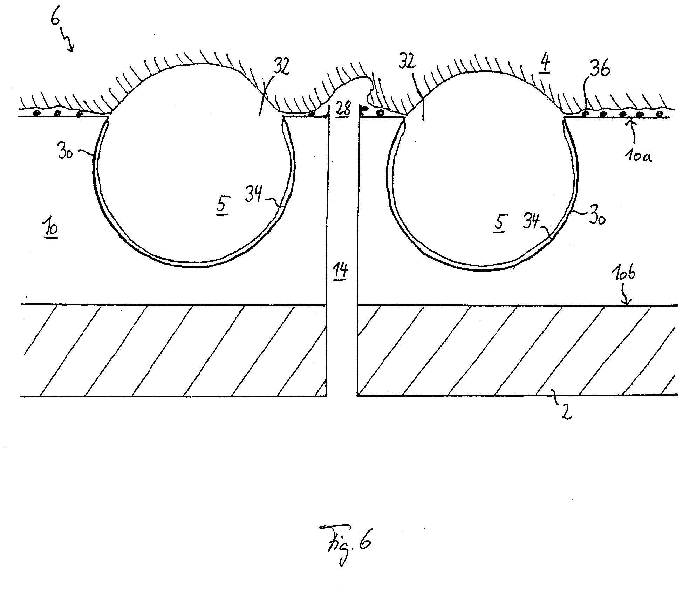

Die **Figur 6** zeigt eine modifizierte Form der in Figur 5 gezeigten Ausführungsform, wobei identische Elemente mit identischen Bezugszeichen versehen sind. Die in der gashaltenden Schicht 10 ausgebildeten Mulden 30 weisen auf der Innenwandung der Mulden 30 eine hydrophobe Beschichtung 34 auf, so daß das feste Material der gashaltenden Schicht nicht vollständig aus dem hydrophoben Material bestehen muß. Die Kontaktfläche zwischen Gas und Flüssigkeit bildet sich analog zu der in Figur 5 gezeigten Ausführungsform aus.

Um den Strömungswiderstand einer Flüssigkeitsströmung entlang der Längsrichtung L an der Kontaktfläche zwischen der Flüssigkeit und dem festen Material der gashaltenden Schicht 10 weiter zu minimieren, kann die gashaltende Schicht 10 zumindest bereichsweise eine Oberflächenbeschichtung 36 mit einem haftungsmindernden Material aufweisen. Die Oberflächenbeschichtung kann beispielsweise Mikro- oder Nanopartikel umfassen, welche eine definierte Oberflächenrauigkeit im Bereich von etwa 10 nm bis etwa 10 µm ausbildet. Weiter kann die Oberflächenbeschichtung auch eine durchgehende Beschichtung mit Teflon oder Nanoteflon aufweisen, welche eine Schichtdicke von etwa 0,15 nm bis etwa 500 nm aufweisen kann. Weiter bevorzugt kann die Oberflächenbeschichtung 36 zur Ausbildung einer Oberflächenstruktur Partikel aus Polymeren, PDMS, Silicium, Siliciumdioxid, Siliciumhydroxid, Metallen, insbesondere Stahl und Stahlfasern, und Epoxidharzen umfassen.

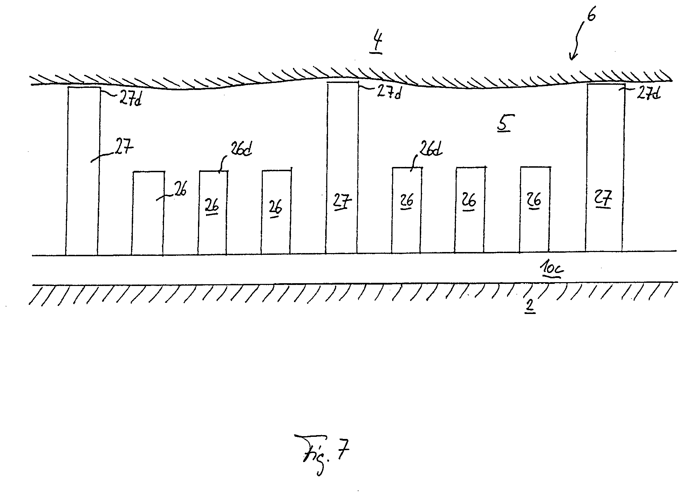

Die **Figur 7** zeigt eine Struktur aus gleichartigen vorstehenden Elementen 26, 27, wobei die vorstehenden Elemente 26, 27 unterschiedliche Größen aufweisen. So können die vorstehenden Elemente 27 um einen Faktor von etwa 1,1 bis etwa 2 weiter von der Basis 10c in Richtung der Flüssigkeit 4 vorstehen als die vorstehenden Elemente 26. Dadurch ergeben sich vorteilhafterweise zwei Strukturierungsebenen der Flüssigkeit-Gas-Grenzfläche. Vorteilhafterweise wird durch diese Strukturen ein vollständiges Entfernen des Gases, welches in der gashaltenden Schicht 10 gehalten wird, in Bereichen der gashaltenden Schicht 10 vermieden.

Bei niedrigen Strömungsgeschwindigkeiten der Flüssigkeit 4 entlang der Längsrichtung L bzw. bei einem geringen Überdruck der Flüssigkeit 4 im Vergleich zum Gasdruck innerhalb der gashaltenden Schicht 10 verläuft die Flüssigkeitgasgrenze im Wesentlichen in Form einer einhüllenden der Kopfbereiche 27d der vorstehenden Elemente 27, welche weiter von der Wandung 2 wegragen als die vorstehenden Elemente 26. Dadurch ergibt sich vorteilhafterweise eine im Wesentlichen durchgehende Luftschicht, welche im Wesentlichen lediglich von den vorstehenden Elementen 27 getragen bzw. gestützt wird. Vorteilhafterweise wird die Reibung zwischen Flüssigkeit 4 und der Wandung 2 stark vermindert, beispielsweise auf einen Wert kleiner als 5% des Reibungswertes ohne Gas in der gashaltenden Schicht 10. Jedoch neigt die gashaltende Schicht 10 in diesem Zustand leicht zu einem Gasverlust bei Druckschwankungen zwischen dem in der gashaltenden Schicht gehaltenen Gas 5 und der angrenzenden Flüssigkeit 4. Mit anderen Worten ist die zum Entweichen von Gasbläschen bzw. zum Losreißen von Gasbläschen notwendige Kraft pro Fläche bzw. Aktivierungsenergie pro Fläche relativ niedrig.

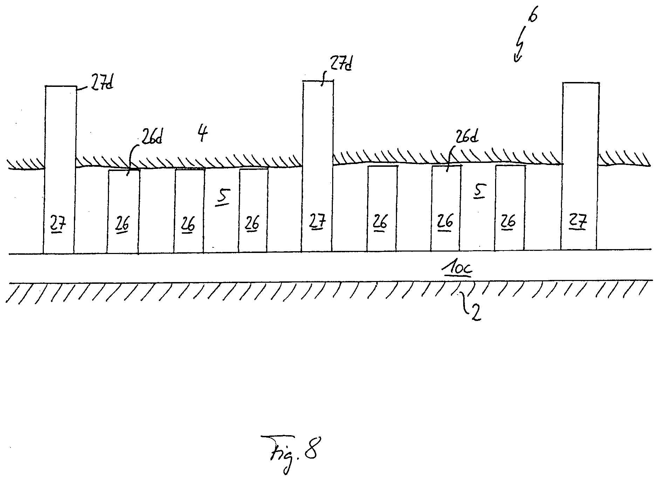

Die **Figur 8** zeigt die in der Figur 7 gezeigte Ausführungsform nach einem aufgetretenen Gasverlust aus der gashaltenden Schicht 10. Die Flüssigkeitsgasgrenze folgt nun im Wesentlichen einer einhüllenden der vorstehenden Elemente 26, welche weniger weit in Richtung der Flüssigkeit 4 vorragen. Die einzelnen mit Gas gefüllten Bereiche der gashaltenden Schicht 10, welche durch die Zwischenräume zwischen den vorstehenden Elementen 26 gebildet werden, sind durch die vorstehenden Elemente 27 begrenzt, welche nun bereichsweise in die Flüssigkeit 4 hineinragen. Insbesondere kann ein Gasaustausch zwischen einzelnen durch die vorstehenden Elemente 27 ausgebildeten Bereiche durch die vorstehenden Elemente 27 verhindert werden.

Die Reduktion des Strömungswiderstandes ist in diesem Zustand immer noch bedeutsam, jedoch im Vergleich zu dem in Figur 7 gezeigten Zustand erheblich reduziert. Jedoch ist die Basis 10c immer noch im Wesentlichen vollständig, das heißt, zu etwa 90% bis etwa 98%, durch das Gas 5 in der gashaltenden Schicht 10 von der Flüssigkeit 4 räumlich getrennt. Vorteilhafterweise ist die notwendige Kraft, um weiteres Gas 5 aus der gashaltenden Schicht 10 durch das Strömen der Flüssigkeit 4 zu entfernen, in dem in Figur 8 gezeigten Zustand größer als in dem in Figur 7 gezeigten Zustand. Dadurch wird vorteilhafterweise nach einem anfänglichen Gasverlust aus der gashaltenden Schicht 10 verhindert, daß sich größere Mengen Gas aus der gashaltenden Schicht lösen.

Bei noch weiterem Gasverlust aus der gashaltenden Schicht 10, beispielsweise durch sehr hohe Druckschwankungen zwischen dem Gas und der angrenzenden Flüssigkeit 4 verbleibt ein geringes Gasvolumen in den hydrophoben Nischen zwischen den vorstehenden Elementen 26. Dadurch wird zwar die reibungsvermindernde Wirkung der Gasschicht bezüglich des Strömungswiderstands einer entlang der Längsrichtung L strömenden Flüssigkeit 4 vermindert, jedoch sind vorteilhafterweise lediglich bis etwa 10% der Oberfläche der Basis 10c in direktem Kontakt mit der Flüssigkeit 4, so daß sich immer noch eine im wesentlichen vollständige Trennung der Wandung 2 bzw. der Basis 10c und der Flüssigkeit 4 ergibt.

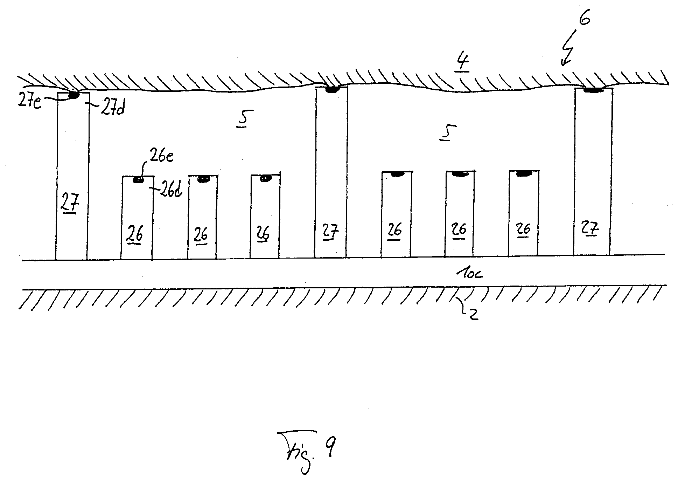

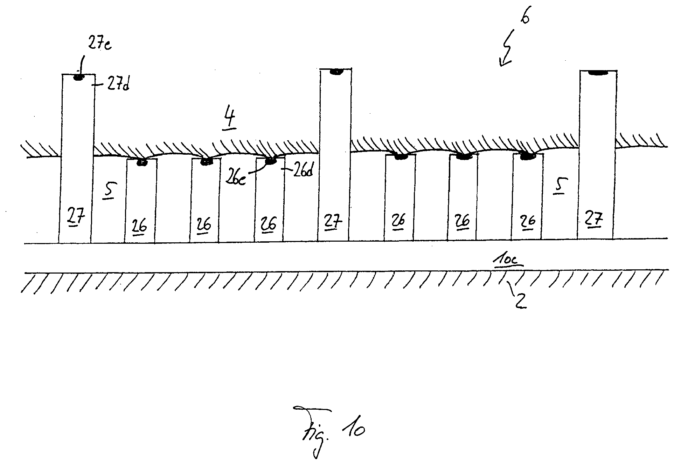

Die **Figuren 9** **und** **10** zeigen eine modifizierte Ausführungsform der in den Figuren 7 und 8 gezeigten Oberflächenabdeckung 6. Daher sind identische Elemente mit identischen Bezugszeichen gekennzeichnet. Zusätzlich umfassen die vorstehenden Elemente 26, 27 hydrophile Oberflächenbereiche 26e, 27e, welche ein verbessertes Anhaften der Grenzfläche zwischen Flüssigkeit und Gas an den zugeordneten vorstehenden Elementen 26, 27, insbesondere an deren Kopfbereichen 26d, 27d, ermöglicht. Dieses lokale Festlegen der Gasflüssigkeitsgrenze wird auch als "pinning" bezeichnet, so daß die hydrophilen Oberflächenbereiche 26e, 27e auch als "pinning-Zentren" bezeichnet werden können. Vorteilhafterweise wird durch das Vorsehen der hydrophilen Oberflächenbereiche 26e, 27e die notwendige Kraft pro Fläche zum Ablösen von Gasbläschen aus der gashaltenden Schicht 10 vergrößert, so daß die Gasverluste aus der gashaltenden Schicht 10 vermindert werden können. Weiter vorteilhafterweise ergibt sich dadurch ein verringerter Strömungswiderstand an der gashaltenden Schicht 10 und eine verbesserte Trennung zwischen der Flüssigkeit 4 und der Basis 10c bzw. der Wandung 2.

Weiter bevorzugt können weitere hydrophile Oberflächenbereiche (nicht gezeigt) zwischen den vorstehenden Elementen 26e, 27e an der Basis 10c ausgebildet sein. Diese hydrophilen Oberflächenbereiche verhindern ein Ablösen bzw. Wegströmen von Gas, welches sich in dem an die Basis 10c angrenzenden Bereich angesammelt hat. Dieses an der Basis 10c angesammelte Gas dient vorteilhafterweise als letztes, nur schwer entfernbares Gasreservoir, welches insbesondere etwa 60% bis etwa 98% der Oberfläche der Basis 10c bzw. der Wandung 2 bedeckt und dadurch die bedeckte Oberfläche von der Flüssigkeit 4 trennt. Dadurch wird vorteilhafterweise auch durch ein geringes Gasvolumen bewirkt, daß die Oberfläche der Wandung 2 im Wesentlichen vollständig vor Oxidation bzw. Korrosion geschützt ist. Weiter vorteilhafterweise wirken die an der Basis 10c verbleibenden Gasreste als Keimzentren für den Wiederaufbau der Gasschicht mittels der Gaszufuhreinrichtung (wie beispielsweise in den Figuren 1, 2, 5 und 6 gezeigt).

Es versteht sich, daß auch drei oder mehr hierarchische Strukturen von vorstehenden Elementen in der gashaltenden Schicht 10 ausgebildet sein können. Mit anderen Worten können zusätzlich zu den in den Figuren 7 bis 10 gezeigten vorstehenden Elementen 26, 27 weitere vorstehende Elemente vorgesehen sein, welche weiter als die vorstehenden Elemente 27 in Richtung der Flüssigkeit 4 von der Basis 10 vorragen.

Es versteht sich, daß die vorstehenden Elemente 26, 27 regelmäßig, quasi regelmäßig oder zufällig räumlich in der gashaltenden Schicht 10 verteilt sein können. Zudem können an verschiedenen Stellen Mulden bzw. Vertiefungen vorgesehen sein, die als Gastaschen dienen, das heißt als Gasreservoir. Gleichermaßen geeignet wie Oberflächenbeschichtungen bestimmter Rauigkeit sind auch poröse Oberflächen, deren an der Oberfläche liegende Poren als Mulden bzw. Gastaschen dienen können. Beispielsweise können auch die Mulden 30 der Figs. 5 und 6 dadurch ausgebildet sein, daß die Basis c aus einem porösen Material besteht, wobei die Poren im Bereich der Durchtrittsöffnungen 32 mit dem äußeren der Basis 10c in Verbindung stehen.

Bei einer Strukturierung der vorstehenden Elemente 26, 27 auf mehreren Längenskalen des Vorstehens in Richtung der Flüssigkeit 4 ist es besonders bevorzugt, wenn die längeren vorstehenden Elemente, welche in den Figuren 7 und 9 die Position der Flüssigkeit-Gas-Grenzfläche bestimmen, auch eine kleinere Flächendichte (Anzahl der vorstehenden Elemente 27 je Quadratzentimeter) aufweisen. Die vorstehenden Elemente 26, welche wie in den Figs. 8 und 10 gezeigt, die Flüssigkeit-Gas-Grenzfläche für den Fall bestimmen, daß die gashaltende Schicht bereits eine nicht unerhebliche Menge Gas verloren hat, weisen demgegenüber bevorzugt eine größere Flächendichte auf. Weiter bevorzugt sind diese vorstehenden Elemente 26 auch kleiner im Durchmesser und, sofern vorhanden, kleiner in der Größe bzw. dem Durchmesser der hydrophilen Oberflächenbereiche.

Bevorzugt können die vorstehenden Elemente 26, 27, welche aus einem superhydrophoben Material bestehen oder eine hydrophobe bzw. superhydrophobe Oberfläche aufweisen, dadurch mit hydrophilen Oberflächenbereichen versehen werden, daß die Oberfläche der vorstehenden Elemente 26, 27 durch Aufbringen oder Aufwachsen von nanopartikulärem Material aufzubringen und anschließend oder gleichzeitig die so entstandenen nanoraue Oberfläche mit hydrophoben, unpolaren Endgruppen oder Tetrafluorethylengruppen oder durch Adsorption von Tetarfluorethylen basierten Molekülen oder anderen apolaren oder hydrophoben Molekülen oder Fetten oder organischen oder anorganischen Ölen hydrophob zu machen. Anschließend lassen sich diese hydrophoben Bereiche gezielt durch Wechselwirkung mit einem Plasma weniger hydrophob machen. Die Bereiche, deren Hydrophobisierung reduziert wird, mit anderen Worten die hydrophil funktionalisiert werden, können beispielsweise Spitzen, Haarenden, höchste Erhebungen, hervorstehende Ecken, Spitzen und Kanten, raue Oberflächen und ähnliches sein.

Für den Fall, daß die Oberflächen der vorstehenden Elemente 26, 27 elektrisch leitfähig sind, beispielsweise bei Verwendung elektrisch leitfähiger Polymere zur Ausbildung der vorstehenden Elemente 26, 27, so läßt sich zur Reduktion der Hydrophobisierung eine elektrische Entladung nutzen, die aufgrund des Spitzeneffekts exakt an den Spitzen und an den stärksten Krümmungen und am meisten hervorragenden Oberflächenpunkten bevorzugt stattfindet, also genau dort, wo die hydrophilen Oberflächenbereiche bevorzugt angeordnet sein sollen. Mittels der Entladung wird die hydrophobe Schutzschicht an den Entladungsstellen lokal zerstört, so daß in Relation zu den hydrophoben Bereichen der vorstehenden Elemente 26, 27 relativ hydrophile Oberflächenbereiche entstehen.

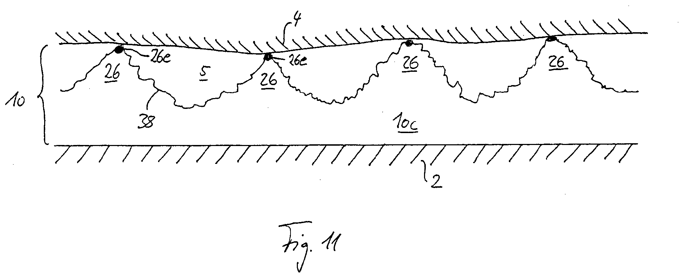

Die **Figur 11** zeigt eine Struktur aus vorstehenden Elementen 26 von im Wesentlichen gleicher Größe, wobei die gashaltende Schicht 10 eine raue Oberfläche 38 aufweist, welche bevorzugt sowohl im Bereich der Basis 10c als auch im Bereich der vorstehenden Elemente 26 mit gleichbleibender Rauheit ausgebildet ist. So können die Strukturen der rauen Oberfläche bevorzugt eine Größe aufweist, welche im Vergleich zu der Größe der vorstehenden Elemente 26 um einen Faktor von etwa 5 bis etwa 20 kleiner ist. Die raue Oberfläche kann auch eine multimodale Rauheitsverteilung aufweisen, welche Rauheiten von etwa 10 µm bis etwa 3 mm umfaßt, wobei insbesondere die vorstehenden Elemente 26 als größte Rauheit innerhalb der Rauheitsverteilung angesehen werden kann.

Dadurch ergeben sich vorteilhafterweise zwei oder mehr Strukturierungsebenen der Flüssigkeit-Gas-Grenzfläche, wie dies bereits zu den Figuren 7 bis 10 beschrieben wurde. Die dortigen Ausführungen gelten analog für die in Figur 11 gezeigte Ausführungsform. Vorteilhafterweise wird durch die raue Oberfläche 38 ein vollständiges Entfernen des Gases, welches in der gashaltenden Schicht 10 gehalten wird, in Bereichen der gashaltenden Schicht 10 vermieden.

Weiter bevorzugt können die vorstehenden Elemente 26 hydrophile Oberflächenbereiche 26e aufweisen, welche ein verbessertes Anhaften der Grenzfläche zwischen Flüssigkeit und Gas an den zugeordneten vorstehenden Elementen 26 ermöglicht. Es versteht sich, daß die raue Oberfläche 38 eine regelmäßige, ein quasi regelmäßige oder ein zufällige räumliche Oberflächenstruktur aufweisen kann.

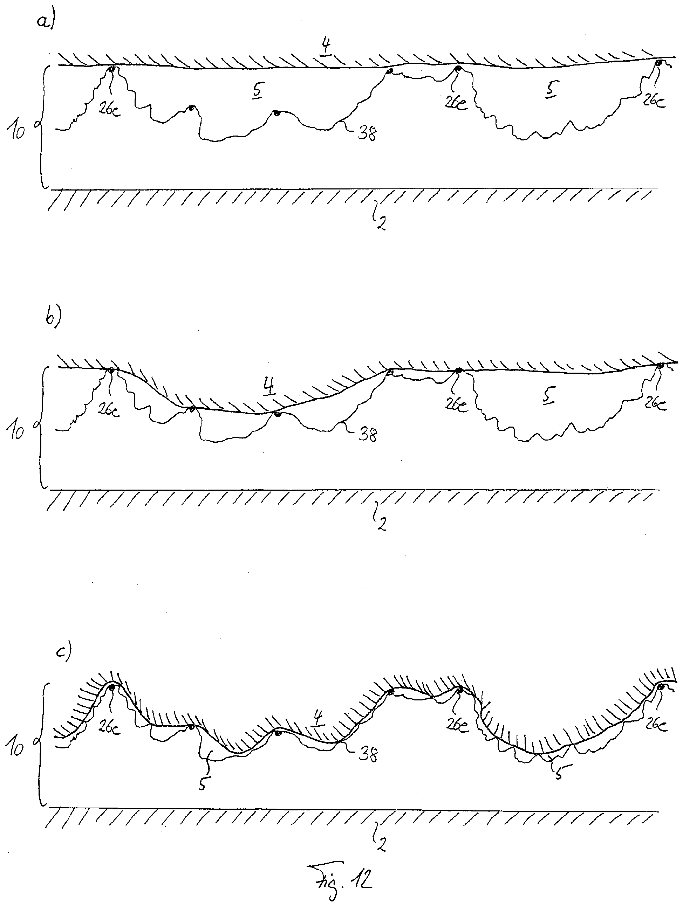

Die **Figuren 12a bis 12c** zeigen eine Ausführungsform, welcher der in der Figur 11 gezeigten Ausführungsform ähnlich ist. In dem in der Figur 12a gezeigten Zustand ist die gashaltende Schicht 10 vollständig mit Gas 5 gefüllt. Nach einem aufgetretenen Gasverlust aus der gashaltenden Schicht 10 verlagert sich die Flüssigkeits-Gas-Grenzfläche wie in Figur 12b gezeigt. Durch einen weiteren Gasverlust verlagert sich die Flüssigkeits-Gas-Grenzfläche wie in Figur 12c gezeigt. Durch den Gasverlust wird zwar der Strömungswiderstand der Wandung 2 relativ zu einer strömenden Flüssigkeit 4 vergrößert, jedoch wird die Flüssigkeit im Wesentlichen an einem direkten Kontakt mit der gashaltenden Schicht 10 bzw. der Wandung 2 gehindert, wodurch die Wandung 2 beispielsweise vor einem korrosiven Einfluß der Flüssigkeit geschützt wird.

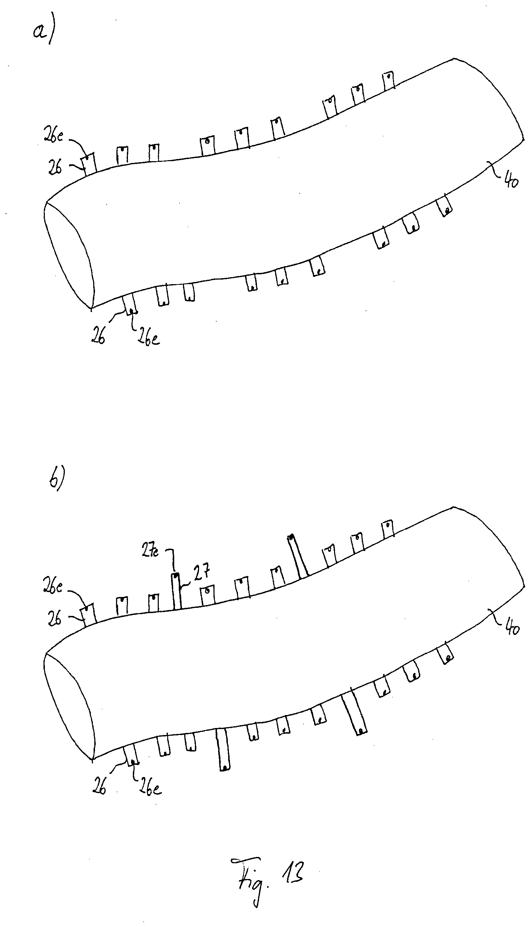

Die **Figuren 13a und 13b** zeigen Fasern 40, deren hydrophobe Oberfläche mit vorstehenden Elementen 26, 27 versehen sind. Dabei zeigt die Figur 13a eine Struktur bzw. Anordnung von vorstehenden Elementen 26, wie sie auch in Figur 1 gezeigt ist. Die Figur 13b zeigt eine Struktur bzw. Anordnung von vorstehenden Elementen 26,27 wie sie auch in den Figuren 9 und 10 gezeigt ist. Die Fasern 40 weisen daher die zu diesen Figuren beschriebenen Eigenschaften auf. Die vorstehenden Elemente sind mit hydrophilen Bereichen 26e, 27e versehen. Die Fasern 40 können als vorstehende Elemente der gashaltenden Schicht der Oberflächenabdeckung dienen und mit dieser integral ausgebildet sein.

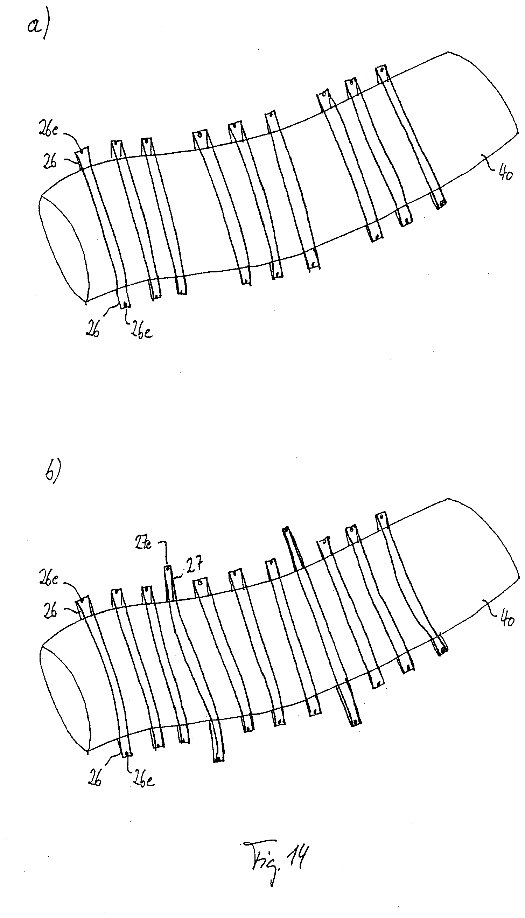

Die **Figuren 14a und 14b** zeigen Fasern 40, die jeweils eine ringförmige Struktur von vorstehenden Elementen 26, 27 aufweisen, die den in den Figuren 13a und 13b gezeigten Strukturen entsprechen. Auch auf den sich entlang des Umfangs der Fasern 40 erstreckenden Ringe 26, 27 sind hydrophile Bereiche 26e, 27e ausgebildet.

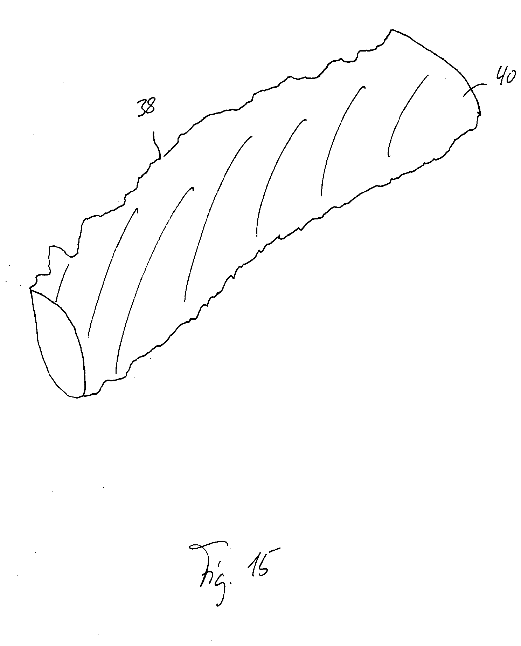

Die **Figur 15** zeigt eine Faser 40, deren hydrophobe, raue Oberfläche 38 eine wie in Figur 11 gezeigte Struktur bzw. Anordnung aufweist.

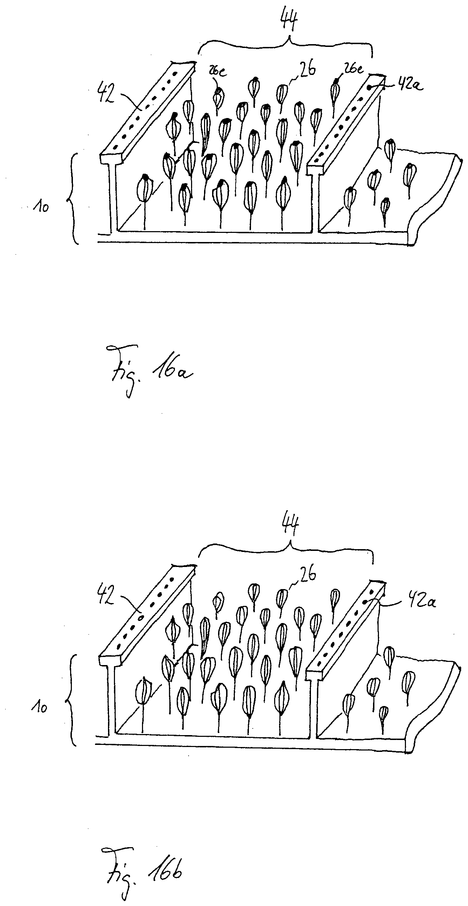

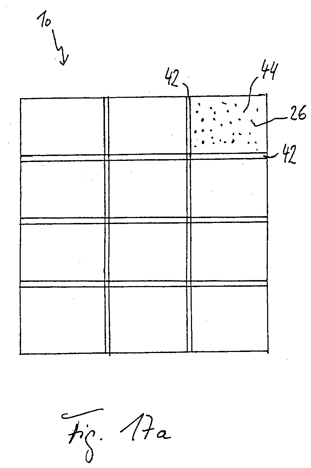

Die **Figuren 16a bis 16d** zeigen jeweils eine perspektivische Ansicht einer bevorzugten zweidimensionalen Anordnung der vorstehenden hydrophoben Elemente 26 einer gashaltenden Schicht 10. Die **Figur 17a** zeigt eine Draufsicht auf die in der Figur 16a gezeigten bevorzugten zweidimensionalen Anordnung.

Die vorstehenden Elemente 26 sind gemäß der in Figur 3a gezeigt Form ausgebildet. Es versteht sich jedoch, daß die vorstehenden Elemente auch jede andere zweckmäßige Gestalt aufweisen können, beispielsweise die in den Figuren 3 und 4 gezeigten Gestalten.

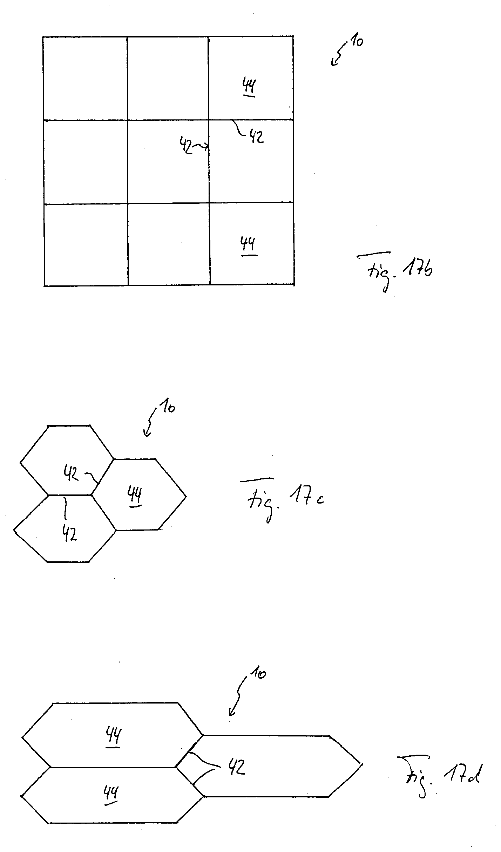

Wie in den Figuren 16a und 17 gezeigt, ist die zweidimensionale Anordnung der vorstehenden Elemente 26 in einzelne Teilbereiche 44, sogenannte "compartments", gegliedert, wobei jeder Teilbereich 44 der gashaltenden Schicht 10 eine Vielzahl von vorstehenden Elementen 26 umfaßt. Die einzelnen Teilbereiche 44 sind durch eine fluidundurchlässige Trennwand 42 gegenüber benachbarten Teilbereichen 44 oder gegenüber der Umgebung abgegrenzt. Unter einem Fluid wird sowohl ein Gas, eine Flüssigkeit sowie ein Gemisch davon verstanden. Folglich verhindert die Trennwand 42, daß sich eine Flüssigkeitsströmung oder eine Gasströmung zwischen den Teilbereichen 44 ausbildet. Insbesondere bei einem Druckunterschied zwischen zwei benachbarten Teilbereichen 44 wird vorteilhafterweise mittels der Trennwände 42 verhindert, daß Gas von einem Teilbereich 44 weg zu dem benachbarten Teilbereich strömt und dadurch der Strömungswiderstand gegenüber einer kontaktierenden Flüssigkeit lokal erhöht wird.

Insbesondere wird vermieden, daß ein Teilbereich 44 durch zuströmendes Gas über seine Gasfassungskapazität hinaus mit Gas beaufschlagt wird und daraufhin Gas in die Flüssigkeit übergeht und dadurch verloren geht.

Bevorzugt können die Trennwände 42 aus dem gleichen Material ausgebildet sein, wie die vorstehenden Elemente 26. Insbesondere kann die gashaltende Schicht 10 mit den vorstehenden Elementen 26 und den Trennwänden 42 gemeinsamen bzw. einstückig ausgebildet sein. Weiter bevorzugt weisen die Trennwände 42 eines Teilbereichs 44 im Wesentlichen die gleich Höhe auf, wie die im Teilbereich 44 enthaltenden vorstehenden Elemente 26. Weiter bevorzugt weisen die vorstehenden Elemente 26 hydrophile Oberflächenbereiche 26e und/oder weisen die Trennwände 42 hydrophile Oberflächenbereiche 42a auf.

Wie in der Figur 16b gezeigt, können die vorstehenden Elementen 26 auch gänzlich hydrophob ausgebildet sein, während die fluidundurchlässigen Trennwände 42 hydrophile Oberflächenbereiche 42a aufweisen.

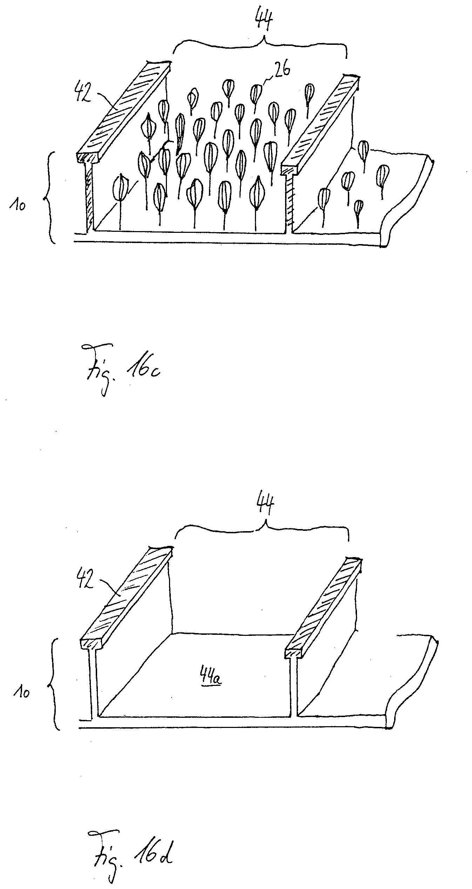

Alternativ können die Trennwände 42 auch vollständig oder bereichsweise aus einem hydrophilen Material ausgebildet sein, wie in der Figur 16c gezeigt, während die vorstehenden Elemente 26 gänzlich hydrophob sein können oder auch hydrophile Oberflächenbereiche 26a, entsprechend den in der Figur 16a gezeigten vorstehenden Elemente 26, aufweisen können (in Figur 16c nicht gezeigt). Beispielsweise können lediglich die der Flüssigkeit zugewandten Oberflächen der Trennwände 42 hydrophil ausgebildet oder beschichtet sein.

Schließlich können die Trennwände 42 auch gänzlich einen Teilbereich 44 ausbilden, wie in der Figur 16d gezeigt. Mit anderen Worten enthält der Teilbereich 44 keine vorstehenden Elemente. Bevorzugt weist der Boden 44a des Teilbereichs 44 eine hydrophobe Oberfläche auf. Die Trennwände können ganz oder bereichsweise hydrophil ausgebildet sein. Beispielsweise können lediglich die der Flüssigkeit zugewandten Oberflächen der Trennwände 42 hydrophil ausgebildet oder beschichtet sein. Insbesondere können die Trennwände 42 im Wesentlichen hydrophob ausgebildet sein, wobei an der Oberkante, das heißt an den der Flüssigkeit zugewandten Oberflächen der Trennwände 42, ein flächiger oder linienförmiger hydrophiler Bereich ausgebildet ist, der bevorzugt geschlossen bzw. nicht durch einen oder mehrere hydrophobe Bereiche unterbrochen ist, so daß der Gasaustausch zwischen zwei benachbarten Teilbereichen 44 auch über die Oberkanten der Trennwände 42 hinweg wirksam unterbunden ist. Die Trennwände können dabei ein abgeschlossenes Volumen des Teilbereichs 44 einschließen, so daß das Gas innerhalb dieses Teilbereichs 44 gehalten wird und im Wesentlichen nicht in einen benachbarten Teilbereich entweichen kann.

Es versteht sich, daß die verschiedenen Ausführungsformen der vorstehenden Elemente 26 in beliebiger Weise mit den verschiedenen Ausführungsformen der Trennwände 42 kombiniert werden können, um einen (abgeschlossenen) Teilbereich 44 auszubilden.

Insbesondere kann zumindest ein Teilbereich 44 optional mit einer Trennwand 42 oder mehreren Trennwänden 42 als eine Fliese bzw. Kachel ausgebildet sein, welche an einem Körper befestigbar ist. Insbesondere kann eine Fliese bzw. Kachel (im folgenden auch Air-Tiles genannt) eine Vielzahl von mehr als 1000, mehr als 10000 oder mehr als 100000 Teilbereichen 44 aufweisen. Vorteilhafterweise kann eine beliebig große Wandung mit einer Vielzahl von derartigen Fliesen bzw. Kacheln mit einer gashaltenden Schicht versehen bzw. bedeckt werden, wodurch sich weiter vorteilhafterweise eine flexible Montage ergibt und lediglich eine geringe Anzahl verschiedener Fliesen bzw. Kacheln bereitgestellt werden muß.

Die Teilbereiche 44 sind derart geformt bzw. ausgebildet und derart an dem Körper angebracht, dass die Längserstreckung der Teilbereiche 44 entlang der Vertikalen kleiner ist als entlang der senkrecht dazu stehenden Horizontalen. Insbesondere wenn die Teilbereiche 44 an einem Schiffsrumpf befestigt sind, ist die Längserstreckung vorteilhafterweise entlang der Schwerkraftrichtung (Vertikale) kleiner als entlang der Wasserströmungsrichtung beim Fahren des Schiffs (im Wesentlichen der Horizontalen), da das in der gashaltenden Schicht enthaltene Gas aufgrund des dann geringeren Abstands der Trennwände 42 die Druckdifferenz zwischen zwei benachbarten Teilbereichen 44 geringer wird. Gleichzeitig ermöglichen größere Abstände zwischen den Trennwänden 42 entlang der Horizontalen eine Minimierung der hydrophilen Bereiche, an denen eine Reibung zwischen dem Wasser und dem Schiff auftritt.

Daher sind die Teilbereiche 44 bevorzugt rechteckig ausgebildet, wie in der Figur 17a gezeigt.

Es versteht sich jedoch, daß die Teilbereiche 44 bevorzugt auch sechseckig bzw. wabenförmig, dreieckig, quadratisch oder in einer anderen Form ausgebildet sein können. Die **Figur 17b** zeigt eine Draufsicht auf eine bevorzugte Ausführungsform, wobei die Trennwände 42 derart angeordnet sind, daß quadratische Teilbereiche 44 ausgebildet werden. Die **Figur 17c** zeigt eine Draufsicht auf eine bevorzugte Ausführungsform, wobei die gleichlangen Trennwände 42 derart angeordnet sind, daß sechseckige Teilbereiche 44 ausgebildet werden. Die **Figur 17d** zeigt eine Draufsicht auf eine bevorzugte Ausführungsform, wobei die Trennwände 42 derart angeordnet sind, daß langgestreckte, wabenförmige bzw. sechseckig Teilbereiche 44 ausgebildet werden.

### Beschichtung zum Schutz von Oberflächen unter Flüssigkeit

Zusammenfassend beschreibt ein Aspekt eine Methode zur Erzielung von Antifouling-Wirkung sowie von einer Schutzwirkung gegen Korrosion und chemischen Angriff von Oberflächen unter Flüssigkeit durch Verwendung einer Beschichtung, die eine Gasschicht unter Flüssigkeit hält.

Zusätzlich kann die Oberfläche - beispielsweise des Schiffes, Rohres, Fensters, Messinstrumentes, Wasserbehälters etc. - mit einer toxischen oder Biozide enthaltenden Schicht oder durch anderweitige Zusatzstoffe eine chemische oder biologische Antifouling-Wirkung entfaltenden Beschichtung ausgestattet werden. Der Vorteil liegt dann darin, dass die Wirkstoffe nur dann freigesetzt werden, wenn sie benötigt werden, nämlich, wenn die Oberfläche kurzzeitig nicht mit einer Gasschicht bedeckt ist, sondern mit Wasser oder mit Meeresorganismen in Berührung kommt. Auf diese Weise erfolgt die Freisetzung toxischer oder biozider Zusatzstoffe nur selten und zwar exakt bei Bedarf - mit der Folge, dass die an die Flüssigkeit, beispielsweise das Meerwasser oder Süßwasser abgegebene Menge an toxischen Substanzen pro Zeit und Fläche um bis zu mehrere Zehnerpotenzen reduziert werden kann, was wiederum zwei vorteilhafte Auswirkungen hat:
(i) die Freisetzung toxischer und biozider Substanzen wird drastisch reduziert - ohne Reduzierung der Antifouling-Wirksamkeit und
(ii) damit wird auch die Zeitdauer, die der Vorrat an solchen Substanzen im Schiffslack oder in der Beschichtung hält, bevor er so weit verbraucht ist, dass die Antifouling-Wirkung nachlässt, drastisch verlängert - und damit die Serviceintervalle der Schiffe und anderer Einrichtungen. Besonders bei Ölplattformen, Offshore-Windparks und anderen schwer zugänglichen Objekten ist dies ein enormer Vorteil.

Die Erzielung von Antifouling-Wirkung kann durch Verwendung von lufthaltenden bzw. allgemein gashaltenden Schichten unter Wasser an sich oder in Kombination mit der Zugabe von toxischen, bioziden oder anderweitig auf biologischen Bewuchs wirkenden Gasen oder Aerosolen über die Gasschicht erreicht werden.

Die Gasschicht an sich bildet schon eine Barriere für die Ansiedlung von Meeresorganismen. Dies gilt insbesondere für die Ansiedlung von Bakterien, Kieselalgen, Einzellern und Mikroorganismen, die den sog. Slime bilden bzw. für das Mikrofouling verantwortlich sind und oft die Basis für die Ansiedlung größerer Organismen bilden. Ist die Oberfläche beispielsweise eines Schiffes etc. - von einer Luftschicht umgeben und kommt nicht mit Wasser in Berührung, so ist eine Ansiedlung und Vermehrung dieser Organismen nicht mehr möglich.

Der Bewuchs von Schiffen und anderen Wasserfahrzeugen sowie dem Wasser ausgesetzten technischen Einrichtungen, Wänden, Bauwerken etc. durch aufwachsende biologische Systeme ist sowohl im Süßwasser als auch im Meereswasser ein großes technisches Problem. Beim Bewuchs von Schiffen kommt erschwerend hinzu, dass der Bewuchs die Reibung der Schiffe im Wasser und damit auch den Treibstoffverbrauch erheblich erhöht. Die bisherige Lösung, zum Schutz vor solchem Bewuchs die Schiffe etc. mit toxischen Farben, Lacken und Beschichtungen auszustatten, ist aus Umweltschutz-Gründen nicht mehr tragbar und wird zunehmend verboten, da nachweisbar ist, dass diese hochgiftigen Schiffsbeschichtungen erhebliche Mengen giftiger Stoffe und Verbindungen, insbesondere Schwermetalle und Schwermetallverbindungen, ins Meerwasser abgeben.

Nicht-toxische Stoffe mit hinreichendem Schutz vor Biofouling müssen aber erst noch gefunden werden. Die Chancen hierfür stehen vermutlich auch in Zukunft schlecht, da die Systeme einerseits den biologischen Bewuchs mit Meeresorganismen verhindern sollen, zugleich aber ausgerechnet in ihrer Wirkung die Meeresorganismen nicht schädigen dürfen, um nicht die Fauna und Flora der Meere und Küsten zu schädigen. Die gestellte Aufgabe stellt also möglicherweise in sich einen Widerspruch dar. Es muss also ein Weg gefunden werden, um selektiv die Wasser- und Meeresorganismen auf der Oberfläche zu beeinträchtigen, ohne dass es zu einer nennenswerten Schädigung der Organismen im Ökosystem des umgebenden Süßwassers oder Meereswassers kommt. Eine technische Lösung für einen solchen Weg zeigt die vorliegende Erfindung.

Da einerseits Meeresorganismen und andere Lebewesen, die sich am Schiff oder anderen technischen Oberflächen in Süß- und Salzwasser niederlassen wollen, an einer Ansiedlung gehindert und bekämpft werden sollen, die Maßnahme aber sehr selektiv nur an diesen Oberflächen wirken soll und die biologischen Organismen oder Meeresorganismen an anderen Orten nicht schädigen soll, liegt die Anwendung einer lokalen Maßnahme nahe, die sich ausschließlich auf die zu schützenden Oberflächen und deren unmittelbare Umgebung bezieht, nahe.

Nach dem erfindungsgemäßen Verfahren wird das geschilderte technische Problem wie folgt gelöst: Da es technisch möglich ist, Gasschichten an einer Oberfläche unter Wasser zu halten und Gas gezielt über Gewebe, poröse Schichten oder kleine Öffnungen oder Düsen in diese Schicht einzubringen, liegt es nahe, die zu schützende Oberfläche mit einer solchen Gas haltenden Oberfläche oder Schicht auszustatten und in regelmäßigen oder unregelmäßigen zeitlichen Abständen oder aber bei Bedarf, d.h. bei beginnendem oder erfolgtem biologischem Bewuchs ein diesen Bewuchs bekämpfendes Gas oder Aerosol (mit entsprechend wirkenden Sol-Partikeln) in die Gasschicht über der Oberfläche einzubringen. Die Behandlung ist besonders dann ohne Nebenwirkungen für das Ökosystem, wenn das Gas oder Aerosol nur geringfügig in Wasser löslich ist oder die Verbindung nach einer gewissen Zeit zerfällt in ungiftige Bestandteile oder nur in hohen Konzentrationen toxisch ist oder wenn einfach CO₂ zum Ersticken der angesiedelten Meeresorganismen auf der Schiffsoberfläche verwendet wird. Die Behandlung und die Dosierung können dabei manuell oder automatisch erfolgen.

Optionale Varianten und Merkmale betreffen eine
- Kombination mit Sensorik und/oder Kameras zur automatischen Erfassung von Umfang und/oder Art des biologischen Bewuchses mit oder ohne quantitative Auswertung des Umfanges des Bewuchses.
- Kombination mit einer ortsselektiven Sensorik und/oder Kameras zur automatischen Erfassung und lokalen Ortung des biologischen Bewuchses mit oder ohne quantitative Auswertung des Umfanges.
- Möglichkeit der ortsselektiven Ausbringung des wirksamen Gases oder Aerosols selektiv an den Stellen, an denen der Bewuchs erfolgt ist oder beginnt.
- Möglichkeit der ortsselektiven Dosierung je nach Stärke und Art des Bewuchses.
- Gassensorik zur ortsaufgelösten Erfassung der Gaszusammensetzung bzw. der Wirkstoffkonzentration in der Gasschicht.
- Gasanalytik durch Entnahme von Gas bzw. Aerosol aus der Schicht, auch in Verbindung mit Massenspektroskopie, IR-Analytik, Gelchromatographie, Gaschromatographie etc. zur ortsaufgelösten und/oder zeitaufgelösten Analytik der Zusammensetzung und/oder Wirkstoffkonzentration in der Gasschicht.
- Begleitende Analytik von gelösten Komponenten des Gases oder Aerosols im Umgebungswasser, um zu jeder Zeit und an jedem Ort unterhalb festgesetzter Grenzwerte zu bleiben und alle Umwelt-Auflagen zuverlässig an jedem Ort und zu jedem Zeitpunkt zu erfüllen und die Behandlung bei Bedarf jederzeit manuell oder- bevorzugt-automatisch zu stoppen durch sofortiges Stoppen der Zufuhr des Wirkgases oder Aerosols.

Die Erzielung von Antifouling-Wirkung kann auch durch Verwendung von lufthaltenden Schichten unter Wasser in Kombination mit toxischen Materialien, Additiven und Oberflächenbeschichtungen erreicht werden.

Schiffsbeschichtungen, die hochwirksam gegen Bewuchs durch Wasser- bzw. Meeresorganismen sind, gibt es bereits. Ihr Problem liegt nicht darin, dass sie toxisch sind - das müssen oder zumindest sollen sie sein, um gegen eine Besiedlung durch die Meeresorganismen wirksam zu sein. Ihr Problem liegt vielmehr darin, dass über den dauernden, langzeitigen Kontakt mit dem Wasser die giftigen Verbindungen und Schwermetalle ins Meerwasser gelangen.

Hier setzt das erfindungsgemäße Verfahren an: Die gegen den Bewuchs wirksamen Beschichtungen werden beibehalten, und somit ist die Antifouling-Wirkung gewährleistet und technisch erprobt. Es lassen sich neben neu entwickelten Antifouling-Beschichtungen auch über Jahre und Jahrzehnte erprobte Beschichtungen einsetzen. Eine Abgabe an das umgebende Wasser und damit an das umgebende Ökosystem wird aber verhindert, indem die Oberfläche mit der Antifouling-Beschichtung als lufthaltende bzw. gashaltende Oberfläche ausgebildet ist und somit das Wasser gar nicht mit der Antifouling-beschichteten Oberfläche in Kontakt kommt, da zwischen der - ggf. toxischen - Oberfläche und dem Wasser sich eine permanente und insbesondere auch unter Betriebsbedingungen persistente Luft- oder Gasschicht befindet. Bei Gasverlust durch Spitzenbelastungen wird die Schicht in einer bevorzugten Variante des Verfahrens wieder mit Gas nachgeladen ("regeneriert"): Technisch ist es möglich, eine solche Gasschicht an einer Oberfläche unter Wasser zu halten und Gas gezielt über Gewebe, poröse Schichten oder kleine Öffnungen oder Düsen in diese Schicht einzubringen. Damit kann bei Gasverlust die Schicht umgehend wieder regeneriert werden. Die Haare, Fasern, Säulen, Spitzen oder Dornen etc., die die Luftschicht "aufspannen" oder aufrecht erhalten, können, müssen aber nicht selbst oder durch ihre Beschichtung eine Antifouling-Wirkung haben.

Optionale Varianten und Merkmale betreffen eine
- Kombination mit einer automatischen - bevorzugt ortsselektiven - Sensorik zur Überwachung gegen Luftverlust.
- Kombination einer solchen Sensorik mit einer automatischen - bevorzugt ortsselektiven - Nachfüllvorrichtung, so dass eine ständige Präsenz der Gasschicht gewährleistet wird und ein länger anhaltender Kontakt des Meerwassers mit der Antifouling-Beschichtung vermieden wird.
- Begleitende Analytik von gelösten toxischen Komponenten der Schicht im Umgebungswasser, um zu jeder Zeit und an jedem Ort unterhalb festgesetzter Grenzwerte zu bleiben und alle Umwelt-Auflagen zuverlässig an jedem Ort und zu jedem Zeitpunkt zu erfüllen.
- Verwendung des Verfahrens und der Vorrichtung auch im Süßwasser.
- Verwendung des Verfahrens und der Vorrichtung auch mit toxischen, antibiotischen, bioziden, Schwermetall-haltigen und anderen Lacken, Beschichtungen und Anstrichen.
- Verwendung des Verfahrens und der Vorrichtung auch mit ungiftigen Lacken, Beschichtungen und Anstrichen.
- Nutzung der Gasschicht auch zur Reibungsreduktion des Schiffes, Bootes etc..
- Verwendung der genannten erfindungsgemäßen Techniken und Verfahren im Süß- oder im Brack- oder im Meereswasser.
- Verwendung der genannten erfindungsgemäßen Techniken und Verfahren nicht nur für die Oberfläche von Schiffen, sondern auch für die äußeren und inneren Oberflächen von anderen technischen Komponenten, die mit Wasser in Berührung kommen, sowie für entsprechende Wandungen, Bauwerke etc. unter Wasser sowie für Rohrleitungen, Bäder etc.

Die Erzielung von Antifouling-Wirkung durch Verwendung von lufthaltenden Schichten unter Wasser kann allein durch die Wirkung der Gasschicht oder in Kombination mit Ultraschall, mechanischen Bewegungen und Verformungen, Stoßwellen, wiederholten thermischen Behandlungen der Oberfläche (z.B. durch elektrische Heizung von elektrisch leitfähigen Härchen, Gewebe etc.) oder UV-Behandlung oder elektrischen Impulsen einschließlich Gasen, die bei der elektrochemischen Zersetzung von Wasser oder Salzwasser oder Meerwasser entstehen, erfolgen.

Die Verwendung von unter Wasser lufthaltenden bzw. gashaltenden Oberflächen stellt die Basis für das hier vorgestellte Verfahren dar. Da es technisch möglich ist, Gasschichten an einer Oberfläche unter Wasser zu halten und Gas gezielt über Gewebe, poröse Schichten oder kleine Öffnungen oder Düsen in diese Schicht einzubringen, liegt es nahe, die zu schützende Oberfläche mit einer solchen Gas haltenden Oberfläche oder Schicht auszustatten und zunächst bereits durch die Gasschicht selbst den Kontakt biologischer Systeme mit zu schützenden Oberfläche zu unterdrücken. Ein solcher mechanischer Kontakt ist aber als Ausgangspunkt für ein Anhaften erforderlich und wird durch eine Luft- oder Gasschicht aber verhindert.

Kommt, z.B. durch Stöße, intensives aneinander reiben etc. ein solcher Kontakt und letztendlich ein Bewuchs doch zustande, kann man zusätzlich in regelmäßigen oder unregelmäßigen zeitlichen Abständen oder aber bei Bedarf, d.h. bei beginnendem oder erfolgtem biologischem Bewuchs weitere Maßnahmen zur physikalischen Bekämpfung der Meeresorganismen zu ergreifen, um den Einsatz von umweltschädigender Chemie zu vermeiden, wobei sich zwei Verfahren in besonderer Weise anbieten: (i) die mechanische Verminderung und Lösung des Haftkontaktes der Meeresorganismen zur Unterlage, an der sie sich festhalten, durch die Bewegung elastischer Haare, durch Stoßwellen und durch Ultraschall sowie (ii) die lokale Heizung (insbesondere resistiv oder durch Induktion oder durch Mikrowellen oder Kombination dieser Verfahren) zur thermischen Zerstörung der anhaftenden Zellschicht.

In einer bevorzugten Variante des Verfahrens werden die Strukturen (Härchen, Fasern etc.), die die Lufthaltung der Oberfläche bewirken, elektrisch leitfähig ausgebildet und direkt geheizt. Alternativ oder zusätzlich dazu kann die Oberfläche, insbesondere das Aerenchym elektrisch leitfähig ausgebildet werden und als lokale Heizung dienen.

In einer weiteren Variante des Verfahrens kann auch Strahlung, beispielsweise ultraviolettes Licht, in Kombination mit der lufthaltenden Oberfläche angewendet werden.

Optionale Varianten und Merkmale betreffen eine
- Verwendung von metallischen Oberflächenstrukturen.
- Verwendung des Verfahrens und der Vorrichtung auch im Süßwasser.
- Verwendung des Verfahrens und der Vorrichtung auch mit toxischen, antibiotischen, bioziden, schwermetallhaltigen und anderen Lacken, Beschichtungen und Anstrichen.
- Verwendung des Verfahrens und der Vorrichtung auch zusammen mit ungiftigen Lacken, Beschichtungen und Anstrichen.
- Nutzung der Gasschicht auch zur Reibungsreduktion des Schiffes, Bootes etc.
- Beschichtung von Schiffsoberflächen - ganz oder teilweise.
- Verwendung der genannten erfindungsgemäßen Techniken und Verfahren im Süß- oder im Brack- oder im Meereswasser.
- Verwendung der genannten erfindungsgemäßen Techniken und Verfahren nicht nur für die Oberfläche von Schiffen, sondern auch für die äußeren und inneren Oberflächen von anderen technischen Komponenten, die mit Wasser in Berührung kommen, sowie für entsprechende Wandungen, Bauwerke etc. unter Wasser sowie für Rohrleitungen, Bäder etc.

Mit anderen Worten können (bevorzugte) Gegenstände der Anmeldung wie folgt beschrieben sein:
Gegenstand 1 betrifft eine Beschichtung zum Schutz einer dauernd oder zeitweise ganz oder teilweise einer Flüssigkeit ausgesetzten Oberfläche oder Grenzfläche vor Korrosion, chemischem Angriff und / oder (Bio)Fouling, dadurch gekennzeichnet, dass die Oberfläche oder Grenzfläche so beschichtet ist, dass sie unter Flüssigkeit eine durchgehende oder nicht durchgehende ständig oder zeitweise bestehende Gasschicht unter Flüssigkeit hält und diese Gasschicht die Oberfläche vor der Korrosion, dem chemischem Angriff und / oder dem (Bio)Fouling schützt.
Gegenstand 2 betrifft eine Beschichtung gemäß Gegenstand 1, dadurch gekennzeichnet, dass es sich bei dem Schutz vor Fouling um Biofouling, insbesondere in Form von Mikrofouling, Makrofouling, den Angriff von Algen, Muscheln und / oder anderen Meeresorganismen oder eine Kombination dieser Formen handelt.
Gegenstand 3 betrifft eine Beschichtung gemäß Gegenstand 1 oder 2, dadurch gekennzeichnet, dass die mit der Gasschicht ganz oder teilweise ständig oder zeitweise überdeckte verwendete Beschichtung selbst Biozide, TBT, Kupfer, Silber, Schwermetalle oder Metallverbindungen oder Metallkomplexe oder Metalllegierungen oder andere, das Fouling reduzierende Komponenten oder Beimischungen enthält und die Freisetzungsrate dieser Stoffe (pro Zeit und Fläche freigesetzte Menge dieser Stoffe) durch die Gasschicht vermindert wird.
Gegenstand 4 betrifft eine Beschichtung gemäß Gegenstand 1, dadurch gekennzeichnet, dass die Beschichtung zum Schutz der Oberflächen auf der Oberfläche von Schiffen, Yachten, Booten und anderen Wasserfahrzeugen oder auf im Meer installierten technischen Einrichtungen und Bauwerken, insbesondere Ölplattformen, Offshore-Windkraftanlagen, Stahlkonstruktionen, Betonkonstruktionen oder anderen ortsfest oder nicht ortsfest im Meer oder im Süßwasser installierten technischen Einrichtungen, Bojen, Leitungen und Kabeln, Antriebseinrichtungen, Schiffsoberflächen, Schiffsschrauben und Steuervorrichtungen, Fenstern etc. die sich zeitweise oder permanent unter Wasser befinden oder von Wasser angespült werden, Schiffsrudern, Scheinwerfern und anderen Licht emittierenden optischen Funktionseinheiten angebracht wird.
Gegenstand 5 betrifft eine Beschichtung gemäß Gegenstand 1, dadurch gekennzeichnet, dass es sich bei dem Gas der durchgehenden oder nicht durchgehenden permanenten oder zeitweise existierenden Gasschicht um Luft, Stickstoff, Sauerstoff, Kohlendioxid, Argon, Helium oder Gemischen aus diesen Gasen handelt und / oder dass es sich bei der Flüssigkeit um Wasser, Salzwasser, Meereswasser oder um Alkohol oder um wässrige oder alkoholische Lösungen handelt.
Gegenstand 6 betrifft eine Beschichtung gemäß Gegenstand 1, dadurch gekennzeichnet, dass die Beschichtung Oberflächenstrukturen, Säulen, Haare, Noppen enthält.
Gegenstand 7 betrifft eine Beschichtung gemäß Gegenstand 1 oder 6, dadurch gekennzeichnet, dass die Beschichtung Oberflächenstrukturen, Säulen, Haare, Noppen oder andere Strukturen eine Höhe von 0,02 mm bis 2 mm haben und eine hydrophobe Oberfläche mit oder ohne hydrophile Patches, End- oder Seitenflächen aufweisen.
Gegenstand 8 betrifft eine Beschichtung gemäß Gegenstand 1, dadurch gekennzeichnet, dass die Gas haltende Beschichtung auf der Außen- oder Innenseite von Rohrleitungen oder von Reaktionsgefäßen für chemische Reaktionen oder auf der Innenseite von Behältnissen zur Aufbewahrung von Flüssigkeiten aufgebracht ist.
Gegenstand 9 betrifft eine Beschichtung gemäß Gegenstand 1, dadurch gekennzeichnet, dass die Lufthaltung unter Ausnutzung des Salvinia-Effektes, des Notonecta-Effektes oder durch hierarchisch strukturierte Oberflächen erfolgt.
Gegenstand 10 betrifft eine Beschichtung gemäß Gegenstand 1, dadurch gekennzeichnet, dass die nicht durchgehende Gasschicht aus einer regelmäßigen, teilweise regelmäßigen oder unregelmäßigen Anordnung von Gasbläschen auf der Oberflächenbeschichtung besteht.

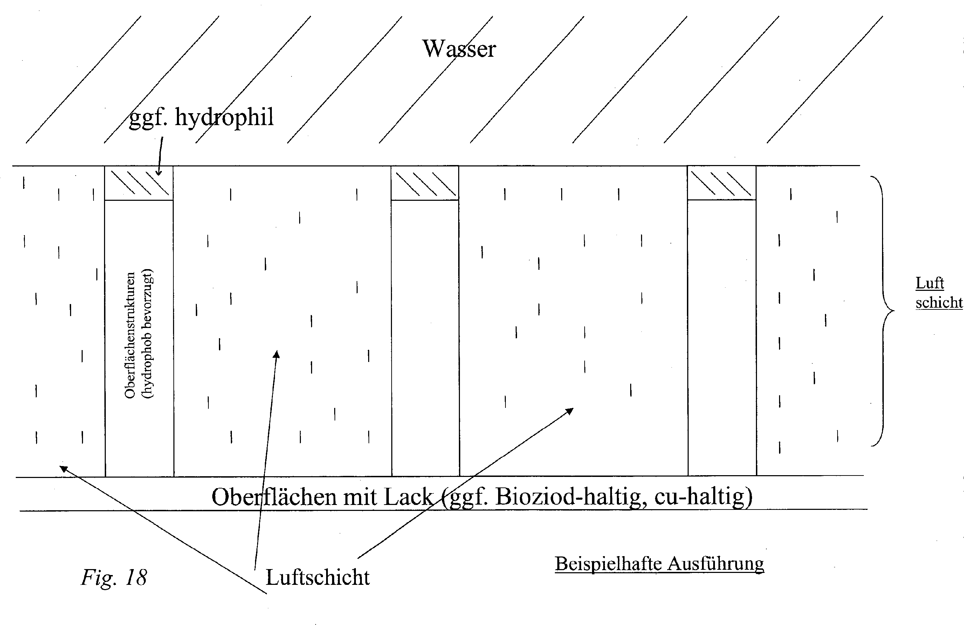

Die **Figur 18** zeigt einen bevorzugten Aufbau einer gashaltenden Schicht, welche eine Vielzahl von hydrophoben Vorsprüngen aufweist, welche optional einen hydrophilen Kopfbereich aufweisen, der bei betriebsgemäßem Gebrauch dem Wasser zugewandt ist. Die gashaltende Schicht ist an einem Lack angeordnet, welche optional ein Biozid oder Kupfer enthält.

### Vorrichtung zum Erzielen einer Gasschicht unter Flüssigkeit

Zusammenfassend beschreibt ein Aspekt Gasschichten auf Oberflächen unter Flüssigkeit, die von großem technologischem Interesse zur Reibungsreduktion bei Schiffen und in Pipelines sowie zur Erzielung von Schutz der Oberfläche vor Belagbildung, (Bio)Fouling, Korrosion und chemischem Angriff sind.

Durch eine geeignete Oberflächenstrukturierung gelingt es, eine an der Oberfläche anhaftende Schicht aus Gas oder aus Gasbläschen mit unter Flüssigkeit zu nehmen. Das Problem ist, dass eine solche Gasschicht nur für begrenzte Zeit stabil ist und dann verlorengeht. Dieses Problem wird durch eine wieder mit Gas nachladbare Schicht gelöst.

Gemäß einem Aspekt kombiniert die vorliegende Erfindung
(1) eine strukturierte, unter Flüssigkeit eine Gasschicht oder Gasbläschen haltende Schicht mit
(2) einem Nachladesystem, bestehend z.B. aus (i) Gaszuleitungen oder -Kanälen sowie Düsen oder Mikrodüsen oder (ii) einer porösen Membran, Textil, Gewebe- Filz-, Flock- oder Fasersystem oder Sinterschicht oder (iii) Gasnachladung über ein Einfangen von z.B. von einem Sprudler erzeugten (Mikro)Gasbläschen über Oberflächenhaare oder Oberflächenstrukturen, die bevorzugt flüssigkeitsabstoßende Oberflächen besitzen sowie mit
(3) einer Nachladevorrichtung, bestehend z.B. aus (i) einer Gas-Pumpe oder (ii) einer Selbstnachladung, etwa durch den Unterdruck, den ein sich relativ zur Flüssigkeit bewegendes Objekt oder eine Strömung erzeugt oder (iii) einem Sprudler für die externe Erzeugung von Gasbläschen, die dann von der strukturierten Oberfläche eingefangen werden.

Da aus der Gasschicht verlorengegangenes Gas wieder nachgeladen werden kann, bleibt die Gasschicht langfristig erhalten und erfüllt ihre gewünschte Funktion. In einer Variante wird der Nachladebedarf über Sensoren gemessen und automatisch Gas in die Schicht nachgeladen.

Luftschichten auf Oberflächen unter Wasser sind von großem technologischem Interesse beispielsweise und insbesondere zur Reibungsreduktion bei Schiffen und in Rohrleitungssystemen und Pipelines, zur Vermeidung von Ablagerungen und der Bildung von unerwünschten Belägen an Oberflächen unter Flüssigkeit, zur Erzielung von Antifouling-Wirkung sowie von einer Schutzwirkung gegen Korrosion und chemischen Angriff von Oberflächen unter Flüssigkeit, jeweils durch Verwendung einer Beschichtung, die eine Gasschicht oder eine Schicht von Gasbläschen oder Gastaschen unter Flüssigkeit hält.

Durch eine geeignete Oberflächenstrukturierung gelingt es, mit Textilien, strukturierten und / oder funktionalisierten Beschichtungen, etwa beispielsweise mit bioinspirierten Beschichtungen nach dem Vorbild der Wasserspinne oder von *Salvinia molesta* oder *Notonecta glauca,* eine Schicht aus Gas, beispielsweise von Luft, mit unter Flüssigkeit, beispielsweise unter Wasser, zu nehmen, wenn man die Oberfläche aus der Gasatmosphäre heraus unter Flüssigkeit, z.B. aus der Luft heraus unter Wasser taucht. Das Problem ist nur, dass eine solche Lage von Luft, typischerweise 1 µm bis 1 mm dick, nur für begrenzte Zeit an der Oberfläche anhaftet und dann, meist in Form der Emission von Gasbläschen aus der Gasschicht, diese Gasschicht verlorengeht.

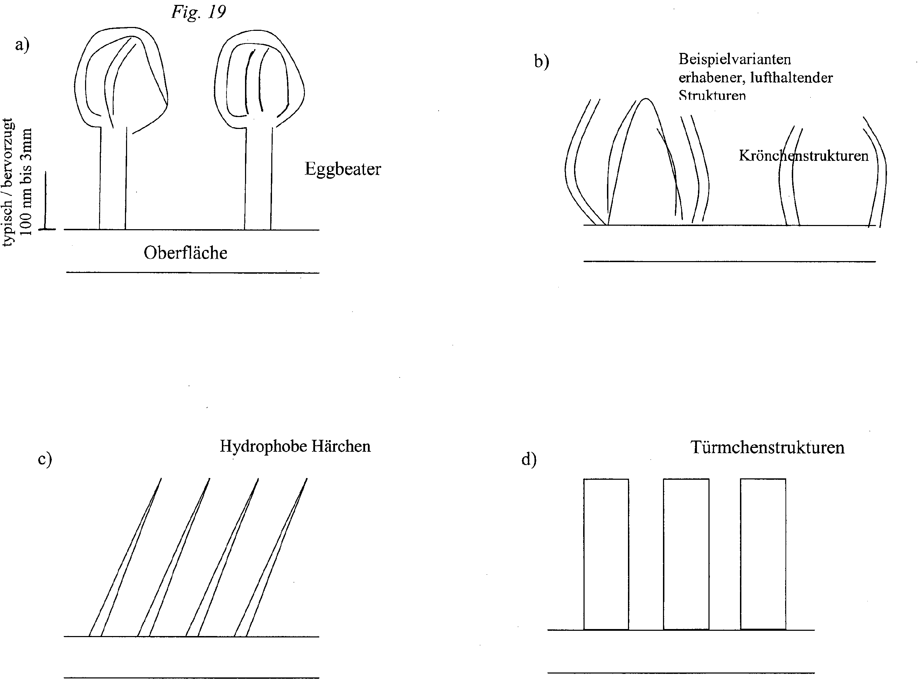

Die vorliegende Erfindung löst nun dieses Problem, indem die Vorrichtung mindestens zwei der folgenden drei Dinge kombiniert:
(1) eine strukturierte, unter Flüssigkeit gashaltende Schicht oder eine strukturierte, unter Flüssigkeit Gasbläschen oder Gastaschen haltende Schicht (siehe Figuren 19 bis 21) mit
(2) einem Nachladekanalsystem, beispielsweise aus (i) Zuleitungen oder Kanälen für die Gaszuführung sowie Düsen oder Mikrodüsen oder (ii) eine poröse Membran, Textil-, Gewebe-, Filz-, Flock- oder Fasersystem oder einer Sinterschicht oder (iii) einer Oberflächennachladung über eine Einfangvorrichtung für von beispielsweise von einem Sprudler erzeugten Gasbläschen oder Mikrogasbläschen, die beispielsweise aus Oberflächenhaaren oder Strukturen besteht, die flüssigkeitsabstoßende Oberflächen mit oder ohne hydrophile Zentren besitzen und damit in Form vorstehender Härchen, Säulen oder Krönchen oder durch Bildung entsprechender Kuhlen oder Mulden mit flüssigkeitsabstoßender Beschichtung die Gasbläschen aus der Flüssigkeit einfangen und an die Oberfläche pinnen oder in eine bestehende Gasschicht integrieren und mit
(3) einer Nachladevorrichtung, beispielsweise in Form von (i) einer Pumpe oder anderen aktiven Nachladevorrichtung oder (ii) einer Selbstnachladung, etwa durch den Unterdruck, den ein sich relativ zur Flüssigkeit bewegendes Wasserfahrzeug erzeugt oder den Unterdruck, den eine Strömung der Flüssigkeit relativ zu einer ruhenden Boje, Messstation, Wandung etc. erzeugt (Druckdifferenz, die durch Strömung der Geschwindigkeit v erzeugt wird = 0,5 . Dichte der Flüssigkeit . v²) oder (iii) einen Sprudler für die externe Erzeugung von Gasbläschen, die dann von der strukturierten Oberfläche eingefangen werden (siehe Punkt (2)).

Alternativ kann auch eine flüssige oder feste Substanz nachdosiert werden, die von alleine oder unter chemischer Reaktion mit der umgebenden Flüssigkeit ein Gas freisetzt und damit die Gasschicht oder Gasbläschenschicht wieder aufbaut oder ergänzt ("Replenishing").

Da aus der Gasschicht verlorengegangenes Gas wieder nachgeladen werden kann, bleibt die Gasschicht auf der Oberfläche unter Flüssigkeit auch langfristig erhalten und erfüllt ihre gewünschte Funktion.

Anstelle der Gasschicht kann auch eine Schicht aus einzelnen Gasbläschen oder Gastaschen (Air Pockets) bestehende oder diese enthaltende Schicht treten.

In einer Variante des Verfahrens wird der Nachladebedarf über Sensoren gemessen und als Ersatz für das fehlende Gas oder das der Gasschicht verlorengegangene Gas überall oder selektiv im Bereich, für den der jeweilige Sensor zuständig ist, wieder durch Aktivierung der Pumpe oder des Sprudlers oder durch Öffnen der entsprechenden Gasventile Gas in den entsprechenden Bereich eingespeist ("Gas Recharging on Demand"). In dieser Variante des Verfahrens wird der Nachladebedarf somit über Sensoren gemessen und es wird das verlorengegangene oder in der Schicht fehlende Gas mit der Vorrichtung automatisch nachgeladen.

Natürlich kann auf diese Weise bei Bedarf auch der erstmalige Aufbau einer Gasschicht erfolgen.

Eine Vorrichtung zum Halten einer Luftschicht unter Wasser oder allgemein einer Gasschicht unter Flüssigkeit, ist dadurch gekennzeichnet, dass die Schicht bei Gasverlust über eine gasdurchlässige Unterlage wieder beladen werden kann, wobei die gasdurchlässige Unterlage eine textile Unterlage, ein anderes Gewebe oder Filz, ein Flockmaterial, eine poröse oder gasdiffusionsgeeignete Keramikschicht, eine Metallschicht mit Poren, ein Metallfilz oder Drahtgeflecht, eine semipermeable Membran, eine - bevorzugt hydrophobe oder superhydrophobe - poröse, mikroporöse oder nanoporöse Schicht (bevorzugt auf der Basis von Polymeren, keramischen Materialien, Metallen oder Kompositen aufgebaut) sein kann.

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Anti-Fouling-Effekten.

Solche Oberflächen wurden bereits im Labormaßstab hergestellt. Unter anderem werden Salvinia-Effekt, hierarchische Strukturierung nach dem Vorbild von Notonecta, Haifischhaut- und Delfinhaut-Effekte erfolgreich eingesetzt.

Das Problem besteht darin, dass es unter widrigen Betriebsbedingungen (Brandung, Wellenschlag bei Schiffsbeschichtungen etc.) oder während langer Zeiträume stellenweise oder insgesamt zu einem teilweisen oder vollständigen, lokalen oder ausgedehnten Gasverlust kommt.

Nach dem erfindungsgemäßen Verfahren werden flächige Elemente zusammen mit einer Vorrichtung verwendet, die so beschaffen sind, dass sie nach partiellem oder totalem Gasverlust wieder mit Gas beladen werden können. Die erfindungsgemäße Vorrichtung zum Halten einer Luftschicht unter Wasser oder allg. einer Gasschicht unter Flüssigkeit, ist dadurch gekennzeichnet, dass die Schicht bei Luftverlust über eine gasdurchlässige Unterlage wieder beladen werden kann, wobei die gasdurchlässige Unterlage eine textile Unterlage, ein anderes Gewebe oder Filz, ein Flockmaterial, eine poröse oder gasdiffusionsgeeignete Keramikschicht, eine Metallschicht mit Poren, ein Metallfilz oder Drahtgeflecht, eine semipermeable Membran, eine - bevorzugt hydrophobe oder superhydrophobe - poröse, mikroporöse oder nanoporöse Schicht (bevorzugt auf der Basis von Polymeren, keramischen Materialien, Metallen oder Kompositen aufgebaut) sein kann.

In einer Variante des Verfahrens wird ein Aerenchym für das Nachladen des Gases verwendet.

Optionale Varianten und Merkmale betreffen eine
- Kombination von obiger Aufladung der Gasschicht ("Replenishing") mit zusätzlichen Vorrichtungen zum künstlichen Wiederbefüllen oder Nachfüllen der Gasschicht, etwa Kapillaren, Membranen, Textilien etc.
- Beschichtung der Oberflächen mit Teflon, Polytetrafluorethylen und deren Derivaten, insbesondere auch Mikro- und Nanopartikeln aus diesen Substanzen.
- Beschichtung der Oberflächen mit kommerziell erhältlichen Antihaftsprays oder auch Mikro- und Nanopartikeln.
- Verwendung von Oberflächenstrukturen aus Polymeren, Harzen, PDMS, Silizium, Siliziumdioxid und Siliziumhydroxid, Metallen, Stahl und Stahlfasern, Edelstahl, Epoxy.
- Einprägung der Oberflächenstrukturen in Lacke einschließlich Schiffslacke mit und ohne anschließende Oberflächenfunktionalisierung oder Beschichtung, beispielsweise mit Teflon oder Nanoteflon (bevorzugte Schichtdicke 0,15 nm bis 500 nm).
- Vorrichtung wie eben beschrieben, ggf. auch gekoppelt mit Mess- und/oder Steuerungs- und/oder Regeleinrichtungen, die den Zustand der Gasschicht insgesamt oder ortsaufgelöst messen und kontrollieren und im Bedarfsfall automatisch eine Nachfüllung von Gas veranlassen.
- Beschichtung von Schiffsoberflächen - ganz oder teilweise.
- Verwendung von metallischen Oberflächenstrukturen.
- Verwendung von Oberflächen mit durchgehender Luftschicht.
- Verwendung von Oberflächen, die ein regelmäßiges oder unregelmäßiges Muster von Gastaschen oder Gasbläschen unter Flüssigkeit ausbilden, aufbauen oder halten.
- Verwendung von hydrophoben oder superhydrophoben Oberflächen und Oberflächenstrukturen für die gashaltenden Oberflächen.
- Verwendung von bevorzugt flüssigkeitsabweisend beschichteten (für den Fall, dass die Flüssigkeit Wasser ist, hydrophob oder superhydrophob beschichteten) Härchen, Säulen, Krönchenstrukturen, Schneebesenstrukturen, Türmchen und anderen erhabenen Strukturen für die Oberflächen zur Gashaltung in oder zwischen diesen Strukturen.
- Verwendung von bevorzugt flüssigkeitsabweisend beschichteten (für den Fall, dass die Flüssigkeit Wasser ist, hydrophob oder superhydrophob beschichteten) von Mulden, Kuhlen, Löchern und Vertiefungen und anderen Vertiefungs-Strukturen für die Oberflächen zur Gashaltung in oder zwischen diesen Strukturen.
- Verwendung von einer Kombination von bevorzugt flüssigkeitsabweisend beschichteten (für den Fall, dass die Flüssigkeit Wasser ist, hydrophob oder superhydrophob beschichteten) Härchen, Säulen, Krönchenstrukturen, Schneebesenstrukturen, Türmchen und anderen erhabenen Strukturen sowie Mulden, Kuhlen Löchern und Vertiefungen für die Oberflächen zur Gashaltung in oder zwischen diesen Strukturen.
- Verwendung von Ventilen und Durchflussreglern und Stellelementen zur Steuerung und / oder zur Regelung der Gaszufuhr zu den Auslassöffnungen, die die Gasschicht erst- oder wiederbefüllen.
- Verwendung als Beschichtung auf Schiffen, in Strömungskanälen, in Rohren oder in chemischen Reaktionsgefäßen.

Mit anderen Worten können bevorzugte Gegenstände der Anmeldung wie folgt beschrieben sein:
Gegenstand 1 betrifft eine Vorrichtung und Oberflächenbeschichtung zum Beladen mit und Halten von einer Gasschicht unter Flüssigkeit, bestehend aus (i) einer strukturierten Oberfläche oder Oberflächenbeschichtung oder textilen Beschichtung, die eine Struktur aufweist, die es erlaubt, beim Untertauchen unter eine Flüssigkeit dauerhaft oder zumindest für kurze Zeit eine durchgehende oder nicht durchgehende Gasschicht zu halten, (letztere, i.e. die nicht-durchgehende Gasschicht beispielsweise bestehend aus regelmäßig oder unregelmäßig angeordneten Gasbläschen oder Gastaschen, den Gas Pockets oder Air Pockets, d.h. kleinen, unter Flüssigkeit mit Gas gefüllten Taschen in der Oberflächentopographie), wobei die Strukturierung der Oberfläche entweder eine regelmäßige oder unregelmäßige topographische Strukturierung ("Relief") oder eine räumlich variierende chemische Funktionalität (chemisches Muster oder "Chemical Pattern") oder eine Kombination von beidem sein kann und (ii) einer Vorrichtung, die es erlaubt, bei Gasverlust die Oberfläche wieder mit Gas zu beladen, wobei das Gas entweder (a) von außen, beispielsweise in Form kleiner vorbeiströmender Gasbläschen in der Flüssigkeit zugeführt wird, die dann von der strukturierten Schicht eingefangen werden oder (b) das Gas von innen aus der Schicht heraus zugeführt wird, beispielsweise aus Düsen oder Poren oder Kanälen oder einem Leitungssystem innerhalb oder unterhalb der strukturierten Schicht, wobei das Gas beispielsweise aus einem Gasreservoir, einem Druckgasreservoir, aus Druckgasflaschen oder mit Hilfe einer Pumpe zugeführt wird oder (c) das Gas direkt in der Schicht erzeugt wird, beispielsweise durch katalytische und / oder elektrochemische Zersetzung von Wasser oder (d) das Gas direkt der Flüssigkeit entzogen wird in Form von in der Flüssigkeit vorhandenem, gelösten Gas oder in Form von Flüssigkeit, die in die Gasschicht hinein verdunstet und das Gas oder einen Teil des Gases der durchgehenden oder nicht durchgehenden Gasschicht oder der Gasbläschen oder Air Pockets auf der Flüssigkeit bildet oder ersetzt ("Self Recharging Gas Layers"), wobei die Erst- oder Wiederbeladung beispielsweise durch einen Unterdruck in der Gasschicht hervorgerufen werden kann, den man durch eine relative Bewegung zwischen Flüssigkeit und Gasschicht hervorruft, beispielsweise durch ein Schiff, das sich relativ zum Wasser bewegt.
Gegenstand 2 betrifft eine Verwendung der Vorrichtung und Beschichtung gemäß Gegenstand 1, dadurch gekennzeichnet, dass diese dauernd oder zeitweise ganz oder teilweise einer Flüssigkeit ausgesetzte Oberfläche oder Grenzfläche durch eine durchgehende oder nicht durchgehende ständig oder zeitweise bestehende Gasschicht unter Flüssigkeit (i) vor einer Belagbildung, beispielsweise unter Mitwirkung von organischen und anorganischen Verbindungen sowie biologischen und biogenen Komponenten aus der Flüssigkeit, und / oder (ii) vor Korrosion und / oder (iii) vor chemischem Angriff durch die Flüssigkeit und / oder in der Flüssigkeit gelöste Gase, Moleküle, Komplexe, Tröpfchen (bei Emulsionen) oder Feststoffpartikel und / oder (iv) vor (Bio)Fouling geschützt wird, wobei es sich im Falle des Fouling insbesondere um Biofouling, insbesondere in Form von Mikrofouling, Makrofouling, den Angriff von Algen, Muscheln, Barnacles und / oder anderen Meeresorganismen oder eine Kombination dieser Formen handeln kann.
Gegenstand 3 betrifft eine Beschichtung gemäß Gegenstand 1, dadurch gekennzeichnet, dass die Vorrichtung und Beschichtung zum Schutz der Oberflächen auf der Oberfläche von Schiffen, Yachten, Booten und anderen Wasserfahrzeugen oder auf im Meer installierten technischen Einrichtungen und Bauwerken, insbesondere Ölplattformen, Offshore-Windkraftanlagen, Stahlkonstruktionen, Betonkonstruktionen oder anderen ortsfest oder nicht ortsfest im Meer oder im Süßwasser installierten technischen Einrichtungen, Bojen, Leitungen und Kabeln, Antriebseinrichtungen, Schiffsoberflächen, Schiffsschrauben und Steuervorrichtungen, Fenstern etc. die sich zeitweise oder permanent unter Wasser befinden oder von Wasser angespült werden, Schiffsrudern, Scheinwerfern und anderen Licht emittierenden optischen Funktionseinheiten angebracht wird.
Gegenstand 4 betrifft eine Vorrichtung und Beschichtung gemäß Gegenstand 1, dadurch gekennzeichnet, dass es sich bei dem Gas der durchgehenden oder nicht durchgehenden permanenten oder zeitweise existierenden Gasschicht um Luft, Stickstoff, Sauerstoff, Kohlendioxid, Argon, Helium oder Gasgemische handelt, die diese Gasen enthalten und / oder dass es sich bei der Flüssigkeit um Wasser, Salzwasser, Meereswasser oder um Alkohol oder um wässrige oder alkoholische Lösungen handelt.
Gegenstand 5 betrifft eine Vorrichtung und Beschichtung gemäß Gegenstand 1, wobei die Beschichtung der Oberfläche Oberflächenstrukturen, Säulen, Haare, Noppen enthält, die bevorzugt ganz oder teilweise eine hydrophobe oder superhydrophobe Oberfläche aufweisen, bevorzugt dadurch gekennzeichnet, dass diese Oberflächenstrukturen, Säulen, Haare, Noppen etc. wiederum mit einer dünnen, bevorzugt 0,1 nm bis 2 µm dicken, besonders bevorzugt 0,1 nm bis 100 nm dicken hydrophoben Schicht beschichtet sind.
Gegenstand 6 betrifft eine Beschichtung gemäß Gegenstand 1 oder 5, dadurch gekennzeichnet, dass die Beschichtung Oberflächenstrukturen, Säulen, Haare, Noppen oder andere Strukturen eine Höhe von 0,01 mm bis 5 mm haben und eine hydrophobe Oberfläche mit oder ohne hydrophile Patches, End- oder Seitenflächen aufweisen.
Gegenstand 7 betrifft eine Beschichtung gemäß Gegenstand 1, dadurch gekennzeichnet, dass die Gas haltende Beschichtung auf der Außen- oder Innenseite von Rohrleitungen oder von Reaktionsgefäßen für chemische Reaktionen oder auf der Innenseite von Behältnissen zur Aufbewahrung von Flüssigkeiten aufgebracht ist.
Gegenstand 8 betrifft eine Beschichtung gemäß Gegenstand 1, dadurch gekennzeichnet, dass die Lufthaltung unter Ausnutzung des Salvinia-Effektes, des Notonecta-Effektes oder durch hierarchisch strukturierte Oberflächen erfolgt.
Gegenstand 9 betrifft eine Vorrichtung und Beschichtung gemäß Gegenstand 1, dadurch gekennzeichnet, dass es sich bei der Oberflächenbeschichtung um Textilien, Gewebe, Faserverbünde oder um gaspermeable Beschichtungen, semipermeable Membranen oder mikro- bzw. nanoporöse Schichten handelt und die Gaszufuhr und / oder Wiederbeladung durch diese Poren und Kanäle in den Textilien, Gewebe, Faserverbünde oder um gaspermeable Beschichtungen, semipermeable Membranen oder mikro- bzw. nanoporöse Schichten erfolgt.
Gegenstand 10 betrifft eine Vorrichtung und Beschichtung gemäß Gegenstand 1, dadurch gekennzeichnet, dass die (Wieder)beladung der Oberfläche mit einer Gasschicht bzw. die Nachladung von Gas über kleine Düsen oder Mikrodüsen erfolgt, bevorzugt durch Düsen oder Mikrodüsen, die in die Oberflächenstruktur der strukturierten Oberfläche eingebettet sind, besonders bevorzugt in den Vertiefungen der Oberflächenschicht liegend, beispielsweise am Fuße der Härchen, Säulen oder anderer erhabener Oberflächenstrukturen, wobei in einer bevorzugten Variante die Strukturen bzw. Säulen selbst als Gaszufuhr-Düsen oder Mikrodüsen fungieren können.

Die **Figur 19a** zeigt eine aus schneebesenförmigen Elementen bestehende Oberflächenstruktur. Die **Figur 19b** zeigt eine aus krönchenförmigen Elementen bestehende Oberflächenstruktur. Die **Figur 19c** zeigt eine aus hydrophoben Härchen bestehende Oberflächenstruktur. Die **Figur 19d** zeigt eine aus türmchenförmigen Elementen bestehende Oberflächenstruktur.

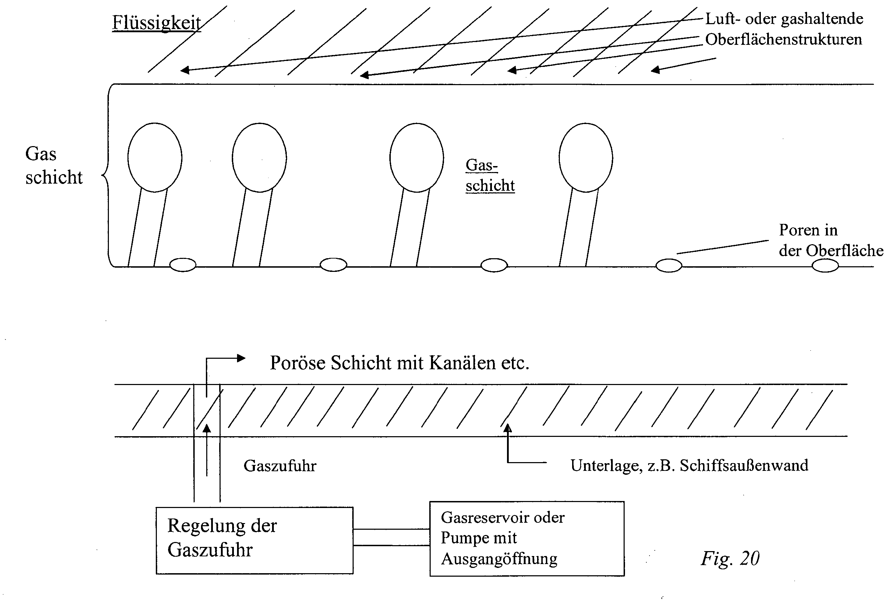

Die **Figur 20** zeigt eine Anordnung einer Oberflächenabdeckung bzw. einer gashaltenden Schicht an einer Schiffswand, wobei der gashaltenden Schicht von der wasserabgewandten Seite Gas zugeführt wird.

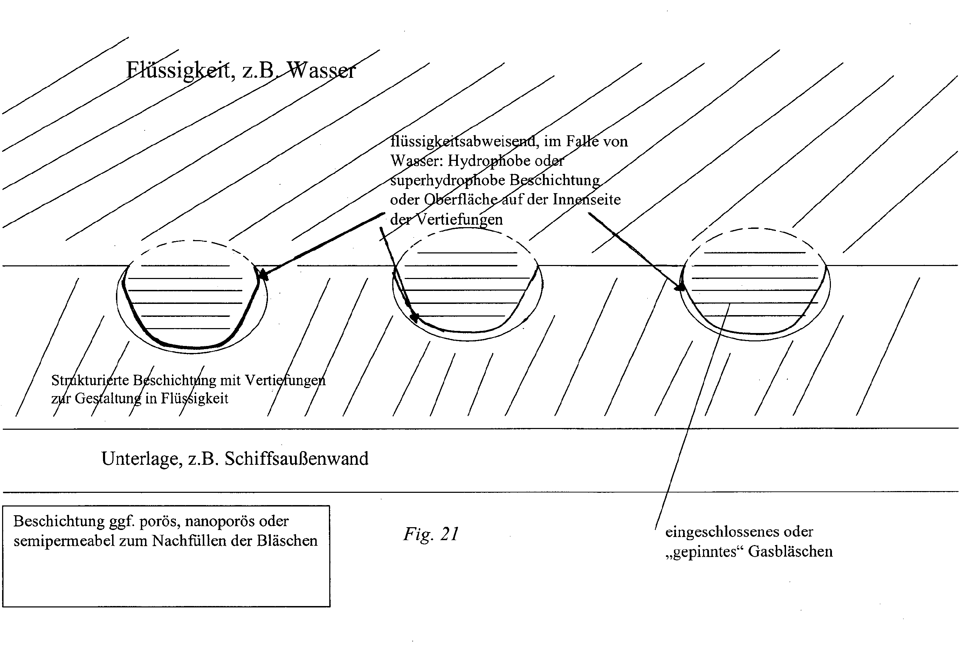

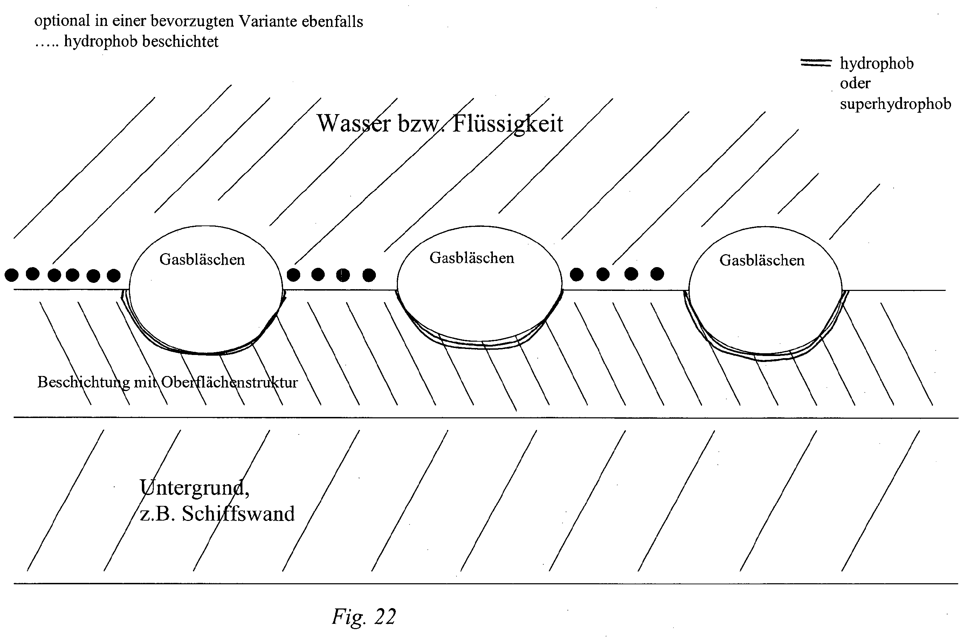

Die **Figur 21** zeigt eine aus Mulden bestehende Oberflächenstruktur. Die **Figur 22** zeigt ebenfalls eine aus Mulden bestehende Oberflächenstruktur, wobei die Oberfläche optional hydrophob beschichtet ist.

### Funktionselemente zum Aufbringen einer Gasschicht unter Flüssigkeit

Zusammenfassend beschreibt ein Aspekt Gasschichten auf Oberflächen unter Flüssigkeit, die von großem technologischem Interesse zur Reibungsreduktion bei Schiffen und in Pipelines sowie zur Erzielung von Schutz der Oberfläche vor Belagbildung, (Bio)Fouling, Korrosion und chemischem Angriff sind.

Durch eine geeignete Oberflächenstrukturierung gelingt es, eine an der Oberfläche anhaftende Schicht aus Gas oder Gasbläschen mit unter Flüssigkeit zu nehmen. Das Problem ist, dass solche Oberflächen oft komplex strukturiert sind und oft nur schwer großflächig hergestellt werden können, wie es beispielsweise für eine Schiffsbeschichtung notwendig wäre. Auch ist eine Herstellung direkt in der Schiffswerft oder auf der Offshore-Plattform etc. nur schwer möglich.

Diese Probleme werden gelöst durch die Aufbringung der besagten strukturierten, gashaltenden Oberfläche auf einen modularen Träger oder durch die Strukturierung der Oberfläche des Trägers selbst, wobei dieser modulare Träger eine starre oder elastische oder duktile "Fliese" oder "Kachel" oder Folie oder Folienelement sein kann, das bevorzugt aus Polymer, Keramik, Metall (Stahl, Kupfer, Silber etc.), Textil, einem porösen Werkstoff, einem Halbleitermaterial oder aus anderen Werkstoffen bestehen kann und eine strukturierte Oberfläche oder hierarchisch strukturierte Oberfläche aufweist, um die Luftschicht zu halten. Der modulare Träger selbst wird dann auf die Oberfläche aufgebracht, aufgeklebt, aufgeschraubt, aufgekittet, aufgelötet oder aufgeschweißt oder durch thermische Behandlung reversibel oder irreversibel mit dem Untergrund verbunden oder anderweitig befestigt auf der Oberfläche des Gegenstandes oder Objektes, das unter Flüssigkeit mit einer Gasschicht ausgestattet werden soll.

Ein Aspekt betrifft eine modulare Aufbringung von Elementen mit reibungsreduzierenden Eigenschaften oder Anti-Fouling-Eigenschaften oder mit unter Flüssigkeit gashaltenden oder eine Gasschicht erzeugenden oder aufbauenden Eigenschaften.

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Antifouling-Effekten.

Solche Oberflächen wurden bereits im Labormaßstab hergestellt. Unter anderem werden Salvinia-Effekt, hierarchische Strukturierung nach dem Vorbild von Notonecta sowie Haifischhaut- und Delfinhaut-Effekte erfolgreich eingesetzt.

Das Problem besteht in der reversiblen, auch nachträglichen, kostengünstigen Aufbringung teils komplexer Oberflächenstrukturen.

Nach dem erfindungsgemäßen Verfahren werden modulare, flächige Elemente verwendet, die die oben genannten Oberflächeneigenschaften besitzen und die auch nachträglich, etwa durch Aufkleben, auf die Oberfläche, beispielsweise die Außenseite von Schiffen oder die Innenseite von Rohrleitungen aufgebracht werden können.

Optionale Varianten und Merkmale betreffen eine
- Aufbringung in Form von Fliesen, Kacheln etc. mit den gewünschten Oberflächeneigenschaften;
- Aufbringung flexibler Flächenelemente;
- Aufbringung durch reversible oder irreversible Klebung;
- Beschichtung von Schiffsoberflächen - ganz oder teilweise;
- Verwendung von metallischen Oberflächenstrukturen;
- Verwendung von Oberflächen und / oder Oberflächenstrukturen aus Kupfer oder Silber;
- Verwendung von Oberflächen und / oder Oberflächenstrukturen aus Eisen oder Stahl;
- Verwendung von Oberflächen, Oberflächenstrukturen, Säulen, Spitzen, Geweben, Faserstrukturen, Faserfilzen und Geflechten aus Eisen oder Eisenlegierungen, Stahl oder Edelstahl ohne oder mit Beschichtung, in letzerem Fall bevorzugt durch dünne Polymerschichten;
- Verwendung von keramischen Oberflächenstrukturen;
- Verwendung von Oberflächen und / oder Oberflächenstrukturen aus Polymeren, Harzen, Resin, Epoxy;
- Verwendung von Oberflächen mit durchgehender Luftschicht;
- Verwendung von Oberflächen, die nur ein regelmäßiges oder unregelmäßiges Muster von Gastaschen oder Gasbläschen unter Flüssigkeit ausbilden, aufbauen oder halten;
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung;
- Verwendung als Beschichtung auf Schiffen, in Strömungskanälen, in Rohren oder in chemischen Reaktionsgefäßen;
- Kombination von obiger Aufladung der Gasschicht ("Replenishing") mit zusätzlichen Vorrichtungen zum künstlichen Wiederbefüllen oder Nachfüllen der Gasschicht, etwa Kapillaren, Membranen, Textilien etc.;
- Beschichtung der Oberflächen mit Teflon, Polytetrafluorethylen und deren Derivaten, insbesondere auch Mikro- und Nanopartikeln aus diesen Substanzen;
- Beschichtung der Oberflächen mit kommerziell erhältlichen Antihaftsprays oder auch Mikro- und Nanopartikeln;
- Verwendung von Oberflächenstrukturen aus Polymeren, Harzen, PDMS, Silizium, Siliziumdioxid und Siliziumhydroxid, Metallen, Stahl und Stahlfasern, Edelstahl, Epoxy;
- Einprägung der Oberflächenstrukturen in Lacke einschließlich Schiffslacke mit und ohne anschließende Oberflächenfunktionalisierung oder Beschichtung, beispielsweise mit Teflon oder Nanoteflon (bevorzugte Schichtdicke 0,15 nm bis 500 nm);
- Vorrichtung wie eben beschrieben, ggf. auch gekoppelt mit Mess- und/oder Steuerungs- und/oder Regeleinrichtungen, die den Zustand der Gasschicht insgesamt oder ortsaufgelöst messen und kontrollieren und im Bedarfsfall automatisch eine Nachfüllung von Gas veranlassen;
- Beschichtung von Schiffsoberflächen - ganz oder teilweise;
- Verwendung von metallischen Oberflächenstrukturen;
- Verwendung von Oberflächen mit durchgehender Luftschicht;
- Verwendung von Oberflächen, die ein regelmäßiges oder unregelmäßiges Muster von Gastaschen oder Gasbläschen unter Flüssigkeit ausbilden, aufbauen oder halten;
- Verwendung von hydrophoben oder superhydrophoben Oberflächen und Oberflächenstrukturen für die gashaltenden Oberflächen;
- Verwendung von bevorzugt flüssigkeitsabweisend beschichteten (für den Fall, dass die Flüssigkeit Wasser ist, hydrophob oder superhydrophob beschichteten) Härchen, Säulen, Krönchenstrukturen, Schneebesenstrukturen, Türmchen und anderen erhabenen Strukturen für die Oberflächen zur Gashaltung in oder zwischen diesen Strukturen;
- Verwendung von bevorzugt flüssigkeitsabweisend beschichteten (für den Fall, dass die Flüssigkeit Wasser ist, hydrophob oder superhydrophob beschichteten) von Mulden, Kuhlen, Löchern und Vertiefungen und anderen Vertiefungs-Strukturen für die Oberflächen zur Gashaltung in oder zwischen diesen Strukturen;
- Verwendung von einer Kombination von bevorzugt flüssigkeitsabweisend beschichteten (für den Fall, dass die Flüssigkeit Wasser ist, hydrophob oder superhydrophob beschichteten) Härchen, Säulen, Krönchenstrukturen, Schneebesenstrukturen, Türmchen und anderen erhabenen Strukturen sowie Mulden, Kuhlen Löchern und Vertiefungen für die Oberflächen zur Gashaltung in oder zwischen diesen Strukturen;
- Verwendung von Ventilen und Durchflussreglern und Stellelementen zur Steuerung und / oder zur Regelung der Gaszufuhr zu den Auslassöffnungen, die die Gasschicht erst- oder wiederbefüllen;
- Verwendung als Beschichtung auf Schiffen, in Strömungskanälen, in Rohren oder in chemischen Reaktionsgefäßen.

Eine Vorrichtung zum Halten einer Luftschicht unter Wasser oder allgemein einer Gasschicht unter Flüssigkeit, kann dadurch gekennzeichnet sein, dass die Gasschicht in einzelne "Compartments" unterteilt ist, Flächenelemente, die am Rande gegen das Entweichen von Gas im Randbereich speziell geschützt sind durch Zonen dichteren Haarbesatzes, durch hydrophile Stege oder Pins hoher Dichte oder durch hydrophile Wände mit Vorsprung, d.h. oben etwas überstehend.

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Anti-Fouling-Effekten.

Solche Oberflächen wurden bereits im Labormaßstab hergestellt. Unter anderem werden Salvinia-Effekt, hierarchische Strukturierung nach dem Vorbild von Notonecta, Haifischhaut- und Delfinhaut-Effekte erfolgreich eingesetzt.

Das Problem besteht aber darin, dass
(i) die Luft an den Rändern der Beschichtung entweicht und
(ii) bei ausgedehnten, mit Gas-Schichten beschichteten Oberflächen oft zwischen verschiedenen Stellen der Schicht erhebliche Druckunterschiede bestehen. Ein Beispiel ist eine vertikale Wand unter Wasser. Durch den hydrostatischen Druck, der linear mit der Wassertiefe zunimmt, sind unterschiedliche Stellen der Oberfläche unterschiedlichem statischem Druck ausgesetzt. Ist die Oberfläche mit einer Gas haltenden Schicht ausgerüstet und somit unter Flüssigkeit mit einer Gasschicht beschichtet, wird das Gas von Bereichen hohen Druckes in Bereiche niedrigen Druckes gedrückt. Es kommt also nicht zur Ausbildung einer homogen dicken Gasschicht und - was noch gravierender ist - die Gasschicht wird schließlich in den Zonen höheren Druckes entweichen.
(iii) Ein entsprechendes Problem besteht auch, wenn es sich bei den Druckgradienten in der Schicht nicht um solche aufgrund des hydrostatischen Druckes, sondern aufgrund dynamischer Druckunterschiede etwa aufgrund unterschiedlicher Strömungsgeschwindigkeiten der umgebenden Flüssigkeit an unterschiedlichen Orten der Oberfläche handelt.

Nach dem erfindungsgemäßen Verfahren wird das Problem gelöst, indem die Gas haltenden Oberflächen nicht eine durchgehende Gasschicht halten, sondern die Gasschicht in einzelne "Compartments" unterteilt ist, gegen Gasströmung dichte Segmente, von denen jede klein genug ist - bei Gravitationsdruck-Gradienten insbesondere eine ausreichend kleine vertikale Ausdehnung hat - dass die Druckunterschiede innerhalb eines Compartments klein genug sind, um eine erheblich inhomogene Verteilung des Gases innerhalb eines Compartments, also erhebliche Unterschiede in der Luftschichtdicke, zu vermeiden.

Nach einem weiteren erfindungsgemäßen Verfahren werden ferner die Ränder der Gas haltenden Oberfläche und die Ränder der einzelnen Compartments durch besondere Maßnahmen gegen ein Entweichen von Gas an den Rändern geschützt, wobei diese Maßnahmen in einer Erhöhung der Flächendichte der Gas haltenden Strukturen, in einer Vergrößerung der hydrophilen Pins, in einer Verwendung linear ausgedehnter Strukturen, in einer speziellen, hydrophilen Beschichtung der begrenzenden Stege, Haare oder Pins bestehen kann.

Optionale Varianten und Merkmale betreffen eine
- Aufbringung in Form von Fliesen, Kacheln etc. (im folgenden auch "Air Tiles" genannt) mit den gewünschten Oberflächeneigenschaften;
- Aufbringung flexibler Flächenelemente;
- Aufbringung durch reversible oder irreversible Klebung;
- Beschichtung von Schiffsoberflächen - ganz oder teilweise;
- Verwendung von metallischen Oberflächenstrukturen;
- Verwendung von Oberflächen mit durchgehender Luftschicht;
- Verwendung von Oberflächen, die nur ein regelmäßiges oder unregelmäßiges Muster von Gastaschen oder Gasbläschen unter Flüssigkeit ausbilden, aufbauen oder halten;
- Verwendung dieser Patterns aus Gastaschen oder Gasbläschen unter Flüssigkeit als eine Art Kugellager mit Kugeln aus Gas bzw. Luft - zur mechanischen Führung und / oder zur Reibungsreduzierung;
- Verwendung als Beschichtung auf Schiffen, in Strömungskanälen, in Rohren oder in chemischen Reaktionsgefäßen.

Das neuartige Konzept der Air-Tiles:
- Fliesen und Folien bzw. Folienelemente, die eine Gasschicht unter Flüssigkeit halten;
- Modular, flexibel, auf glatte oder raue, gekrümmte oder nicht gekrümmte Oberflächen beliebiger Größe aufbringbar;
- auf bestehende Oberflächen leicht aufbringbar;
- Fliesen mit einer Luftschicht - oder allgemeiner: einer Gasschicht - unter Flüssigkeit;
- modular;
- auf bestehende Oberflächen leicht aufbringbar;
- Fliesen, d.h. flächige Elemente beliebiger Form, die unter Flüssigkeit, beispielsweise unter Wasser, eine Gasschicht halten oder aufbauen;
- sowie Anordnungen aus solchen Elementen;
- sowie Geräte, etwa Schiffe oder Rohrleitungen, die solche Elemente an ihren äußeren oder inneren Oberflächen besitzen;
- Fliesen, d.h. flächige Elemente beliebiger Form, die unter Flüssigkeit, beispielsweise unter Wasser, eine Gasschicht halten oder aufbauen;
- sowie Anordnungen aus solchen Elementen;
- einschließlich Flächenelementen, die in periodischer Anordnung die Flächen komplett ausfüllen;
- sowie Geräte, etwa Schiffe oder Rohrleitungen, die solche Elemente an ihren äußeren oder inneren Oberflächen besitzen;
- Kein großer Druckgradient innerhalb einer Fliese;
- Hydrostatischer Druckunterschied innerhalb einer Fliese ist max. Dichte der Flüssigkeit x Höhe der Fliese x Erdbeschleunigung;
- Probleme durch Randeffekte werden durch Compartmentierung und Randversiegelung innerhalb einer Fliese gelöst;
- Bei Systemen mit nachladbarer Gasschicht können Zuleitungen oder Gasladesysteme in das Modul bzw. den modularen Träger integriert werden;
- Modulares Konzept;
- Einfache Montage;
- Einfacher Austausch & Auswechseln schadhafter Fliesen
- Durch flexiblen Träger auch auf gekrümmte Oberflächen aufbringbar;
- Reversible Beschichtung;
- Kleben und Entkleben sind technisch bereits gelöst;
- Erfordert keine bestimmte Schiffskonstruktion;
- Erfordert keinen speziellen Untergrund;
- Gut geeignet für den riesigen Refurbishing-Markt;
- Ebenso wie für Neuschiffe.

Das neuartige Konzept der Air-Tiles:
- Auch teilweise Beschichtungen sind möglich;
- Kurze Montagezeiten;
- Geeignet für unterschiedlichste Schiffsgrößen;
- Somit ist das Up-Scaling sehr leicht möglich;
- Man muss keine großen Flächen produzieren, sondern nur große Stückzahlen einzelner Fliesen bzw. Folienelemente oder Folienrollen;
- Unterschiedlichste Materialien für die lufthaltende Schicht, für das Trägermaterial und für den Kleber sind möglich;
- Dies erlaubt eine einfache Anpassung an die Umgebungsbedingungen (Süßwasser, Salzwasser etc.);
- Einzelne Elemente, bevorzugt. 0,2 cm x 0,2 cm bis 10 cm x 10 cm werden innerhalb der Oberfläche eines Trägerelementes ("Air Tile" oder "Fliese") als lufthaltende Flächeneinheit mit abschließendem - bevorzugt hydrophilem - Rand abgetrennt;
- Idee: Schaffung von Compartments zur Abtrennung gegen Luftaustausch zwischen den Compartments
- 10 cm x 10 cm bis 100 cm x 100 cm als bevorzugte Größe für die Air Tiles (mit Assembly von Compartments innerhalb jeder Fliese) und mit Assembly der Air Tiles zur Beschichtung größerer Flächen
- Reibungsreduktion mit modularer Beschichtung, ortsselektiv, wo und wie benötigt;
- Lufthaltung unter realen Betriebsbedingungen;
- Gleichzeitige Montage unterschiedlicher Typen von Tiles oder Folien oder Folienelementen mit unterschiedlicher Beschichtung an unterschiedlichen Stellen des zu beschichtenden Objektes, beispielsweise des Schiffes, wird durch den modularen Ansatz möglich, je nach lokal benötigter Funktion und lokal vorhandenen Druck- und Strömungsverhältnissen (zur Lufthaltung) und Lichtverhältnissen (bezüglich Biofouling relevant)

Modulares Konzept:
- Einfache Montage;
- Einfacher Austausch & Auswechseln schadhafter Fliesen;
- Weiterer Vorteil: Entkopplung von Produktionsstandort der Oberflächenbeschichtung vom Ort, an dem diese auf die Oberfläche, beispielsweise das Schiff, aufgebracht wird. Dies ist wichtig, da die Herstellung der teilweise komplex strukturierten Oberflächen zur Gashaltung unter Flüssigkeit einen speziellen Herstellungsprozess benötigen, der nicht ohne Weiteres neben jedem zu beschichtendem Schiff oder gar auf See im Offshorebereich als Produktionsstätte aufgebaut werden kann.
- Weiterer Vorteil: kompakte Herstellungsanlagen, da keine riesigen Oberflächen, sondern nur kleine Fliesen oder Folienelemente oder Folienbahnen hergestellt werden müssen.
- Weiterer Vorteil: keine Begrenzung der Größe der zu beschichtenden Oberflächen durch die Größe der Produktionsanlage: mit einer ausreichend großen Zahl kleiner Fliesen lassen sich beliebig große Flächen beschichten.
- Folglich Vorteil: Keine weiterer Scale-Up des Produktionsprozesses hin zu größeren Flächen über die Einzelfliese oder die einzelne Folienbahn oder das einzelne Folienelement (= ein Flächenelement bestimmter Größe und Form) erforderlich.
- Nicht toxisch;
- Nicht teuer;
- Niedriges Gewicht
- Optisch ansprechend und der Schiffsoptik anpassbar (Farbe, Aufdruck etc.);
- Nicht brennbar oder entflammbar;
- Leicht kombinierbar mit Fliesen, die auf anderen reibungsrteduzierenden Technologien beruhen (Fliesen mit Haifisch-Haut / Delphin-Haut, hydrophobe oder superhydrophobe Oberflächen etc.);
- Keine besonderen Fachkenntnisse bei der Aufbringung erforderlich
- Keine Spezialgeräte erforderlich;
- Preiswerte Massenfertigung eines Standard-Produktes im Werk statt aufwendige Spezialbeschichtung mit Spezialmaschinen vor Ort am Schiff.

Aufgabe: Persistent Air Layers Under Water
Inhalt: Lufthaltung an künstlichen Oberflächen
Lufthaltung:
- auf Dauer oder zeitl. Begrenzt
- unter Unterdruck
- im Strömungsgradienten

Vorteile und Eigenschaften
- Modulare Herstellung, an beliebige Objektgrößen und Objektformen anpassbar, flächen vollständig ausfüllend beschichtbar;
- Lufthaltung auf Dauer;
- Lufthaltung unter Unterdruck;
- Lufthaltung im Strömungsgradienten

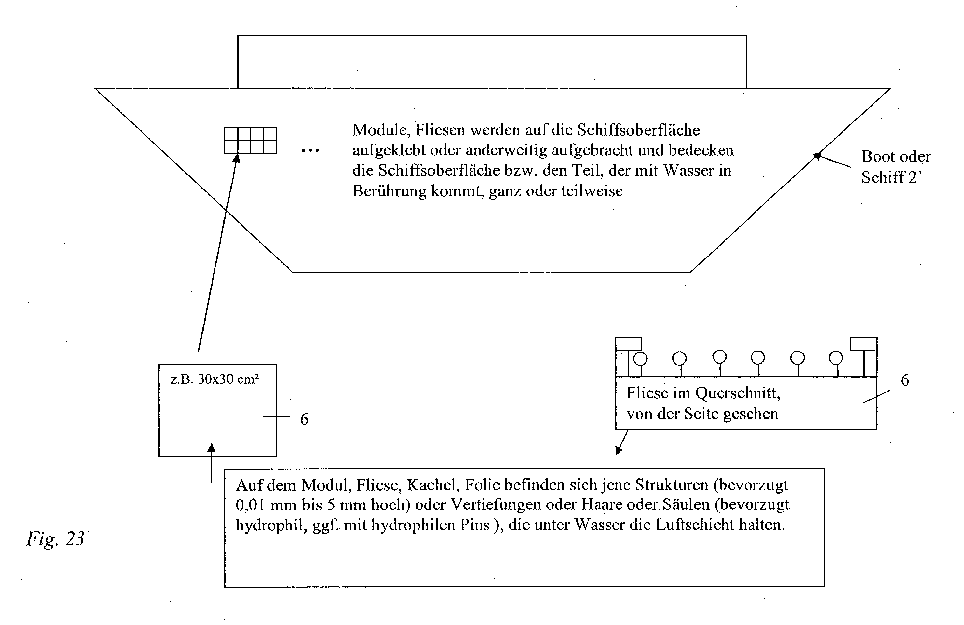

Die **Figur 23** zeigt ein Schiff 2', dessen Wandung mit einer Vielzahl von Fliesen 6 bzw. Kacheln ("Air-Tiles") versehen ist, so daß dort eine gashaltende Schicht die Schiffswandung vor dem Einfluß des Wassers schützt.

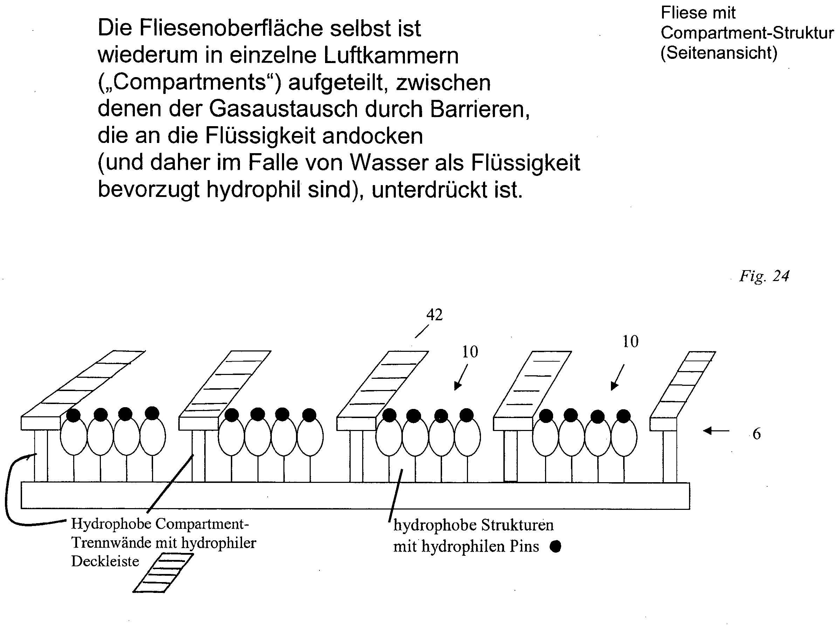

Die **Figur 24** zeigt eine Oberflächenabdeckung bzw. Fliese 6, welche Trennwände 42 aufweist, um eine Vielzahl von Bereichen der gashaltenden Schicht 10 fluidisch voneinander zu trennen.

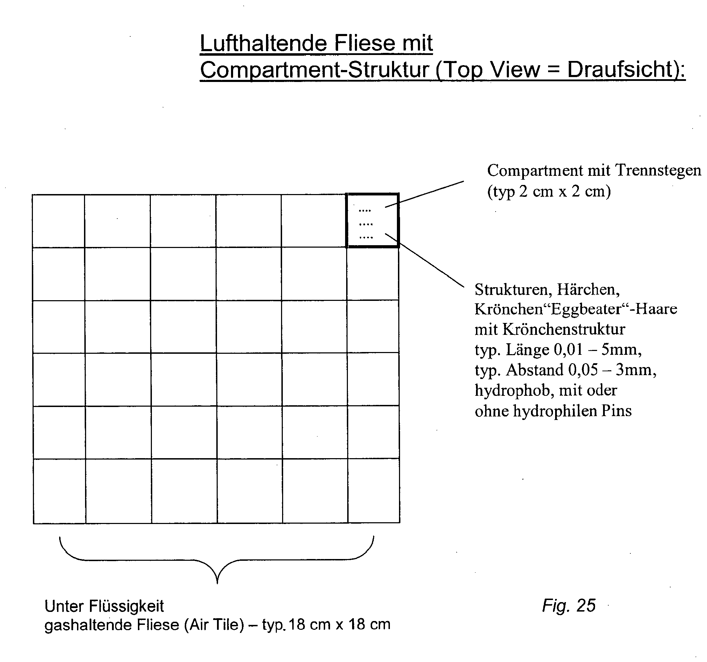

Die **Figur 25** zeigt eine Fliese bzw. Kachel ("Air-Tiles").

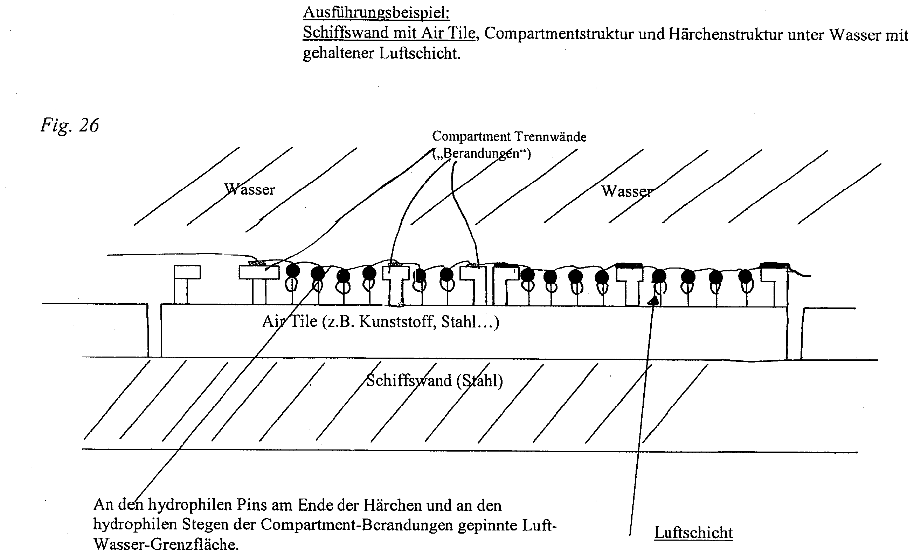

Die **Figur 26** zeigt einen Schnitt durch eine Schiffswandung, die mit Fliesen 6 bzw. Kacheln ("Air-Tiles") versehen ist.

Zusammenfassend beschreibt ein Aspekt eine Verwendung von Luft haltenden bzw. Gas haltenden Oberflächen unter Wasser oder einer anderen Flüssigkeit zum Schutz von Oberflächen vor Korrosion durch die Flüssigkeit oder durch Komponenten, Ionen oder Zusätze und Bestandteile, die in der Flüssigkeit enthalten sind - unter Einschluss auch von möglichen reaktiven Feststoffpartikeln, die sich in der Flüssigkeit befinden.

Der Schutz von Oberflächen vor Korrosion ist von großer technischer Bedeutung. Insbesondere sind Festkörperoberflächen, beispielsweise Metalloberflächen unter Flüssigkeit, beispielsweise unter Wasser, insbesondere unter Salzwasser, starken Korrosionsangriffen ausgesetzt. Hier hilft die Beschichtung mit korrosionsschützenden Lacken, was aber auch mit erheblichen Nachteilen verbunden ist. Dabei sind insbesondere zu nennen: (i) Lacke werden mit der Zeit brüchig und rissig bzw. lösen sich ab, (ii) sie geben oft auch toxische Bestandteile an das Wasser ab, (iii) sie haben bei Anwendung im Bereich höherer Temperaturen oft nur eine begrenzte Langzeit-Temperaturbeständigkeit und (iv) sie sind - insbesondere bei Anwendung unter chemisch aggressiven Medien wie Säuren, Laugen, starken Oxidationsmitteln oder Reduktionsmitteln, oft selbst gegen das flüssige Medium nicht ausreichend beständig.

Gelöst werden diese vier Probleme nach dem erfindungsgemäßen Verfahren, indem man das flüssige Medium gar nicht mit dem Gefäß oder dem Rohr oder einer sonstigen Wandung in Berührung kommen lässt, sondern "Gefäße und Behältnisse aus Luft (oder einem anderen Gas) verwendet, d.h. zwischen die eigentliche Gefäßwand oder Rohrwand oder andere Begrenzung eine Schicht aus einem (vorzugsweise in der gegebenen chemischen Umgebung inertes) Gas als Gasschicht auf die Oberfläche aufbringt und damit eine Berührung zwischen reaktiver Flüssigkeit und Gefäßwand vermeidet, wobei für die Aufbringung einer persistenten Gasschicht auf die Oberfläche eine Gas haltende Beschichtung, beispielsweise unter Nutzung des Salvinia- oder des Notonecta-Effektes, aufgebracht ist.

Optionale Varianten und Merkmale betreffen eine
- Nutzung der Gasschicht auch zum Gasaustausch, zum Einbringen von Reaktionsgasen (Edukten), zur Entfernung von Reaktionsprodukten oder zum Reinigen und Spülen.
- Aufbringung flexibler, Gas haltender Flächenelemente.
- Beschichtung von Schiffsoberflächen - ganz oder teilweise.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Gasschicht oder dieser Patterns aus Gastaschen oder Gasbläschen unter Flüssigkeit auch zur Reibungsreduzierung.
- Verwendung als Beschichtung auf Schiffen, in Strömungskanälen, in Rohren oder in chemischen Reaktionsgefäßen.

Die Herstellung von unter Flüssigkeit Gas haltenden Oberflächen unter Verwendung von einem periodischen oder nicht-periodischen Array metallischer Pins (kleine Metallstäbchen oder -Dornen oder Drähte, die senkrecht oder geneigt zur Oberfläche, auf die sie aufgebracht wurden, stehen), bevorzugt aus Edelstahl, mit oder ohne Kapillare zur Wiederbefüllung der Gasschicht, mit oder ohne Nutzung des Salvinia-Effektes unter Nutzung eines hydrophilen Bereichs am Ende eines ansonsten hydrophoben Metall-Pins.

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Antifouling-Effekten.

Das Problem besteht darin, solche Oberflächen, etwa nach dem Vorbild von Salvinia molesta oder Notonecta glauca, technisch so zu implementieren, dass sie unter den mechanisch und chemisch anspruchsvollen Betriebsbedingungen etwa der Hochseeschifffahrt über hohe Lebensdauern bzw. Standzeiten verfügen:
- So muss bei erheblichem Wellengang und Brandung eine mechanische Stabilität der Beschichtung gewährleistet sein
- sowie unter der korrosiven Wirkung des Meerwassers eine entsprechende Korrosionsbeständigkeit.

Ein ideales Material, das beiden genannten Bedingungen gerecht wird, ist Edelstahl. Nach dem erfindungsgemäßen Verfahren werden daher entsprechende unter Wasser lufthaltende Schichten so ausgeführt, dass wesentliche Komponenten der Schicht, bevorzugt auch die Haare, Säulen oder anderweitigen auf die Oberfläche zur Haltung der Luftschicht aufgebrachten Strukturen aus Stahl, bevorzugt aus rostfreiem Edelstahl bestehen.

Ein weiterer Vorteil ist, dass in einer Variante des Verfahrens die lufthaltenden Strukturen ganz oder teilweise als Edelstahlkanülen ausgebildet sein können, an deren Seite oder an deren Ende sich eine Öffnung befindet, über die die Luft- oder Gasschicht bei Gasverlust wieder mit Gas befüllt ("nachgeladen") werden kann.

Weitere Varianten bzw. Merkmale betreffen eine
- Aufbringung von Metallspänen oder Stahlspänen.
- Aufbringung von Arrays kleiner Metallstifte.
- Verwendung von Stahl, Eisen und Eisenlegierungen, aber auch anderen Metallen oder Carbonfasern, als lufthaltende Strukturen.
- Verwendung von Metallfilzen oder Metalldrahtgeflechten oder -Geweben.
- Verwendung von metallischen Oberflächenstrukturen durch Prägung oder anderweitige Bearbeitung von metallischen Oberflächen.
- Verwendung von geprägten oder anderweitig strukturierten Metallblechen oder Metallfolien einschließlich der Verwendung dünner und dünnster Stahlbleche.
- Verwendung von Oberflächenstrukturen, die nur ein regelmäßiges oder unregelmäßiges Muster von Gastaschen oder Gasbläschen unter Flüssigkeit ausbilden, aufbauen oder halten anstelle einer durchgehenden Gasschicht.
- Verwendung zur Reibungsreduzierung in Flüssigkeit.
- Verwendung als Beschichtung auf Schiffen, in Strömungskanälen, in Rohren oder in chemischen Reaktionsgefäßen.

Verwendung von Luft haltenden bzw. Gas haltenden Oberflächen unter Wasser oder einer anderen Flüssigkeit zum Schutz vor Adhäsion an der festen Wandung oder Gefäßoberfläche beim Erstarren der Flüssigkeit, beispielsweise beim Gefrieren von Wasser.

Gefäße, in denen ein Medium zum Erstarren gebracht werden soll, beispielsweise Wasser, etwa in einem IceMaker oder im Eisfach eines Kühlschrankes, haben meist zwei Probleme: (i) die thermische Kontraktion oder Expansion während der Erstarrung und (ii) die Adhäsion des erstarrten Mediums an der Gefäßwandung.

Nach dem erfindungsgemäßen Verfahren wird das Problem der Anhaftung gelöst, indem das Gefäß mit einer Gas haltenden Beschichtung ausgestattet wird und somit die Flüssigkeit selbst nicht mit den Gefäß- oder Rohrwänden in Berührung kommt. Macht man die Gas haltenden Strukturen sowohl elastisch - bevorzugt gegen die Oberfläche geneigt - als auch lang genug, dass sie die Expansion oder Kontraktion bei der Phasenumwandlung durch Deformation der Strukturen sowie durch Änderung ihres Neigungswinkels und die simultane Änderung des Gasvolumens der Schicht kompensieren können, ist auch das Problem (i) gelöst. Alternativ lassen sich elastische Gefäßwände, beispielsweise aus Gummi, Silikonkautschuk etc., verwenden, die dann die Gas haltende Beschichtung tragen.

Weitere Varianten bzw. Merkmale betreffen eine
- Aufbringung in Form von Fliesen, Kacheln etc. mit den gewünschten Oberflächeneigenschaften.
- Aufbringung flexibler Flächenelemente.
- Aufbringung durch reversible oder irreversible Klebung.
- Beschichtung von Gefäßwänden und Oberflächen - ganz oder teilweise.
- Verwendung von metallischen Oberflächenstrukturen.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung.
- Verwendung als Beschichtung in Rohren oder in chemischen Reaktionsgefäßen.

Vorrichtung zum Halten einer Luftschicht unter Wasser oder allg. einer Gasschicht unter Flüssigkeit, dadurch gekennzeichnet, dass sich die Gasschicht nicht durchgehend ausbildet, sondern in Form eines Arrays kleiner Gasblasen an vordefinierten Stellen auf der Oberfläche ausbildet, in bevorzugter Ausführung so gestaltet, dass sich die Gasbläschen an vordefinierten Stellen auf der Oberfläche ausbilden, die durch topographische Struktur und/oder durch die chemische Funktionalisierung der Oberfläche eine energetische Bevorzugung für die Stabilisierung der Gasbläschen bilden, wobei in einer bevorzugten Variante des Verfahrens Nukleationszentren an den Stellen gesetzt werden, an denen sich die Gasbläschen bilden sollen und die Nukleationszentren dadurch ausgezeichnet sind, dass sie - etwa durch eine Stelle erhöhter topographischer Rauigkeit oder chemischer Inhomogenität der Oberfläche - die Aktivierungsenergie zur Bildung eines Gasbläschens lokal an definierter Stelle herabsetzen.

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Anti-Fouling-Effekten.

Solche Oberflächen wurden bereits im Labormaßstab hergestellt. Unter anderem werden Salvinia-Effekt, hierarchische Strukturierung nach dem Vorbild von Notonecta, Haifischhaut- und Delfinhaut-Effekte erfolgreich eingesetzt.

Das Problem besteht aber darin, dass
(i) die Luft an den Rändern der Beschichtung entweicht und
(ii) bei ausgedehnten, mit Gas-Schichten beschichteten Oberflächen oft zwischen verschiedenen Stellen der Schicht erhebliche Druckunterschiede bestehen. Ein Beispiel ist eine vertikale Wand unter Wasser. Durch den hydrostatischen Druck, der linear mit der Wassertiefe zunimmt, sind unterschiedliche Stellen der Oberfläche unterschiedlichem statischem Druck ausgesetzt. Ist die Oberfläche mit einer Gas haltenden Schicht ausgerüstet und somit unter Flüssigkeit mit einer Gasschicht beschichtet, wird das Gas von Bereichen hohen Druckes in Bereiche niedrigen Druckes gedrückt. Es kommt also nicht zur Ausbildung einer homogen dicken Gasschicht und - was noch gravierender ist - die Gasschicht wird schließlich in den Zonen höheren Druckes entweichen.
(iii) Ein entsprechendes Problem besteht auch, wenn es sich bei den Druckgradienten in der Schicht nicht um solche aufgrund des hydrostatischen Druckes, sondern aufgrund dynamischer Druckunterschiede etwa aufgrund unterschiedlicher Strömungsgeschwindigkeiten der umgebenden Flüssigkeit an unterschiedlichen Orten der Oberfläche handelt.
(iv) Das Problem besteht ferner in der reversiblen, auch nachträglichen, kostengünstigen Aufbringung teils komplexer Oberflächenstrukturen.
(v) Hinzu kommt der Wunsch nach einer einfachen Möglichkeit der Regenerierung oder - idealerweise - der Selbstregenerierung der Luftschicht.

Statt einer durchgehenden Luftschicht oder Compartments von Luftschichten kann eine Anordnung einzelner Luftbläschen verwendet werden, die sich, induziert durch Oberflächenstrukturen, an gewünschten Stellen bevorzugt bilden. Hierzu wird eine Vorrichtung zum Halten einer Luftschicht unter Wasser oder allg. einer Gasschicht unter Flüssigkeit hergestellt und verwendet, dadurch gekennzeichnet dass sich die Gasschicht nicht durchgehend ausbildet, sondern in Form eines Arrays kleiner Gasblasen an vordefinierten Stellen auf der Oberfläche ausbildet, in bevorzugter Ausführung so gestaltet, dass sich die Gasbläschen an vordefinierten Stellen auf der Oberfläche ausbilden, die durch topographische Struktur und/oder durch die chemische Funktionalisierung der Oberfläche eine energetische Bevorzugung für die Stabilisierung der Gasbläschen bilden, wobei in einer bevorzugten Variante des Verfahrens Nukleationszentren an den Stellen gesetzt werden, an denen sich die Gasbläschen bilden sollen und die Nukleationszentren dadurch ausgezeichnet sind, dass sie - etwa durch eine Stelle erhöhter topographischer Rauigkeit oder chemischer Inhomogenität der Oberfläche - die Aktivierungsenergie zur Bildung eines Gasbläschens lokal an definierter Stelle herabsetzen.

In einer Variante der genannten Vorrichtung werden flächige Elemente verwendet, die die oben genannten Oberflächeneigenschaften besitzen und die auch nachträglich, etwa durch Aufkleben, auf die Oberfläche, beispielsweise die Außenseite von Schiffen oder die Innenseite von Rohrleitungen aufgebracht werden können.

Weitere Varianten bzw. Merkmale betreffen eine
- Aufbringung in Form von Fliesen, Kacheln etc. mit den gewünschten Oberflächeneigenschaften.
- Aufbringung flexibler Flächenelemente.
- Aufbringung durch reversible oder irreversible Klebung.
- Beschichtung von Schiffsoberflächen - ganz oder teilweise
- Verwendung von metallischen Oberflächenstrukturen.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung.
- Verwendung als Beschichtung auf Schiffen, in Strömungskanälen, in Rohren oder in chemischen Reaktionsgefäßen.

Vorrichtung zum Halten einer Luftschicht unter Wasser oder allg. einer Gasschicht unter Flüssigkeit, dadurch gekennzeichnet, dass sich die Gasschicht unter Flüssigkeit selbständig ausbildet, d.h. es muss keine Luftschicht unter Wasser eingebracht werden, sondern die durchgehende oder nicht durchgehende Gasschicht (letztere in Form von Gasbläschen auf Oberflächen) bildet sich von alleine aus, etwa unter Verwendung von in der Flüssigkeit gelösten Gasmolekülen oder unter Verdunstung der Flüssigkeit unter Unterdruck, dadurch gekennzeichnet, dass durch topographische und/oder chemische Strukturierung Bereiche hergestellt werden, in denen das Eindringen der Flüssigkeit so hohe Oberflächenenergie kosten würde, dass sich von alleine unter Flüssigkeit flüssigkeitsfreie Bereiche ausbilden ("Air Pockets") - in einer Variante des Verfahrens durch die periodische oder nicht-periodische Belegung der Oberfläche mit Haaren oder Dornen einer bestimmten Form, bevorzugterweise mit nicht-benetzenden Haaren oder anderen Strukturen, die eine offenen oder geschlossene "Krone" bilden, die die sich bildende Gasblase ganz oder teilweise umschließt

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Anti-Fouling-Effekten.

Solche Oberflächen wurden bereits im Labormaßstab hergestellt. Unter anderem werden Salvinia-Effekt, hierarchische Strukturierung nach dem Vorbild von Notonecta, Haifischhaut- und Delfinhaut-Effekte erfolgreich eingesetzt.

Das Problem besteht darin, dass es unter widrigen Betriebsbedingungen (Brandung, Wellenschlag bei Schiffsbeschichtungen etc.) oder während langer Zeiträume stellenweise oder insgesamt zu einem teilweisen oder vollständigen, lokalen oder ausgedehnten Gasverlust kommt.

Ein anschließendes künstliches Wiederbefüllen oder Nachfüllen mit Gas ist aufwendig, muss überwacht werden und benötigt geeignete, ggf. kostspielige Vorrichtungen zur Befüllung und Überwachung, bei Compartment-Struktur der Luftschicht sogar eine Befüllungsmöglichkeit je Compartment, bei Gasbläschenstruktur sogar eine Befüllungsmöglichkeit je Gasbläschen.

Dieses Problem kann dadurch gelöst werden, dass eine Vorrichtung zum Halten einer Luftschicht unter Wasser oder allg. einer Gasschicht unter Flüssigkeit verwendet wird, dadurch gekennzeichnet, dass sich die Gasschicht unter Flüssigkeit selbständig ausbildet, d.h. es muss keine Luftschicht unter Wasser eingebracht werden, sondern die durchgehende oder nicht durchgehende Gasschicht (letztere in Form von Gasbläschen auf Oberflächen) bildet sich von alleine aus, etwa unter Verwendung von in der Flüssigkeit gelösten Gasmolekülen oder unter Verdunstung der Flüssigkeit unter Unterdruck, dadurch gekennzeichnet, dass durch topographische und/oder chemische Strukturierung Bereiche hergestellt werden, in denen das Eindringen der Flüssigkeit so hohe Oberflächenenergie kosten würde, dass sich von alleine unter Flüssigkeit flüssigkeitsfreie Bereiche ausbilden ("Air Pockets") - in einer Variante des Verfahrens durch die periodische oder nicht-periodische Belegung der Oberfläche mit Haaren oder Dornen einer bestimmten Form, bevorzugterweise mit nicht-benetzenden Haaren oder anderen Strukturen, die eine offenen oder geschlossene "Krone" bilden, die die sich bildende Gasblase ganz oder teilweise umschließt.

In einer Variante der genannten Vorrichtung werden flächige Elemente verwendet, die die oben genannten Oberflächeneigenschaften besitzen und die auch nachträglich, etwa durch Aufkleben, auf die Oberfläche, beispielsweise die Außenseite von Schiffen oder die Innenseite von Rohrleitungen aufgebracht werden können.

Weitere Varianten bzw. Merkmale betreffen eine
- Kombination von obiger Selbstaufladung der Gasschicht und zusätzlichen Vorrichtungen zum künstlichen Wiederbefüllen oder Nachfüllen der Gasschicht, etwa Kapillaren, Membranen, Textilien etc.,
- Vorrichtung wie eben beschrieben, ggf. auch gekoppelt mit Mess- und/oder Steuerungs- und/oder Regeleinrichtungen, die den Zustand der Gasschicht insgesamt oder ortsaufgelöst messen und kontrollieren und im Bedarfsfall automatisch eine Nachfüllung veranlassen.
- Aufbringung in Form von Fliesen, Kacheln etc. mit den gewünschten Oberflächeneigenschaften.
- Aufbringung flexibler Flächenelemente.
- Aufbringung durch reversible oder irreversible Klebung.
- Beschichtung von Schiffsoberflächen - ganz oder teilweise.
- Verwendung von metallischen Oberflächenstrukturen.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung.
- Verwendung als Beschichtung auf Schiffen, in Strömungskanälen, in Rohren oder in chemischen Reaktionsgefäßen.

Vorrichtung zum Halten von Luft- oder Gasbläschen ("Air Pockets") unter Flüssigkeit, dadurch gekennzeichnet, dass Haare mit einer offenen oder geschlossenen Krönchenstruktur oder auch Gruppen von einfachen, gekrümmten Haaren direkt auf der Oberfläche verwendet werden, so dass die Haare als Gruppe ein Krönchen, d.h. ein offenes "Behältnis" bilden, in dem die Luft- oder Gasblase umschlossen und auch im strömenden Medium oder gegen den Auftrieb unter Flüssigkeit festgehalten wird - in bevorzugter Ausführung mit einer Ausstattung der Haaroberflächen mit einer hydrophoben oder superhydrophoben Oberfläche oder Oberflächenbeschichtung, ebenfalls bevorzugt mit elastischen Haaren, die sich bei mechanischer Belastung gemeinsam mit der Gasblase verformen können, ferner - in einer Variante der Erfindung - Verwendung der Gasblasen in den Krönchen als Nukleationszentren für die selbständige oder künstliche Wiederauffüllung einer Gasschicht, ausgehend von Gasblasen in den Krönchen.

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Anti-Fouling-Effekten.

Solche Oberflächen wurden bereits im Labormaßstab hergestellt. Unter anderem werden Salvinia-Effekt, hierarchische Strukturierung nach dem Vorbild von Notonecta, Haifischhaut- und Delfinhaut-Effekte erfolgreich eingesetzt.

Ein Problem besteht in der kontrollierten Nukleation und Halten einzelnen Gasbläschen an definierter Stelle und deren Festhalten gegen äußere Kräfte.

Nach dem erfindungsgemäßen Verfahren wird eine Vorrichtung zum Halten von Luft- oder Gasbläschen ("Air Pockets") unter Flüssigkeit hergestellt und eingesetzt, dadurch gekennzeichnet, dass Haare mit einer offenen oder geschlossenen Krönchenstruktur oder auch Gruppen von einfachen, gekrümmten Haaren direkt auf der Oberfläche verwendet werden, so dass die Haare als Gruppe ein Krönchen, d.h. ein offenes "Behältnis" bilden, in dem die Luft- oder Gasblase umschlossen und auch im strömenden Medium oder gegen den Auftrieb unter Flüssigkeit festgehalten wird - in bevorzugter Ausführung mit einer Ausstattung der Haaroberflächen mit einer hydrophoben oder superhydrophoben Oberfläche oder Oberflächenbeschichtung, ebenfalls bevorzugt mit elastischen Haaren, die sich bei mechanischer Belastung gemeinsam mit der Gasblase verformen können, ferner - in einer Variante der Erfindung - Verwendung der Gasblasen in den Krönchen als Nukleationszentren für die selbständige oder künstliche Wiederauffüllung einer Gasschicht, ausgehend von Gasblasen in den Krönchen.

In einer Variante des Verfahrens gelangen offene oder geschlossene Krönchen zur Verwendung, die sich nicht direkt auf der Oberfläche befinden, sondern die sich am Ende eines Stieles oder eines mehradrigen oder gegabelten Stieles befinden (offene oder geschlossene "Schneebesenstruktur").

In einer weiteren Variante der genannten Vorrichtung werden flächige Elemente verwendet, die die oben genannten Oberflächeneigenschaften besitzen und die auch nachträglich, etwa durch Aufkleben, auf die Oberfläche, beispielsweise die Außenseite von Schiffen oder die Innenseite von Rohrleitungen aufgebracht werden können.

Weitere Varianten bzw. Merkmale betreffen eine
- Verwendung elastischer Haare oder Strukturen zur Ausbildung der Krönchenstruktur.
- Verwendung von Härchen oder Krönchen, die ganz oder teilweise hydrophobe oder superhydrophobe Oberflächen aufweisen oder ganz oder teilweise mit einer Beschichtung versehen sind, die hydrophob oder superhydrophob ist.
- Verwendung offener oder geschlossener Krönchenstrukturen.
- Verwendung in Kombination mit einer Gas haltenden Unterlage-Schicht ("Aerenchym") .
- Herstellung der Krönchenstrukturen aus Polymeren, Metallen oder keramischen Werkstoffen.
- Herstellung der Krönchenstrukturen durch Abformung von Masterstrukturen und/oder durch dreidimensionale Laserstrukturierung oder Herstellung von Masterstrukturen durch dreidimensionale Laserstrukturierung bzw. Laserlithographie und anschließende Abformung dieser Strukturen ohne oder nach einem erfolgtem Inversionsprozess.
- Kombination von obiger Selbstaufladung der Gasschicht und zusätzlichen Vorrichtungen zum künstlichen Wiederbefüllen oder Nachfüllen der Gasschicht, etwa Kapillaren, Membranen, Textilien etc.,
- Vorrichtung wie eben beschrieben, ggf. auch gekoppelt mit Mess- und/oder Steuerungs- und/oder Regeleinrichtungen, die den Zustand der Gasschicht insgesamt oder ortsaufgelöst messen und kontrollieren und im Bedarfsfall automatisch eine Nachfüllung veranlassen.
- Aufbringung in Form von Fliesen, Kacheln etc. mit den gewünschten Oberflächeneigenschaften.
- Aufbringung flexibler Flächenelemente.
- Aufbringung durch reversible oder irreversible Klebung.
- Beschichtung von Schiffsoberflächen - ganz oder teilweise.
- Verwendung von metallischen Oberflächenstrukturen.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung.
- Verwendung als Beschichtung auf Schiffen, in Strömungskanälen, in Rohren oder in chemischen Reaktionsgefäßen.

Vorrichtung zum Halten einer Luftschicht unter Wasser oder allg. einer Gasschicht unter Flüssigkeit, dadurch gekennzeichnet, dass sich unter der Schicht, die die Luft, beispielsweise nach dem Vorbild von Notonecta glauca oder Salvinia molesta, hält, eine poröse, mikroporöse oder nanoporöse, hydrophobe oder superhydrophobe Schicht befindet, das als Luftspeicher wirken kann bei mechanischer Belastung der Luftschicht, so dass die Luftschicht nicht freigegeben, sondern in die Luftspreicher-Schicht gedrückt wird und nach der Belastung wieder ganz oder teilweise freigegeben wird.

Das Halten einer Gasschicht unter Flüssigkeiten, beispielsweise unter Wasser, ist von großem technischem Interesse. Großes Anwendungspotential für solche Oberflächen besteht beispielsweise im Bereich von Schiffsbeschichtungen, unter anderem zur Reibungsreduktion und zur Erzielung von Anti-Fouling-Effekten.

Solche Oberflächen wurden bereits im Labormaßstab hergestellt. Unter anderem werden Salvinia-Effekt, hierarchische Strukturierung nach dem Vorbild von Notonecta, Haifischhaut- und Delfinhaut-Effekte erfolgreich eingesetzt.

Das Problem besteht darin, dass es unter widrigen Betriebsbedingungen (Brandung, Wellenschlag bei Schiffsbeschichtungen etc.) oder während langer Zeiträume stellenweise oder insgesamt zu einem teilweisen oder vollständigen, lokalen oder ausgedehnten Gasverlust.

Ein anschließendes künstliches Wiederbefüllen oder Nachfüllen mit Gas ist aufwendig, muss überwacht werden und benötigt geeignete, ggf. kostspielige Vorrichtungen zur Befüllung und Überwachung, bei Compartment-Struktur der Luftschicht sogar eine Befüllungsmöglichkeit je Compartment, bei Gasbläschenstruktur sogar eine Befüllungsmöglichkeit je Gasbläschen.

Der Luftverlust bei Druckbelastungen der Schicht wird vermieden, indem man geschützte Rückzugsmöglichkeiten für die Luft schafft. Dies geschieht durch die Verwendung einer Vorrichtung zum Halten einer Luftschicht unter Wasser oder allg. einer Gasschicht unter Flüssigkeit, dadurch gekennzeichnet, dass sich unter der Schicht, die die Luft, beispielsweise nach dem Vorbild von Notonecta glauca oder Salvinia molesta, hält, eine poröse, mikroporöse oder nanoporöse, hydrophobe oder superhydrophobe Schicht befindet, das als Luftspeicher wirken kann bei mechanischer Belastung der Luftschicht, so dass die Luftschicht nicht freigegeben, sondern in das Aerenchym gedrückt wird und nach der Belastung wieder freigegeben wird.

In einer weiteren Variante der genannten Vorrichtung werden flächige Elemente verwendet, die die oben genannten Oberflächeneigenschaften besitzen und die auch nachträglich, etwa durch Aufkleben, auf die Oberfläche, beispielsweise die Außenseite von Schiffen oder die Innenseite von Rohrleitungen aufgebracht werden können.

Weitere Varianten bzw. Merkmale betreffen eine
- Verwendung einer Luftspeicherschicht in der Form eines dichten Besatzes feiner, dünner Härchen oder Drähtchen in Form eines "Pelzes".
- Verwendung einer Luftspeicherschicht in der Form eines dichten Besatzes feiner, dünner Härchen oder Drähtchen in Form eines Filzes oder Faser-Netzwerkes mit ungeordneten oder bevorzugt ausgerichteten Fasern.
- Verwendung einer wie eben beschriebenen Luftspeicherschicht wobei der Filz oder das Netzwerk aus Textilfasern oder aus Metalldrähten aufgebaut ist.
- Verwendung einer Luftspeicherschicht in der Form eines porösen, mikro- oder nanoporösen Materials.
- Verwendung einer wie eben beschriebenen Luftspeicherschicht wobei das poröse Material ein Polymer oder ein Metall oder ein keramisches Material ist.
- Verwendung einer wie eben beschriebenen Luftspeicherschicht, wobei das poröse Material eine Polymerblend-Schicht ist, aus der eine der Polymerkomponenten - vorzugsweise nach der Schichtbildung mit begleitender Phasenseparation - durch ein selektives Lösungsmittel entfernt wurde auf diese Weise eine poröse Schicht hergestellt wurde, die dann - mit oder ohne zusätzliche Hydrophobierung der Poreninnenflächen und/oder der Schichtoberfläche - als Luftspeicher dient.
- Verwendung eines Precursors für die Herstellung einer metallischen oder keramischen Schicht als eine der beiden Komponenten und Herstellung einer solchen porösen metallischen oder keramischen Schicht durch nachträgliche thermische Behandlung.
- Aufbringung in Form von Fliesen, Kacheln etc. mit den gewünschten Oberflächeneigenschaften
- Aufbringung flexibler Flächenelemente.
- Aufbringung durch reversible oder irreversible Klebung.
- Beschichtung von Schiffsoberflächen - ganz oder teilweise.
- Verwendung von metallischen Oberflächenstrukturen.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung.
- Verwendung als Beschichtung auf Schiffen, in Strömungskanälen, in Rohren oder in chemischen Reaktionsgefäßen.

Kugellager aus Luft- oder Gas-Kügelchen: Reduzierung von Reibung von Oberflächen unter Flüssigkeit durch die Beschichtung oder teilweise Beschichtung mit einer Anordnung aus Gasbläschen, bevorzugt in "Air Pockets", energetisch bevorzugten, gegen Ablösung gepinnten Nischen oder Umklammerungen, die sich direkt auf der Oberfläche befinden können, die aber auch ihrerseits wieder am Ende eines Haares oder einer Stange oder Miniatur-Blattfeder oder anderweitiger Halterung aufgehängt befinden können, sowie die Gestaltung von Dreh- Wälz und Gleitlagern unter Flüssigkeit aus solchen Gaskügelchen, wobei die gepinnten Gaskügelchen die Funktion der Kugeln in konventionellen Kugellagern übernehmen - mit den Vorteilen des Self-Recharging, der Abnutzungsfreiheit und damit der unbegrenzten Lebensdauer bezüglich mechanischem Abrieb, und mit zusätzlichen Vorteilen bezüglich der Freiheit von Abriebpartikeln (müssen in Schwebe gehalten werden, führen zu weiterem mechanischen Verschleiß, können sich ablagern und feste Sedimente bilden, die die Lebensdauer mechanischer beweglicher Komponenten begrenzen können) und der Freiheit von Lubrikationsmitteln (Öle etc., altern, müssen gewechselt werden, sind giftig und Lebensmittel-inkompatibel, müssen entsorgt werden).

Die Entwicklung von Lagern, etwa von Roll-, Wälz- und Gleit-Lagern, mit langer Standzeit, niedriger Abnutzung, geringer Reibung und insbesondere der Vermeidung von Haftreibung beim Anfahren sowie von Lagerschädigung bei langen Standzeiten sind eine technische Herausforderung. Magnetlager sind kostspielig und nicht unter allen Umgebungsbedingungen einsetzbar.

Insbesondere unter Flüssigkeiten, vor allem, wenn es sich auch noch um korrosive Flüssigkeiten (wie etwa Salzwasser) oder chemisch aggressive Flüssigkeiten handelt (Säuren, Laugen, Oxidationsmittel), besteht ein echtes technisches Problem.

Das Problem wird gelöst durch Verwendung von Kugellager, Wälz- und Gleitlagern aus Luft- oder Gas-Kügelchen sowie von Anordnungen von solchen Kügelchen (für Gleitlager beispielsweise von ebenen Flächen, die mit solchen Kügelchen aus Gas unter Flüssigkeit (Gasbläschen) beschichtet sind): Reduzierung von Reibung von Oberflächen unter Flüssigkeit wird erreicht durch die Beschichtung oder teilweise Beschichtung mit einer Anordnung aus Gasbläschen, bevorzugt in "Air Pockets", energetisch bevorzugten, gegen Ablösung gepinnten Nischen oder Umklammerungen, die sich direkt auf der Oberfläche befinden können, die aber auch ihrerseits wieder am Ende eines Haares oder einer Stange oder Miniatur-Blattfeder oder anderweitiger Halterung aufgehängt befinden können, sowie die Gestaltung von Dreh- Wälz und Gleitlagern unter Flüssigkeit aus solchen Gaskügelchen, wobei die gepinnten Gaskügelchen die Funktion der Kugeln in konventionellen Kugellagern übernehmen - mit den Vorteilen des Self-Recharging, der Abnutzungsfreiheit und damit der quasi-unbegrenzten Lebensdauer bezüglich mechanischem Abrieb, und mit zusätzlichen Vorteilen bezüglich der Freiheit von Abriebpartikeln (müssen in Schwebe gehalten werden, führen zu weiterem mechanischen Verschleiß, können sich ablagern und feste Sedimente bilden, die die Lebensdauer mechanischer beweglicher Komponenten begrenzen können) und der Freiheit von Lubrikationsmitteln (Öle etc. altern, müssen gewechselt werden, sind giftig und Lebensmittel-inkompatibel, müssen entsorgt werden).

Weitere Varianten bzw. Merkmale betreffen eine
- Verwendung einer persistenten Gasschicht oder von Compartments aus Gasschichten, die auf der Oberfläche gehalten werden, anstelle der einzelnen Gaskügelchen (Gasbläschen).
- Verwendung von ortsfesten, gepinnten Gasbläschen.
- Verwendung nicht ortsfester oder nicht gepinnter Gasbläschen, die sich in einer definierten Umgebung bewegen können, ähnlich wie die Kugeln der "normalen" Kugellager in und ggf. mit ihrem Kugelkäfig beweglich sind.
- Kombination mit Möglichkeiten zur Aufladung der Gasschicht bzw. der Gaskügelchen ("Replenishing") bei Gasverlust über zusätzliche Vorrichtungen zum künstlichen Wiederbefüllen oder Nachfüllen der Gasschicht, etwa Kapillaren, Membranen, Textilien etc.,
- Vorrichtung wie eben beschrieben, ggf. auch gekoppelt mit Mess- und/oder Steuerungs- und/oder Regeleinrichtungen, die den Zustand der Gasschicht insgesamt oder ortsaufgelöst messen und kontrollieren und im Bedarfsfall automatisch eine Nachfüllung veranlassen
- Aufbringung in Form von Fliesen, Kacheln etc. mit den gewünschten Oberflächeneigenschaften
- Aufbringung flexibler Flächenelemente.
- Aufbringung durch reversible oder irreversible Klebung.
- Beschichtung von Schiffsoberflächen - ganz oder teilweise.
- Verwendung von metallischen Oberflächenstrukturen.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung.
- Verwendung als Beschichtung auf Schiffen, in Strömungskanälen, in Rohren oder in chemischen Reaktionsgefäßen.

Chemische Reaktionsgefäße, Rohre und Leitungen mit Wänden aus Luft: Chemische Reaktionsgefäße stabiler Form für Reaktionen von Flüssigkeiten oder von Flüssigkeiten mit inkludierten Feststoffkomponenten und -Partikeln, dadurch gekennzeichnet, dass die - in einer bevorzugten Ausführungsvariante formstabilen und formdefinierten - Reaktionsgefäßwände aus einer Luft- oder Gasschicht bestehen, hervorgerufen durch eine feste Gefäßwand, beschichtet mit Strukturen zur Gashaltung unter Flüssigkeit,
sowie die Nutzung solcher Vorrichtungen für die Durchführung chemischer Reaktionen und physikalischer und chemischer Prozesse, in einer Variante des Verfahrens unter zusätzlicher Nutzung der Gasschicht zur Zufuhr von Reaktions-Edukten und zur Abfuhr von Reaktionsprodukten,
in einer weiteren Variante des Verfahrens zur Nutzung der Gasschicht zum Reinigen und Spülen mit Gas, zur Kontrolle des Druckes im Reaktionsgefäß, zur Abfuhr oder Zufuhr von Wärmeenergie oder zu einer Kombination der genannten Dinge.

Das Bereitstellen geeigneter Reaktionsgefäße für Reaktionen unter extremen Bedingungen, bei hohen Temperaturen oder mit chemisch reaktiven flüssigen Medien stellt hohe Anforderungen an die Gefäßwände.

Ein weiteres Problem ist, dass die Gefäßwände nach dem in-Kontakt-kommen mit einer Flüssigkeit oft aufwändig gereinigt werden müssen, bevor man eine andere Flüssigkeit in den Behälter geben oder durch die Leitung schicken kann. Dies gilt insbesondere für den Bereich der Lebensmittelbehältnisse. Nach dem Transport toxischer Flüssigkeiten in einem Behältnis oder einer Rohrleitung muss diese sorgfältig gereinigt werden, bevor flüssige Lebensmittel wie Getränke, Milch etc. eingefüllt werden dürfen.

Nach dem erfindungsgemäßen Verfahren werden die genannten Probleme gelöst, indem die Gefäßwände nicht mit der Flüssigkeit in Berührung kommen und damit auch keine Flüssigkeitsreste an den Gefäßwänden verbleiben können, aber auch keine chemischen Reaktionen der Flüssigkeit mit den Gefäßwänden zustande kommen kann, indem zwischen die Flüssigkeit und die Gefäßwand eine persistente Gasschicht eingefügt wird. Mann verwendet quasi "Chemische Reaktionsgefäße, Rohre und Leitungen mit Wänden aus Luft": Es kommen chemische Reaktionsgefäße stabiler Form für Reaktionen von Flüssigkeiten oder von Flüssigkeiten mit inkludierten Feststoffkomponenten und -Partikeln zum Einsatz, dadurch gekennzeichnet, dass die - in einer bevorzugten Ausführungsvariante formstabilen und formdefinierten - Reaktionsgefäßwände aus einer Luft- oder Gasschicht bestehen, hervorgerufen durch eine feste Gefäßwand, beschichtet mit Strukturen zur Gashaltung unter Flüssigkeit.

Ferner können solche Vorrichtungen für die Durchführung chemischer Reaktionen und physikalischer und chemischer Prozesse genützt werden, in einer Variante des Verfahrens unter zusätzlicher Nutzung der Gasschicht zur Zufuhr von Reaktions-Edukten und zur Abfuhr von Reaktionsprodukten.

In einer weiteren Variante des Verfahrens geht es um die zur Nutzung der Gasschicht zum Reinigen und Spülen mit Gas, zur Kontrolle des Druckes im Reaktionsgefäß, zur Abfuhr oder Zufuhr von Wärmeenergie oder zu einer Kombination der genannten Dinge.

Weitere Varianten bzw. Merkmale betreffen eine
- Verwendung von einer persistenten Gasschicht oder von Compartments aus Gasschichten, die auf der Oberfläche gehalten werden, anstelle der einzelnen Gaskügelchen (Gasbläschen).
- Verwendung von ortsfesten, gepinnten Gasbläschen.
- Verwendung nicht ortsfester oder nicht gepinnter Gasbläschen, die sich in einer definierten Umgebung bewegen können, ähnlich wie die Kugeln der "normalen" Kugellager in und ggf. mit ihrem Kugelkäfig beweglich sind.
- Kombination mit Möglichkeiten zur Aufladung der Gasschicht bzw. der Gaskügelchen ("Replenishing") bei Gasverlust über zusätzliche Vorrichtungen zum künstlichen Wiederbefüllen oder Nachfüllen der Gasschicht, etwa Kapillaren, Membranen, Textilien etc.,
- Vorrichtung wie eben beschrieben, ggf. auch gekoppelt mit Mess- und/oder Steuerungs- und/oder Regeleinrichtungen, die den Zustand der Gasschicht insgesamt oder ortsaufgelöst messen und kontrollieren und im Bedarfsfall automatisch eine Nachfüllung veranlassen
- Aufbringung in Form von Fliesen, Kacheln etc. mit den gewünschten Oberflächeneigenschaften
- Aufbringung flexibler Flächenelemente.
- Aufbringung durch reversible oder irreversible Klebung.
- Beschichtung von Innen- und Außen-Oberflächen - ganz oder teilweise.
- Verwendung von metallischen Oberflächenstrukturen.
- Verwendung von Oberflächen mit durchgehender Luftschicht.
- Verwendung von Oberflächen, die nur ein regelmäßiges oder unregelmäßiges Muster von Gastaschen oder Gasbläschen unter Flüssigkeit ausbilden, aufbauen oder halten.
- Verwendung dieser Patterns aus Gastaschen oder Gasbläschen unter Flüssigkeit als eine Art "Kugellager mit Kugeln aus Gas bzw. Luft - zur mechanischen Führung und / oder zur Reibungsreduzierung.
- Verwendung als Beschichtung auf Schiffen, in Strömungskanälen, in Rohren oder in chemischen Reaktionsgefäßen.

Unter Wasser Luft haltende strukturierte Oberflächen mit Lufthaltung in Form von "Air Layers", "Air Compartments" and "Air Pockets": Durchgehende Luftschichten, Luft-Compartments begrenzter flächiger Ausdehnung und lokale Lufttaschen und - Bläschen sowie der Variante der unregelmäßigen Strukturierung mit kontinuierlichem Übergang zwischen diesen Fällen.

Gasschichten auf Oberflächen unter Flüssigkeit sind von großem technologischem Interesse zur Reibungsreduktion bei Schiffen und in Pipelines sowie zur Erzielung von Schutz der Oberfläche vor Belagbildung, (Bio)Fouling, Korrosion und chemischem Angriff.

Durch eine geeignete Oberflächenstrukturierung gelingt es, eine an der Oberfläche anhaftende Schicht aus Gas oder Gasbläschen mit unter Flüssigkeit zu nehmen. Das Problem ist, geeignete Oberflächenstrukturen zu finden, die auch bei Druckfluktuationen die durchgehende oder nicht durchgehende Gasschicht unter Flüssigkeit permanent oder zumindest für gewisse Zeiträume halten.

Diese Probleme werden gelöst durch eine hydrophobe oder superhydrophobe Oberfläche, die topographisch so strukturiert ist, dass die Gasschicht oder Gasbläschen gehalten und in einer bevorzugten Variante zusätzlich durch hydrophile Pins am Entweichen gehindert werden und / oder für die Gasbläschen gewisse topographisch vorgeformte Bereiche, "Air Pockets", in der Oberflächenstruktur geschaffen werden, in der eine stabile Einlagerung von kleinen Gasvolumina unter Flüssigkeit stabil ermöglichen. Dabei kann man auch auf der Millimeterskala oder Mikrometerskala oder Nanometerskala raue Oberflächen verwenden oder mehrere dieser Längenskalen kombinieren, um auf diese Weise mit der Nanorauigkeit Superhydrophobie zu erzielen und mit der Rauigkeit im Milli- und Mikrometerbereich die Air Pockets zu schaffen, bevorzugt mit typischen Abmessungen im Bereich zwischen 10 µm und 5 mm, besonders bevorzugt zwischen 0,1 mm und 3 mm. Diese können dann in einer bevorzugten Variante ebenfalls hydrophile Pinningzentren auf ihrer ansonsten hydrophoben oder superhydrophoben Oberfläche aufweisen, um die Luft-Wasser-Grenzfläche festzuhalten und das Entweichen von Gasbläschen auf diese Weise zu verhindern.

Das Pinning kann dabei auch auf mehreren Strukturierungsebenen erfolgen (Hierarchical Pinning oder Two-Level oder Multi-Level Pinning Effect) oder auf unregelmäßig strukturierten Oberflächen auch mit einer kontinuierlichen Hierarchie (was unendlich vielen Hierarchieebenen entspricht) erfolgen. Dabei können sich beim Pinning in den oberen Hierarchieebenen ausgedehnte, mit Luft beschichtete Bereiche ausbilden, oberhalb einer sog. Perkolationsschwelle auch durchgehende, in sich zusammenhängende Luftschichten, und erst, wenn Luft irgendwo entweicht und das Wasser die tiefergelegenen Hierarchieebenen erreicht, ergeben sich räumlich voneinander getrennte Luftvolumina an der Oberfläche ("Compartmentstruktur"), bis bei noch weiterem Eindringen des Wassers etwa aufgrund von sehr hohen Druckschwankungen noch mehr Luft verlorengeht und schließlich nur noch die Air Pockets als letzte Luftreserve übrigbleiben. Diese Air Pockets haben dann eine dreifache Funktion:
- Sie dienen als letztes, nur schwer entfernbares Luftreservoir, als "Notreserve an Luft".
- Sie schützen dennoch einen wesentlichen Teil, der typischerweise 60% bis 98% der gesamten Oberfläche beträgt, vor Oxidation, chemischem Angriff oder (Bio)Fouling und wirken reibungsreduzierend, und vor allem:
- Sie wirken als Keimzentren für den Wiederaufbau der Luftschicht, sowohl bei aktivem Wiederbefüllen der Gasschicht ("Replenishing") als auch beim Selbstregenerationsprozess der Luftschicht.

Dieses Ziel der Strukturierung auf mehreren Ebenen bzw. Längen- und ggf. auch Höhenskalen sowie - in einer bevorzugten Variante - zusätzlich des hierarchischen Pinnings zeigt im einfachsten Falle die erwähnten drei Stufen der Lufthaltung:
1. Durchgehende Luftschicht, getragen und gestützt nur von einzelnen Stützstrukturen (Haare, Säulen, Erhebungen), die hydrophob oder superhydrophob mit oder ohne hydrophile Pinningzentren (letztere, sofern vorhanden, bevorzugt am oberen Ende dieser Stützstrukturen) sein können. Die Reibung zwischen Flüssigkeit und Festkörper wird dadurch massiv vermindert, z.T. bis unterhalb von 5% des Wertes ohne Luftschicht. Die Anfälligkeit für Luftverlust bei Druckschwankungen ist in diesem (für alle anderen technischen Eigenschaften nahezu idealen Zustand) allerdings hoch. Die Aktivierungsbarrieren bezüglich Luftverlust - z.B. durch Entweichen von Gasbläschen - hinsichtlich der benötigten Kraft pro Fläche oder hinsichtlich der Energie pro Fläche sind relativ niedrig.
2. Bei erfolgtem Luftverlust geht die Stufe 1 (Zustand wie eben beschrieben) in die Stufe 2 über: Es bilden sich größere, zusammenhängende, mit Luft bedeckte Bereiche der Oberfläche unter Flüssigkeit aus, jedoch ist die Perkolationsschwelle für durchgängige Luftschichten unterschritten. Die einzelnen mit Luft bedeckten Oberflächenbereiche sind durch Trennwände bzw. durch nicht mit Luft beschichtete Oberflächenbereiche begrenzt, die mit Wasser in Berührung sind und in der Regel einen Gasaustausch zwischen den einzelnen Luftinseln oder Compartments blockieren. Die Reibungsreduktion ist in diesem Zustand immer noch bedeutsam, jedoch im Vergleich zu Zustand 1 erheblich reduziert. Der Schutz der Oberflächen vor Kontakt mit dem Wasser bzw. der Flüssigkeit ist noch weitgehend vollständig oder zumindest größtenteils erhalten (typischerweise sind nur max. 2% bis 10% der gesamten Oberfläche mit dem Wasser in Berührung). Die Aktivierungsschwelle, die benötigt wird, um der Schicht Luft bzw. allg. Gas zu entreißen, ist signifikant höher als in Stufe 1.
3. Bei noch weiterem Gasverlust, z.B. durch sehr hohe Druckschwankungen, verbleibt schließlich Stufe 3, in der die Oberfläche nur noch kleine Luftvolumina in hydrophoben Nischen, den sog. Air Pockets, speichert. Auch diese können auf ihrer dem Wasser zugewandten Seite hydrophile Pinningzentren aufweisen. Die reibungsvermindernde Wirkung der Luftschicht ist in diesem Zustand im Vergleich zu Stufe 1 wesentlich abgeschwächt, im Extremfall nicht mehr vorhanden. Dennoch wird auch in Stufe 3 je nach topographischer Ausgestaltung eine erhebliche Verminderung der Kontaktfläche der Festkörperoberfläche des eingetauchten Festkörpers mit dem Wasser erzielt, meist um mehr als einen Faktor 10, d.h. nur weniger als 10 % der Oberfläche ist in direktem Kontakt mit dem Wasser bzw. allg. der Flüssigkeit im Vergleich zu dem Zustand ohne gasgefüllte "Air Pockets".

Ganz allgemein kann die Luft natürlich durch ein beliebiges Gas ersetzt werden. Auch kann an die Stelle des Wassers eine beliebige andere Flüssigkeit treten. "Hydrophob oder superhydrophob" ist dann durch "bezüglich dieser Flüssigkeit nicht benetzend oder super-nichtbenetzend" (definiert ganz analog zu hydrophob und superhydrophob durch die entsprechenden Kontaktwinkel) zu ersetzen, und die hydrophilen Pins sind durch Pins zu ersetzen, die bezüglich dieser Flüssigkeit "liquidophil", also benetzend, sind. Dabei genügen relative Unterschiede: Die "hydrophilen Pins" müssen nicht zwangsläufig wirklich hydrophil im Sinne der Lehrbuchdefinition sein, es genügt in manchen Fällen, wenn sie einfach benetzender sind als die übrige hydrophobe (oder allg. für beliebige Flüssigkeiten: "liquidophobe") Oberfläche.

Die drei Hierarchieebenen der Strukturierung können in Varianten der Ausgestaltung auch entweder nicht vorhanden sein (es gibt nur eine Ebene) oder es kann zwei Ebenen geben oder alle drei, oder es kann sich statt einer regelmäßig oder quasiregelmäßig strukturierten Oberfläche um eine zufallsstrukturierte Oberfläche mit einer bestimmten Rauigkeit handeln, die an verschiedenen Stellen kleinere oder größere Pinningzentren hat und deren natürliche Vertiefungen als Air Pockets dienen. Gleichermaßen geeignet wie Oberflächen bestimmter Rauigkeit sind natürlich poröse Oberflächen, bei denen die an der Oberfläche liegenden Poren als Air Pockets dienen können.

Eine besondere Variante einer solchen Oberfläche sind Fasern oder Textilfasern, die aufgrund einer wie oben beschriebenen gestalteten Oberfläche eine durchgehende oder nicht durchgehende Schicht aus Luft unter Wasser halten können. Alles ist wie eben beschrieben, nur dass die beschriebene strukturierte Oberfläche jetzt die Oberfläche einer Faser ist. Auch diese kann wieder - wie auch die schon zuvor beschriebenen Oberflächen -
- durch regelmäßige oder unregelmäßige topographische Strukturierung oder
- durch regelmäßige oder unregelmäßige chemische Strukturierung (d.h. eine ortsabhängige chemische oder biochemische Oberflächenfunktionalisierung) oder
- durch Oberflächenrauigkeit oder
- durch Oberflächenporosität
oder durch eine Kombination der genannten Arten strukturiert sein und hydrophile Pinningzentren aufweisen oder auch nicht. Interessant an den an ihrer Oberfläche unter Flüssigkeit Gas haltenden Fasern ist, dass man aus ihnen unter Flüssigkeit an ihrer Oberfläche Gas haltende Textilien, Gewebe, Geflechte, Matten, Stränge, Seile, Filze, Badekleidung (Badehose, Badeanzug etc.) etc. aufbauen bzw. herstellen kann. Ebenso ist es denkbar, Schiffe, Boote, Wassersport-Geräte (inkl. Surfbrett etc.), Bojen, Bohrinseln, Meßstationen, Messgeräte, dem Wasser ausgesetzte Komponenten von Offshore-Windparks, andere Wasserbauwerke etc. mit derartigen Luft haltenden Textilien zu beschichten, beispielsweise um Reibungsreduktion, Antifouling-Wirkung oder Korrosionsschutz zu erreichen.

Anwendungen des Salvinia-Effektes:
- Kontaktlose Gefäße für hochreaktive Flüssigkeiten.
- Flüssigkeiten, von einem Gaspolster eines Reaktionspartners umgeben, der das Gas oder ein Bestandteil des Gases oder in Form von feinen Tröpfchen oder von feinen Partikeln zugeführt werden kann.
- Anwendung des Luftpolsters zur thermischen Isolierung.
- Anwendung des Luftpolsters zur Reibungsreduzierung.
- Anwendung des Luftpolsters zur Erzielung eines Antifouling-Effektes.
- Anwendung des Luftpolsters zur Vermeidung von Biofilmbildung.
- Der Befall der Härchen und der Substratoberfläche selbst könnte durch Beschichtung mit Bakteriziden, Fungiziden, Nanosilber, Nanokupfer oder silber- und kupfer-haltigen Komponenten erzielt werden.
- Anwendung des Luftpolsters zur elektrischen Isolation und galvanischen Trennung, insbesondere bei Elektrolyten.

Nachhaltige Erzielung des Salvinia-Effektes durch nachladbare Luftschichten:
- Nachladung der Luftschicht über Pumpen oder Druckgasflaschen mittels feiner Düsen oder durch eine gas-permeable Oberfläche zwischen den einzelnen Härchen.
- Nachladung der Luftschicht durch galvanische, katalytische, photokatalytische oder galvanokatalytische Zersetzung des flüssigen Mediums (z.B. Wasser).
- Nachladung der Luftschicht durch gelöste Gaskomponenten in der Flüssigkeit durch so starke Erhöhung der Oberflächenenergie des eindringenden Wassers, dass dieses fernbleibt und somit die Summe der Partialdrücke aller Gase und Flüssigkeiten im Flüssigen Medium gleich dem Gasdruck in der Salvinia-Gasschicht ist - was bedeuten kann, dass dies weniger als der hydrostatische Druck in der Flüssigkeit ist,
- Sowie gezielte Nutzung dieses Effektes, um die Aktivierungsenergie für die Bildung von Gasblasen zu erhöhen: Das flüssige Medium wird durch den Unterdruck in der Gasschicht noch an die Oberfläche der Blatthaare "angesogen".

Erzeugung von Luftschichten mit und ohne Salvinia-Effekt:
Anwendung unter anderem für:
- Lufthaltung.
- Reibungsreduzierung.
- Auftrieb.
- Antifouling.
- Korrosionsschutz.
- Adhäsionsschutz - Herstellung antiadhäsiver Oberflächen durch "Beschichtung mit Luft" in der beschriebenen Weise.
- In Kombination mit einem Auto-Reloading oder einem aktiven Reloading der Luftschicht könnte hier ein sehr effizientes System zur Reibungsreduzierung aufgebaut werden.
- Weitere Reibungsreduzierung würde durch apolare Teflon-Gleiter am Ende der Haare bewirkt werden.
- Durch Abflachen des Neigungswinkels der oberen Enden der Haare wird die Ableitung der Benetzungsenergie nach der Eindringtiefe das Wassers in die Haarschicht und damit die Repelling Force, mit der die Flüssigkeit beim Eindringen zurückgewiesen wird, massiv erhöht.
- Die Elastizität der angewinkelten Haare soll ferner durch eine geeignete Wahl der Federkonstanten dafür sorgen, dass sich eher das Haar biegt als dass Flüssigkeit das Haar benetzt.

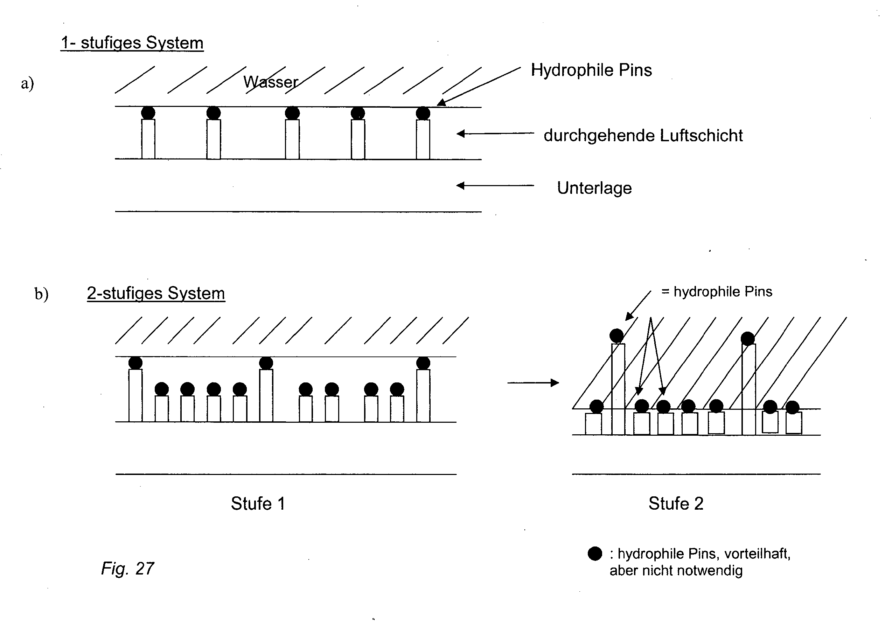

Die Figur 27a zeigt eine gashaltende Schicht mit einem einstufigen System von Vorsprüngen, wobei alle Vorsprünge im Wesentlichen die gleiche Längserstreckung aufweisen.

Die Figur 27b zeigt eine gashaltende Schicht mit einem zweistufigen System von Vorsprüngen, wobei sich Vorsprünge in kurze und lange Vorsprünge aufteilen.

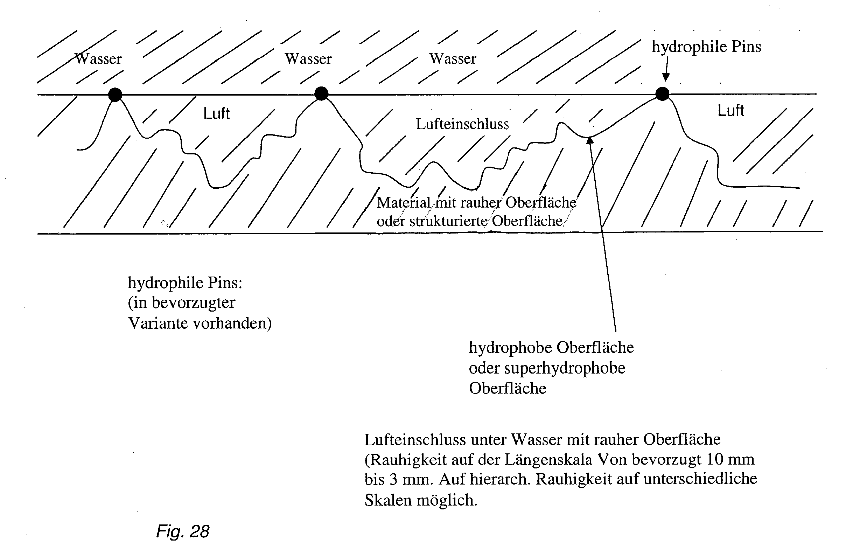

Die Figur 28 zeigt eine gashaltende Schicht, welche durch eine raue Oberfläche ausgebildet ist, wobei die Rauigkeit auf Oberfläche eine Längenskala von etwa 10 µm bis etwa 3 mm aufweisen kann.

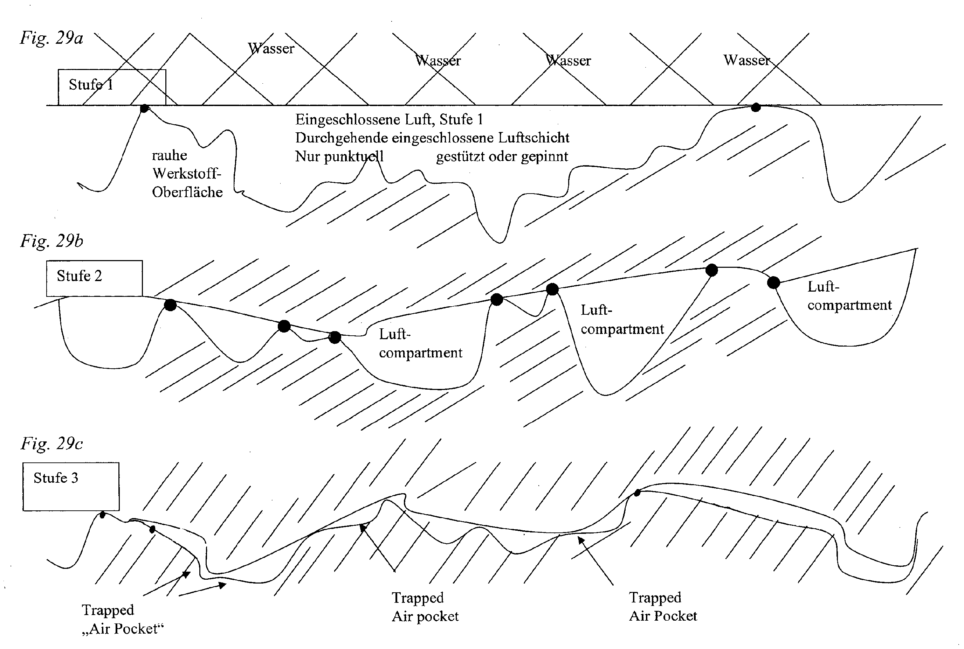

Die Figur 29a zeigt eine gashaltende Schicht mit einer rauen Oberflächen, welche als einstufiges System ausgebildet ist. Die Figur 29b zeigt eine gashaltende Schicht mit einer rauen Oberflächen, welche als zweistufiges System ausgebildet ist. Die Figur 29c zeigt eine gashaltende Schicht mit einer rauen Oberflächen, welche als dreistufiges System ausgebildet ist.

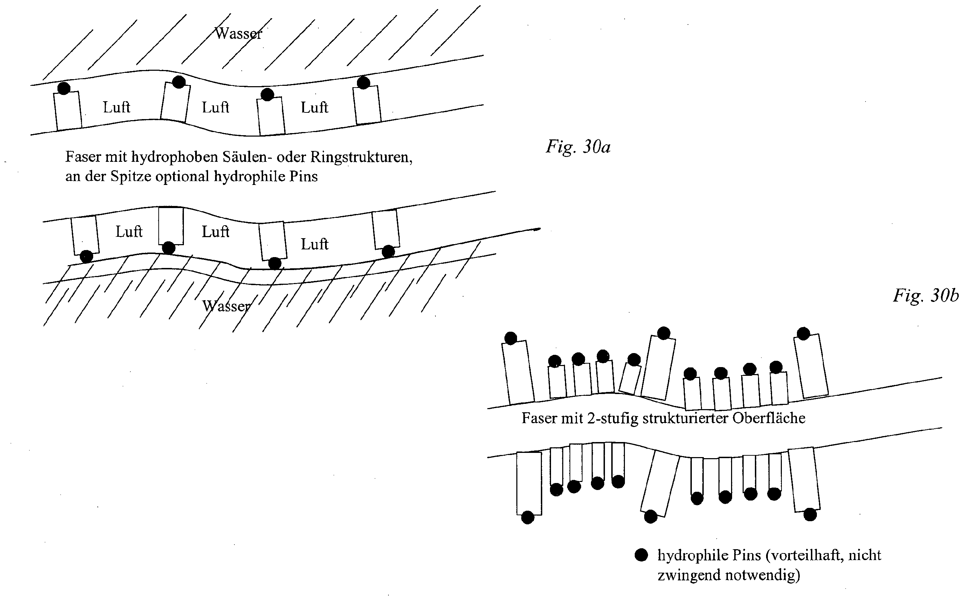

Die Figur 30a zeigt eine gashaltende Schicht, welche an einem fadenförmigen Element ausgebildet ist. Die Figur 30b zeigt eine fadenförmiges bzw. faserförmiges Element mit einer gashaltenden Schicht, welche als zweistufiges System ausgebildet ist.

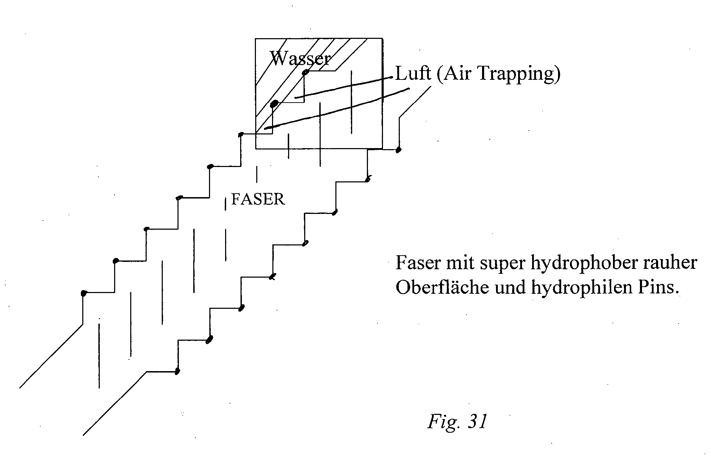

Die Figur 31 zeigt eine gashaltende Schicht, welche an einem fadenförmigen Element ausgebildet ist, und eine superhydrophobe Oberfläche ausweist, an welcher hydrophile Stellen bzw. Pins ausgebildet sind.

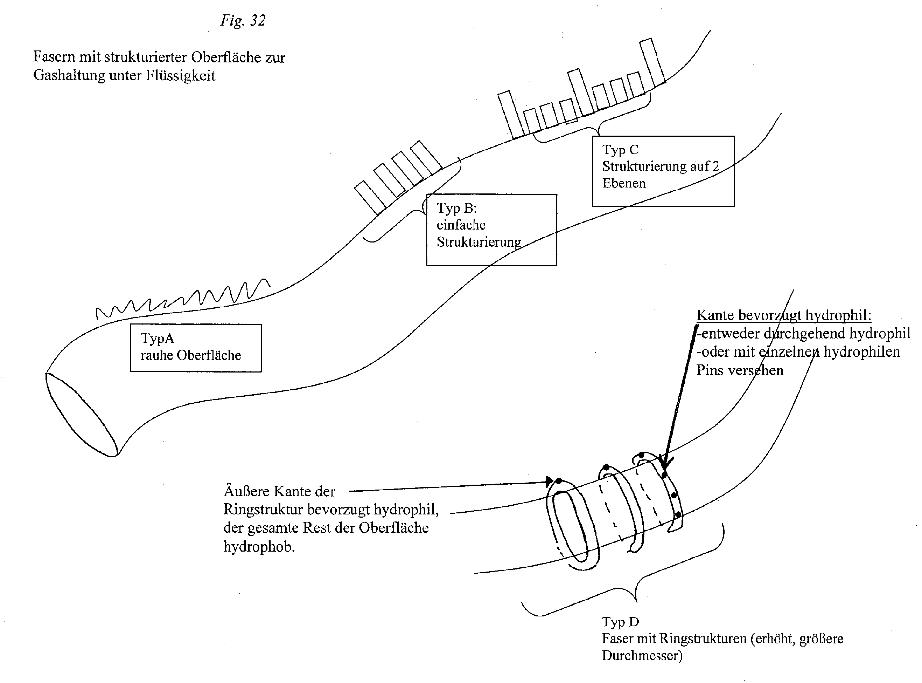

Die Figur 32 zeigt eine gashaltende Schicht, welche an einem fadenförmigen Element ausgebildet ist, wobei verschiedene Oberflächengestaltungen gezeigt sind.

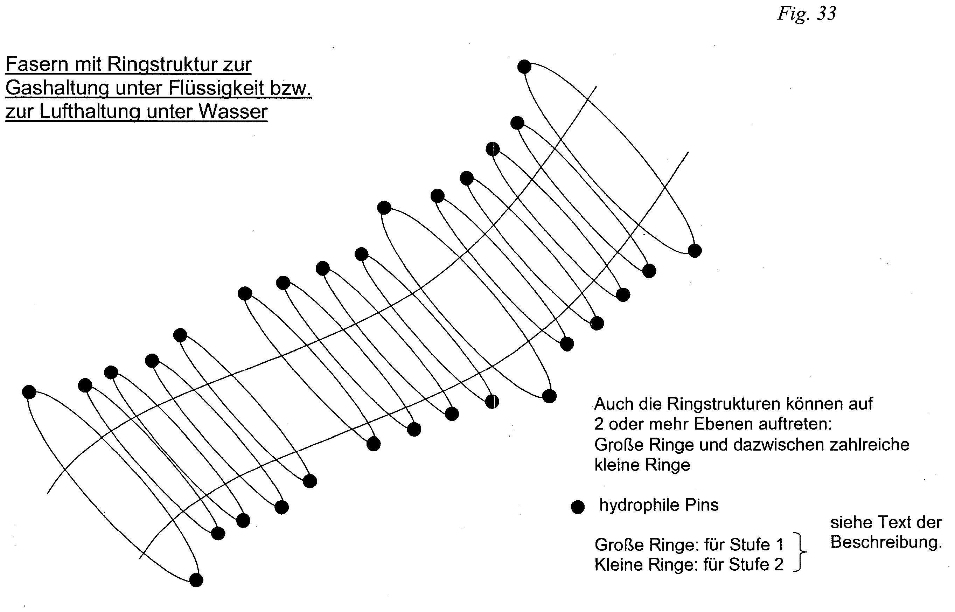

Die Figur 33 zeigt eine gashaltende Schicht, welche an einem fadenförmigen Element ausgebildet ist, wobei die gashaltende Schicht eine ringförmige Struktur aufweist.

### Bezugszeichenliste

- 2: Wandung
- 4: Flüssigkeit
- 5: Gas
- 6: Oberflächenabdeckung
- 10: gashaltende Schicht
- 10c: Basis der gashaltenden Schicht 10
- 12: gasdurchlässige Lage
- 14: Gaszufuhreinrichtung
- 16: Gaskanal
- 18: Gasquelle
- 20: Ventil
- 22: Regeleinrichtung
- 24: Sensoreinrichtung
- 26: vorstehendes Element
- 27: vorstehendes Element
- 28: Gasaustrittseinrichtung
- 30: Mulde
- 32: Öffnung der Mulde 30
- 34: hydrophobe Beschichtung der Mulde 30
- 36: Oberflächenbeschichtung
- 38: raue Oberfläche
- 40: Faser
- 42: Trennwand
- 44: Teilbereich der gashaltenden Schicht 10
- A: Gasaustrittsrichtung
- L: Längsrichtung

## Patentansprüche

1. Verwendung einer gashaltenden Schicht (10), welche an einem in eine Flüssigkeit eintauchbaren oder mit einer Flüssigkeit benetzbaren Körper (2) angeordnet ist und welche mit einer flüssigkeitszugewandten Seite (10a) bei Eintauchen/Benetzen des Körpers (2) mit der Flüssigkeit (4) in Kontakt ist, wobei der Körper (2) eine Wandung eines Wasserfahrzeugs oder eines im Wasser angeordneten Bauwerks oder eine Innenwandung eines Flüssigkeitsbehälters oder einer Flüssigkeitsleitung ist, wobei die gashaltende Schicht (10) auf der flüssigkeitszugewandten Seite (10a) Ausnehmungen (30) und/oder vorstehende Elemente (26, 27) aufweist, wobei die Oberflächen der Ausnehmungen (30) zumindest bereichsweise hydrophob sind und die Oberfläche der vorstehenden Elemente (26, 27) im Wesentlichen hydrophob ist,
wobei eine Gasschicht (5) mit einer Dicke von 10 nm bis 10 mm, die in dem eingetauchten/benetzten Bereich der gashaltenden Schicht (10) gehalten wird, die Flüssigkeit (4) und den eingetauchten/benetzten Bereich des Körpers (2) zumindest bereichsweise voneinander trennt, wobei die Flüssigkeit (4) ein polares Lösungsmittel ist, und
wobei die gashaltende Schicht (10) durch fluidundurchlässige Trennwände (42) in eine Vielzahl von Teilbereichen (44) unterteilt ist und die Trennwände (42) zumindest bereichsweise hydrophil ausgebildet sind.

2. Verwendung nach Anspruch 1, wobei zwischen der gashaltenden Schicht (10) und der Wandung des Körpers (2) eine Korrosionsschutzschicht und/oder eine Bewuchsschutz- bzw. Antifoulingschicht angeordnet ist und wobei die gashaltende Schicht (10) zumindest bereichsweise die Korrosionsschutzschicht und/oder die Bewuchsschutz- bzw. Antifoulingschicht von der Flüssigkeit trennt.

3. Verwendung der gashaltenden Schicht (10) nach Anspruch 1 oder 2, wobei die fluidundurchlässigen Trennwände (42), welche gleichlang sind, derart angeordnet sind, dass sechseckige Teilbereiche (44) ausgebildet werden, oder wobei die fluidundurchlässigen Trennwände (42) derart angeordnet sind, dass langgestreckte, wabenförmige oder sechseckige Teilbereiche (44) ausgebildet werden.

4. Verwendung der gashaltenden Schicht (10) nach einem der vorigen Ansprüche, wobei in jedem der Teilbereiche (44) vorstehende Elemente (26, 27) in einer zweidimensionalen Anordnung vorhanden sind.

5. Verwendung der gashaltenden Schicht (10) nach einem der vorigen Ansprüche, wobei die vorstehenden Elemente (26, 27) einen zentralen Oberflächenbereich (26e, 27e) aufweisen, der hydrophil ist und der von einem hydrophoben Oberflächenbereich der vorstehenden Elemente (26, 27) umgeben ist.

6. Verwendung der gashaltenden Schicht (10) nach einem der vorigen Ansprüche, wobei die gashaltende Schicht (10) von einer der flüssigkeitszugewandten Seite (10a) gegenüberliegenden körperzugewandten Seite (10b) der gashaltenden Schicht (10) her mit Gas beaufschlagt wird.

7. Verwendung der gashaltenden Schicht (10) nach einem der vorigen Ansprüche, wobei eine gasdurchlässige Schicht (12) an der körperzugewandten Seite (10b) der gashaltenden Schicht (10) angeordnet ist.

8. Verwendung der gashaltenden Schicht (10) nach Anspruch 7, wobei die gasdurchlässige Schicht (12) als flüssigkeitsundurchlässige und/oder hydrophobe Lage ausgebildet ist.

9. Verwendung der gashaltenden Schicht (10) nach Anspruch 7 oder 8, wobei eine Gaszufuhreinrichtung (14) mit der gasdurchlässigen Schicht (12) derart verbunden ist, dass Gas (5) von einer Gaszufuhreinrichtung (14) durch die gasdurchlässige Schicht (12) zur gashaltenden Schicht (10) strömen kann.

10. Verwendung der gashaltenden Schicht (10) nach einem der Ansprüche 7 bis 9, wobei die gasdurchlässige Schicht (12) als poröse, semipermeable Membran ausgebildet ist.

11. Verwendung der gashaltenden Schicht (10) nach Anspruch 9 oder 10, wobei die Gaszufuhreinrichtung (14) als gasdurchlässige Schicht ausgebildet ist, welche an der körperzugewandten Seite der gasdurchlässigen Schicht (12) angeordnet ist.

12. Verwendung der gashaltenden Schicht (10) nach einem der Ansprüche 1 bis 5, wobei die gashaltende Schicht (10) von der flüssigkeitszugewandten Seite (10a) der gashaltenden Schicht (10) her mit Gas beaufschlagt wird.

13. Verwendung der gashaltenden Schicht (10) nach Anspruch 12, wobei zumindest eine Gasaustrittseinrichtung (28) vorgesehen ist, welche eine Gasaustrittsöffnung an der flüssigkeitszugewandten Seite (10a) der gashaltenden Schicht (10) aufweist; und
wobei eine Gaszufuhreinrichtung (14) vorgesehen ist, welche mit der Gasaustrittseinrichtung (28) verbunden ist, wobei von der Gaszufuhreinrichtung (14) bereitgestelltes Gas aus der Gasaustrittseinrichtung (28) strömt und von der gashaltenden Schicht zumindest partiell aufgenommen wird.

14. Verwendung der gashaltenden Schicht (10) nach Anspruch 12, wobei die Gasaustrittseinrichtung (28) die gashaltende Schicht (10) durchstößt.

15. Verwendung der gashaltenden Schicht (10) nach einem der Ansprüche 12 bis 14, wobei eine gasdurchlässige Schicht (12) an der körperzugewandten Seite (10b) der gashaltenden Schicht (10) angeordnet ist.

16. Verwendung der gashaltenden Schicht (10) nach Anspruch 15, wobei die gasdurchlässige Schicht (12) als flüssigkeitsundurchlässige und/oder hydrophobe Lage ausgebildet ist.

17. Verwendung der gashaltenden Schicht (10) nach Anspruch 15 oder 16, wobei die gasdurchlässige Schicht (12) als poröse, semipermeable Membran ausgebildet ist.

18. Verwendung der gashaltenden Schicht (10) nach einem der vorigen Ansprüche, wobei die Oberflächenstruktur der gashaltenden Schicht (10) ein Material, ausgewählt aus Polymeren, Lacken, Keramiken, Harzen, PDMS, Gummi, Silikonkautschuk und Metallen, umfaßt.

19. Verwendung der gashaltenden Schicht (10) nach Anspruch 18, wobei die gashaltende Schicht aus einem geprägten Kunstharz oder Lack besteht.

20. Verwendung der gashaltenden Schicht (10) nach einem der vorigen Ansprüche, wobei die Flüssigkeit (4) Wasser (4') ist und der Körper (2) ein Wasserfahrzeug (2') ist, dessen Wandung in einer Betriebsposition des Wasserfahrzeugs (2') zumindest bereichsweise in das Wasser (4') eingetaucht ist.

21. Verwendung der gashaltenden Schicht (10) nach Anspruch 20, wobei die gashaltende Schicht (10) zumindest bereichsweise derart mit einem Gas beaufschlagt wird, um den Strömungswiderstand zwischen dem Wasser (4') und dem Wasserfahrzeug (2') zu vermindern.

22. Verwendung der gashaltenden Schicht (10) nach Anspruch 20 oder 21, wobei die gashaltende Schicht (10) mit einem bewuchshemmenden Gas beschickt wird.

23. Verwendung der gashaltenden Schicht (10) nach einem der vorigen Ansprüche, wobei die Gasschicht (5) in Form eines Arrays kleiner Gasblasen an vordefinierten Stellen auf der Oberfläche der gashaltenden Schicht (10) ausbildet ist.

24. Verwendung einer gashaltenden Schicht (10), welche an einem in eine Flüssigkeit eintauchbaren oder mit einer Flüssigkeit benetzbaren Körper (2) angeordnet ist und welche mit einer flüssigkeitszugewandten Seite (10a) bei Eintauchen/Benetzen des Körpers (2) mit der Flüssigkeit in Kontakt ist, wobei der Körper (2) eine Faser ist, wobei die gashaltende Schicht (10) auf der flüssigkeitszugewandten Seite (10a) vorstehende Elemente (26, 27) aufweist, deren Oberfläche im Wesentlichen hydrophob ist,
wobei eine Gasschicht (5) mit einer Dicke von 10 nm bis 10 mm, die in dem eingetauchten/benetzten Bereich der gashaltenden Schicht (10) gehalten wird, die Flüssigkeit (4) und den eingetauchten/benetzten Bereich des Körpers (2) zumindest bereichsweise voneinander trennt, wobei die Flüssigkeit (4) ein polares Lösungsmittel ist, und
wobei die vorstehenden Elemente (26, 27) einen zentralen Oberflächenbereich (26e, 27e) aufweisen, der hydrophil ist und der von einem hydrophoben Oberflächenbereich der vorstehenden Elemente (26, 27) umgeben ist.

## Claims

1. Use of a gas-retaining layer (10), which is arranged on a body (2) that can be immersed in a liquid or that can be wetted with a liquid, and which, when the body (2) is immersed/wetted, is in contact with the liquid (4) with a liquid-facing side (10a) thereof, wherein the body (2) is a wall of a watercraft or a construction arranged in the water or an inner wall of a liquid container or a liquid line, wherein the gas-retaining layer (10) on the liquid-facing side (10a) has recesses (30) and/or protruding elements (26, 27), wherein the surfaces of the recesses (30) are at least partially hydrophobic and the surface of the protruding elements (26, 27) is substantially hydrophobic,
wherein a gas layer (5) with a thickness of 10 nm to 10 mm, which is held in the immersed/wetted region of the gas-retaining layer (10), separates the liquid (4) and the immersed/wetted area of the body (2) at least partially from each other, wherein the liquid (4) is a polar solvent, and
wherein the gas-retaining layer (10) is subdivided into a plurality of subregions (44) by fluid-impermeable partition walls (42), and the partition walls (42) are formed to be at least partially hydrophilic.

2. Use according to claim 1, wherein a corrosion protection layer and/or an anti-fouling layer is arranged between the gas-retaining layer (10) and the wall of the body (2), and wherein the gas-retaining layer (10) at least partially separates the corrosion protection layer and/or the anti-fouling layer from the liquid.

3. Use of the gas-retaining layer (10) according to claim 1 or 2, wherein the fluid-impermeable partition walls (42), which have the same length, are arranged such that hexagonal subregions (44) are formed, or wherein the fluid-impermeable partition walls (42) are arranged such that elongated, honeycomb-shaped or hexagonal subregions (44) are formed.

4. Use of the gas-retaining layer (10) according to one of the preceding claims, wherein in each of the subregions (44) protruding elements (26, 27) are present in a two-dimensional arrangement.

5. Use of the gas-retaining layer (10) according to one of the preceding claims, wherein the protruding elements (26, 27) have a central surface area (26e, 27e), which is hydrophilic and which is surrounded by a hydrophobic surface area of the protruding elements (26, 27).

6. Use of the gas-retaining layer (10) according to one of the preceding claims, wherein the gas-retaining layer (10) is fed with gas from a body-facing side (10a) of the gas-retaining layer (10) opposite the liquid-facing side (10a).

7. Use of the gas-retaining layer (10) according to one of the preceding claims, wherein a gas-permeable layer (12) is arranged on the body-facing side (10b) of the gas-retaining layer (10).

8. Use of the gas-retaining layer (10) according to claim 7, wherein the gas-permeable layer (12) is formed as a liquid-impermeable and/or hydrophobic sheet.

9. Use of the gas-retaining layer (10) according to claim 7 or 8, wherein a gas supply device (14) is connected to the gas-permeable layer (12) such that gas (5) can flow from a gas supply device (14) through the gas-permeable layer (12) to the gas-retaining layer (10).

10. Use of the gas-retaining layer (10) according to one of claims 7 to 9, wherein the gas-permeable layer (12) is formed as a porous, semipermeable membrane.

11. Use of the gas-retaining layer (10) according to claim 9 or 10, wherein the gas supply device (14) is formed as a gas-permeable layer arranged on the body-facing side of the gas-permeable layer (12).

12. Use of the gas-retaining layer (10) according to one of claims 1 to 5, wherein the gas-retaining layer (10) is fed with gas from the liquid-facing side (10a) of the gas-retaining layer (10).

13. Use of the gas-retaining layer (10) according to claim 12, wherein at least one gas outlet device (28) is provided, which has a gas outlet opening on the liquid-facing side (10a) of the gas-retaining layer (10); and
wherein a gas supply device (14) is provided, which is connected to the gas outlet device (28), wherein gas provided by the gas supply device (14) flows out of the gas outlet device (28) and is at least partially absorbed by the gas-retaining layer.

14. Use of the gas-retaining layer (10) according to claim 12, wherein the gas outlet device (28) penetrates the gas-retaining layer (10).

15. Use of the gas-retaining layer (10) according to one of claims 12 to 14, wherein a gas-permeable layer (12) is arranged on the body-facing side (10b) of the gas-retaining layer (10).

16. Use of the gas-retaining layer (10) according to claim 15, wherein the gas-permeable layer (12) is formed as a liquid-impermeable and/or hydrophobic sheet.

17. Use of the gas-retaining layer (10) according to claim 15 or 16, wherein the gas-permeable layer (12) is formed as a porous, semipermeable membrane.

18. Use of the gas-retaining layer (10) according to one of the preceding claims, wherein the surface structure of the gas-retaining layer (10) comprises a material selected from polymers, lacquers, ceramics, resins, PDMS, rubber, silicone rubber, and metals.

19. Use of the gas-retaining layer (10) according to claim 18, wherein the gas-retaining layer consists of an embossed synthetic resin or lacquer.

20. Use of the gas-retaining layer (10) according to one of the preceding claims, wherein the liquid (4) is water (4') and the body (2) is a watercraft (2'), the walls of which are at least partially immersed in the water (4') in an operating position of the watercraft (2').

21. Use of the gas-retaining layer (10) according to claim 20, wherein the gas-retaining layer (10) is at least partially fed with a gas such as to reduce the flow resistance between the water (4') and the watercraft (2').

22. Use of the gas-retaining layer (10) according to claim 20 or 21, wherein the gas-retaining layer (10) is charged with an antifouling gas.

23. Use of the gas-retaining layer (10) according to one of the preceding claims, wherein the gas layer (5) is formed in the form of an array of small gas bubbles at predefined locations on the surface of the gas-retaining layer (10).

24. Use of a gas-retaining layer (10), which is arranged on a body (2) that can be immersed in a liquid or that can be wetted with a liquid, and which, when the body (2) is immersed/wetted, is in contact with the liquid (4) with a liquid-facing side (10a) thereof, wherein the body (2) is a fiber, wherein the gas-retaining layer (10) on the liquid-facing side (10a) has protruding elements (26, 27), the surfaces of which are substantially hydrophobic,
wherein a gas layer (5) with a thickness of 10 nm to 10 mm, which is held in the immersed/wetted region of the gas-retaining layer (10), separates the liquid (4) and the immersed/wetted area of the body (2) at least partially from each other, wherein the liquid (4) is a polar solvent, and
wherein the protruding elements (26, 27) have a central surface area (26e, 27e), which is hydrophilic and which is surrounded by a hydrophobic surface area of the protruding elements (26, 27).

## Revendications

1. Utilisation d'une couche contenant du gaz (10), laquelle est située sur un corps immergeable dans un liquide ou pouvant être mouillé par un liquide (2) et qui est en contact, par un côté face au liquide (10a) en cas d'immersion/mouillage du corps (2), avec le liquide (4), dans lequel le corps (2) est la cloison d'un véhicule aquatique ou d'un bâtiment disposé dans l'eau ou la cloison intérieure d'un récipient de liquide ou d'une conduite de liquide, la couche contenant du gaz (10) comprenant sur le côté face au liquide (10a), des enfoncements (30) et/ou des éléments saillants (26, 27), les surfaces des enfoncements (30) étant au moins en partie hydrophobes et la surface des éléments saillants (26, 27) étant substantiellement hydrophobe,
où une couche de gaz (5) a une épaisseur allant de 10 nm à 10 mm, laquelle est maintenue dans la zone immergée/mouillée de la couche contenant du gaz (10), sépare l'un de l'autre au moins en partie, le liquide (4) et la zone immergée/mouillée du corps (2), le liquide (4) étant un solvant polaire, et
où la couche contenant du gaz (10) est subdivisée en une pluralité de sous-régions (44) par des cloisons de séparation (42) étanches aux fluides et les cloisons de séparation (42) sont réalisées au moins en partie hydrophiles.

2. Utilisation selon la revendication 1, dans laquelle une couche de protection anticorrosion et/ou une couche antisalissure se trouve agencée entre la couche contenant du gaz (10) et la cloison du corps (2) et où la couche contenant du gaz (10) sépare au moins en partie du liquide la couche de protection anticorrosion et/ou la couche antisalissure.

3. Utilisation de la couche contenant du gaz (10) selon la revendication 1 ou 2, dans laquelle les cloisons de séparation (42) étanches aux fluides, lesquelles sont de mêmes longueurs, sont disposées de telle manière qu'il se forme des parties de zone (44) hexagonales, ou dans laquelle les cloisons de séparation (42) étanches aux fluides, sont disposées de telle manière qu'il se forme des sous-régions (44) longitudinalement étendues, hexagonales ou de forme alvéolaire.

4. Utilisation de la couche contenant du gaz (10) selon l'une des revendications précédentes, dans laquelle, dans chacune des sous-régions (44), des éléments saillants (26, 27) sont présents dans une structure bidimensionnelle.

5. Utilisation de la couche contenant du gaz (10) selon l'une des revendications précédentes, dans laquelle les éléments saillants (26, 27) présentent une zone de surface centrale (26e, 27e), laquelle est hydrophile et est entourée d'une zone de surface hydrophobe des éléments saillants (26, 27).

6. Utilisation de la couche contenant du gaz (10) selon l'une des revendications précédentes, dans laquelle la couche contenant du gaz (10) est alimentée en gaz à partir d'un côté face au corps (10b) vis-à-vis du côté face au liquide (10a) de la couche contenant le gaz (10).

7. Utilisation de la couche contenant du gaz (10) selon l'une des revendications précédentes, dans laquelle une couche perméable aux gaz (12) est disposée sur le côté face au corps (10b) de la couche contenant du gaz (10).

8. Utilisation de la couche contenant du gaz (10) selon la revendication 7, dans laquelle la couche perméable aux gaz (12) est réalisée comme une feuille étanche aux liquides et/ou hydrophobe.

9. Utilisation de la couche contenant du gaz (10) selon la revendication 7 ou 8, dans laquelle un dispositif d'amenée de gaz (14) est connecté à la couche perméable aux gaz (12) de telle sorte que du gaz (5) peut s'écouler d'un dispositif d'amenée de gaz (14) par la couche perméable aux gaz (12) jusqu'à la couche contenant du gaz (10).

10. Utilisation de la couche contenant du gaz (10) selon l'une des revendications 7 à 9, dans laquelle la couche perméable aux gaz (12) est réalisée comme une membrane poreuse, semi-perméable.

11. Utilisation de la couche contenant du gaz (10) selon la revendication 9 ou 10, dans laquelle le dispositif d'amenée de gaz (14) est réalisé comme une couche perméable aux gaz, laquelle est disposée sur le côté face au corps de la couche perméable aux gaz (12).

12. Utilisation de la couche contenant du gaz (10) selon l'une des revendications 1 à 5, dans laquelle la couche contenant du gaz (10) est alimentée en gaz à partir du côté face au liquide (10a) de la couche contenant le gaz (10).

13. Utilisation de la couche contenant du gaz (10) selon la revendication 12, dans laquelle au moins un dispositif de sortie de gaz (28) est prévu, lequel présente un orifice de sortie de gaz sur le côté face au liquide (10a) de la couche contenant du gaz (10); et
un dispositif d'amenée de gaz (14) est prévu, lequel est connecté au dispositif de sortie de gaz (28), le gaz fourni du dispositif d'amenée de gaz (14) s'écoulant du dispositif de sortie de gaz (28) et étant au moins partiellement absorbé par la couche contenant du gaz.

14. Utilisation de la couche contenant du gaz (10) selon la revendication 12, dans laquelle le dispositif de sortie de gaz (28) pénètre la couche contenant du gaz (10).

15. Utilisation de la couche contenant du gaz (10) selon l'une des revendications 12 à 14, dans laquelle une couche perméable aux gaz (12) est disposée sur le côté face au corps (10b) de la couche contenant du gaz (10).

16. Utilisation de la couche contenant du gaz (10) selon la revendication 15, dans laquelle la couche perméable aux gaz (12) est réalisée comme une feuille étanche aux liquides et/ou hydrophobe.

17. Utilisation de la couche contenant du gaz (10) selon la revendication 15 ou 16, dans laquelle la couche perméable aux gaz (12) est réalisée comme une membrane poreuse, semi-perméable.

18. Utilisation de la couche contenant du gaz (10) selon l'une des revendications précédentes, dans laquelle la structure de surface de la couche contenant du gaz (10) comprend une matière sélectionnée parmi des polymères, des laques, des céramiques, des résines, du PDMS, de la gomme, du caoutchouc de silicone et des métaux.

19. Utilisation de la couche contenant du gaz (10) selon la revendication 18, dans laquelle la couche contenant du gaz est constituée d'une résine artificielle gaufrée ou de laque.

20. Utilisation de la couche contenant du gaz (10) selon l'une des revendications précédentes, dans laquelle le liquide (4) est de l'eau (4') et le corps (2) est un véhicule aquatique (2') dont la cloison est, en position de fonctionnement du véhicule aquatique (2'), au moins en partie immergée dans l'eau (4').

21. Utilisation de la couche contenant du gaz (10) selon la revendication 20, dans laquelle la couche contenant du gaz (10) est alimentée en gaz au moins en partie de manière à diminuer la résistance d'écoulement entre l'eau (4') et le véhicule aquatique (2').

22. Utilisation de la couche contenant du gaz (10) selon la revendication 20 ou 21, dans laquelle la couche contenant du gaz (10) est chargée d'un gaz antisalissure.

23. Utilisation de la couche contenant du gaz (10) selon l'une des revendications précédentes, dans laquelle la couche contenant du gaz (5) est réalisée sous forme d'une matrice de bulles de gaz sur des points prédéfinis à la surface de la couche contenant du gaz (10).

24. Utilisation d'une couche contenant du gaz (10), laquelle est située sur un corps immergeable dans un liquide ou pouvant être mouillé par un liquide (2) et qui est en contact, par un côté face au liquide (10a) en cas d'immersion/mouillage du corps (2), avec le liquide, le corps (2) étant une fibre, la couche contenant du gaz (10) présentant sur le côté face au liquide (10a), des éléments saillants (26, 27) dont la surface est essentiellement hydrophobe,
une couche de gaz (5) d'une épaisseur allant de 10 nm à 10 mm, laquelle est maintenue dans la zone immergée/mouillée de la couche contenant du gaz (10), séparant au moins en partie l'un de l'autre le liquide (4) et la zone immergée/mouillée du corps (2), le liquide (4) étant un solvant polaire, et
les éléments saillants (26, 27) présentant une zone de surface centrale (26e, 27e), laquelle est hydrophile et est entourée d'une zone de surface hydrophobe des éléments saillants (26, 27).
